(19) Europäisches Patentamt
European Patent Office
Office européen des brevets

(11) EP 2 377 891 B1

(12) # EUROPEAN PATENT SPECIFICATION

(45) Date of publication and mention of the grant of the patent:
**21.11.2018 Bulletin 2018/47**

(21) Application number: **09834988.9**

(22) Date of filing: **25.12.2009**

(51) Int Cl.:
***C07K 16/28*** (2006.01)
***A61K 39/395*** (2006.01)
***A61P 35/00*** (2006.01)
***A61P 43/00*** (2006.01)
***C12N 15/02*** (2006.01)
***C12N 15/09*** (2006.01)
***G01N 33/53*** (2006.01)
***G01N 33/574*** (2006.01)
***C12P 21/08*** (2006.01)

(86) International application number:
**PCT/JP2009/071524**

(87) International publication number:
**WO 2010/074192 (01.07.2010 Gazette 2010/26)**

(54) **DIAGNOSIS AND TREATMENT OF CANCER USING ANTI-LGR7 ANTIBODY**

DIAGNOSE UND BEHANDLUNG VON KREBS MIT ANTI-LGR7-ANTIKÖRPER

DIAGNOSTIC ET TRAITEMENT DU CANCER À L'AIDE D'UN ANTICORPS ANTI-LGR7

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **26.12.2008 JP 2008333149**

(43) Date of publication of application:
**19.10.2011 Bulletin 2011/42**

(73) Proprietors:
- **The University of Tokyo**
**Bunkyo-ku,**
**Tokyo 113-8654 (JP)**
- **Chugai Seiyaku Kabushiki Kaisha**
**Kita-ku**
**Tokyo 115-8543 (JP)**

(72) Inventors:
- **ABURATANI, Hiroyuki**
**Tokyo 113-8654 (JP)**
- **UEHARA, Yuriko**
**Tokyo 167-0043 (JP)**
- **FUNAHASHI, Shinichi**
**Tokyo 153-0041 (JP)**

(74) Representative: **Bösl, Raphael Konrad**
**Isenbruck Bösl Hörschler LLP**
**Patentanwälte**
**Prinzregentenstraße 68**
**81675 München (DE)**

(56) References cited:
**WO-A1-02/28418**
**WO-A1-99/48921**
**WO-A1-99/56129**
**WO-A2-03/016487**
**WO-A2-03/093827**
**US-A1- 2005 107 595**

- **APARNA A. KAMAT ET AL: "The role of relaxin in endometrial cancer", CANCER BIOLOGY & THERAPY, vol. 5, no. 1, 1 January 2006 (2006-01-01) , pages 71-77, XP55032608, ISSN: 1538-4047, DOI: 10.4161/cbt.5.1.2289**
- **HANSELL D J ET AL: "THE ROLE OF RELAXIN IN PROMOTING PROSTATE CANCER CELLULAR MOTILITY AND WOUND HEALING", JOURNAL OF CELLULAR BIOCHEMISTRY. SUPPLEMENT, A.R. LISS, NEW YORK, NY, US, no. 18D, 1 March 1994 (1994-03-01), page 230, XP002908332, ISSN: 0733-1959**
- **L. A. VODSTRCIL ET AL: "Progesterone Withdrawal, and Not Increased Circulating Relaxin, Mediates the Decrease in Myometrial Relaxin Receptor (RXFP1) Expression in Late Gestation in Rats", BIOLOGY OF REPRODUCTION, vol. 83, no. 5, 1 November 2010 (2010-11-01), pages 825-832, XP55032558, ISSN: 0006-3363, DOI: 10.1095/biolreprod.110.084301**
- **IVELL, R. ET AL.: 'Immunoexpression of the relaxin receptor LGR7 inbreast and uterine tissues of humans and primates.' REPROD. BIOL. ENDOCRINOL. vol. 1, no. 114, 24 November 2003, pages 1/13 - 13/13, XP021009265**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 377 891 B1

## Description

Technical Field

The present invention relates to monoclonal antibodies that bind to the LGR7 protein and to such antibodies for use in the treatment of cancer.

Background Art

The LGR7 molecule is a protein encoded by the gene of Ensembl ID ENSG00000171509 at 4q32 on human chromosome. Based on the features of its amino acid sequence, the molecule is classified as a member of the LGR family of the G-protein-coupled seven-transmembrane hormone receptor protein family (Leucine-rich GPCR family; hereinafter, referred to as LGR family) (Non-patent Document 1), and it is registered as NM_021634/NP_067647 in RefSeq. A sequence in which Leu at amino acid position 70 is substituted with Met has also been reported (Patent Document 1). Furthermore, three splice variants have been reported. In LGR7.1 (AY899848.1), exon 6a is inserted between exons 6 and 7, and exon 15a is inserted between exons 15 and 16. LGR7.2 (AY899849.1) has a gene structure from which exons 12 and 13 are deleted. In LGR7.10 (AY899850.1), exon 3 is deleted. Members of the LGR family are classified into three groups: the first group includes the hormone receptors FSHR(LGR1), LHCGR(LGR2), and TSHR(LGR3); the second group includes LGR4, LGR5, and LGR6 whose ligands are unknown; and the third group includes LGR7 and LGR8 whose ligands are relaxin, insulin-like peptide 3 (INSL3), and such (Non-patent Document 2). In all cases, the ligands are two heterogeneous peptides and they mainly transduce cAMP-mediated signals into cells. The LGR family has a structure comprising a seven-transmembrane protein region and a long N-terminal extracellular domain, and the extracellular domain has 9 to 17 leucine-rich repeats (LRRs) comprising about 25 amino acid residues. LGR7 has ten LRRs (Non-patent Document 1). It also has an LDL-A domain at the N terminus immediately before LRR, which is not found in other LGR family molecules (Non-patent Document 3). It has been reported that LDL-A is necessary for signal transduction, and it is involved in membrane trafficking of LGR7 (Non-patent Document 4). It is known from analyses of TSHR and such that in LGR family molecules, the extracellular LRR binds with high affinity to a ligand which further binds to the second extracellular loop domain, thereby causing G-protein-coupled signal transduction (Non-patent Document 5). Relaxins are known as ligands that bind to LGR7, and include human Relaxin 2 and Relaxin 3. Relaxin 2 has higher binding ability, and it is thought to function as a ligand of LGR7 *in vivo* (Non-patent Documents 6 and 7).

There is a report on the association of relaxin (ligand) with thyroid carcinoma and prostate cancer (Non-patent Document 8). Regarding prostate cancer, it is reported that androgen-independent growth is promoted in the prostate cancer cell line LNCaP introduced with p53 which has amino acid mutation R to H at position 273 (Non-patent Document 9). Since the level of H2 relaxin is elevated in these cells, it is suggested that H2 relaxin expression is involved in prostate cancer progression. It is shown that p53 R273H binds directly to the H2 relaxin promoter and induces PSA expression via an androgen receptor. However, there are still no articles that report the association between LGR7 and cancer.

On the other hand, some patent documents have reported higher LGR7 gene expression in uterine cancer and ovarian cancer than in normal tissues, and the association between LGR7 and cancer. However, it was not known whether LGR7-expressing cancers can be treated using antibodies, and none of the documents demonstrates an antibody-mediated anticancer effect (Patent Documents 2 to 5).

Even among ovarian cancers, clear cell adenocarcinoma is known to be a type of cancer that is less effective by chemotherapy (Non-patent Documents 10 and 11). Sugiyama *et al.* reported that the response rate towards chemotherapy using cisplatin and taxane compounds, which is a standard therapeutic method, is 72.5% in serous adenocarcinoma, and as low as 11.1% in clear cell adenocarcinoma (Non-patent Document 10). Meanwhile, the number of patients with clear cell adenocarcinoma is increasing in recent years. According to Nihon Sanfujinka Gakkai-shi Vol. 57, No. 11, p. 1711 (2005), clear cell adenocarcinoma accounts for 22% of all ovarian cancers in Japan. This is highly different from the number (6%) reported in the 1998 FIGO Annual Report overseas. Furthermore, according to the report by Japan Society of Obstetrics and Gynecology, the ratio of clear cell adenocarcinoma to all epithelial malignant tumors in ovarian cancers was 4% from 1971 to 1977, approximately 10% from 1978 to 1983, but more than 20% in 2002, and it is continuing to increase. Therefore, it is desirable to develop therapeutic methods against clear cell adenocarcinoma.
Polyclonal anti-LGR7 antibodies and their use in studying expression of the LGR7 protein in tissue are disclosed in Non-Patent Document 12. The authors found that LGR7 is expressed in the stromal cells of endometrium and breast.
Methods of diagnosing cancer by detecting the expression of relaxin and its receptor LGR7 in a majority of endometrial carcinomas is disclosed in Non-Patent Document 13. Also disclosed is the inhibition of endogenous relaxin signaling via LGR7 antagonists causing reduction of endometrial carcinoma cell invasiveness. Compositions useful for diagnosis and treatment of tumors are disclosed in Patent Document 6. The document discloses a polypeptide designated TAT186 and refers in general to antibodies recognizing TAT186.
Methods of modulating apoptosis by administration of relaxin agonists and antagonists are disclosed in Patent Document 7. Thereby, anti-LGR7 antibodies and their use in cancer diagnostic methods are disclosed.
The nucleotide sequence of LGR7 and its use for producing the LGR7 protein and antibody binding to the LGR7 protein, for producing a transgenic animal with an alteration in the LGR7 gene and for screening for the presence of a ligand for LGR7 are disclosed in Patent Document 8.
The relaxin receptor LGR7 and its nucleotide sequence as well as its usefulness as therapeutic agent inhibiting the activity of relaxin and Ins1.3 and in screening and design of relaxin agonists and antagonists for the treatment of various conditions are disclosed in Patent Document 9. Methods of screening for therapeutic agents useful in the treatment of hematological diseases, cardiovascular diseases, disorders of the peripheral and central nervous system, respiratory diseases, genito-urological diseases, and gastro-intestinal diseases using as test compound the LGR7 protein are disclosed in Patent Document 10.

[Prior Art Documents]

[Patent Documents]

[Patent Document 1] WO 9948921
[Patent Document 2] US 2005107595
[Patent Document 3] WO 2003016487
[Patent Document 4] WO 2003093827
[Patent Document 5] WO 2005107396
[Patent Document 6] US 2005/107595
[Patent Document 7] WO 02/28418
[Patent Document 8] WO 99/48921
[Patent Document 9] WO 03/016487
[Patent Document 10] WO 03/093827

[Non-Patent Documents]

[Non-Patent Document 1] Hsu, S. Y. et al., Molec. Endocr., 14, 1257-1271 (2000)
[Non-Patent Document 2] Hsueh A. J. W. et al., Journal of Endocrinology, 187, 333-338 (2005)
[Non-Patent Document 3] Bathgate RA. et al., Pharmacol Rev, 58, 7-31 (2006)
[Non-Patent Document 4] Kern A. et al., Endocrinology, 148, 1181-1194 (2007)
[Non-Patent Document 5] Kristiansen K., Pharmacology & Therapeutics, 103, 21-80 (2004)
[Non-Patent Document 6] Halls M. L. et al., British Journal of Pharmacology, 150, 677-691 (2007)
[Non-Patent Document 7] Van Der Westhuizen, E. T. et al., Current Drug Targets, 8, 91-104 (2007)
[Non-Patent Document 8] Hombach-Klonisch S. et al., American Journal of Pathology, 169, 617-632 (2006)
[Non-Patent Document 9] Vinall R. L. et al., Oncogene, 25, 2082-2093 (2006)
[Non-Patent Document 10] Sugiyama T. et al., Cancer, 88, 2584 (2000)
[Non-Patent Document 11] Enomoto T. et al., Proceedings of ASCO. 2003; 1797, Chicago
[Non-Patent Document 12] Ivell R. et al., Reprod. Biol. Endocrinol., 114, 1/13-13/13 (2003)
[Non-Patent Document 13] Kamat A. A. et al., Cancer Biology & Therapy, 5, 71-77 (2006)

## Summary of the Invention

[Problems to be Solved by the Invention]

An objective of the present invention is to provide novel antibodies that bind to the LGR7 protein and the novel antibodies for use in treating cancer.

[Means for Solving the Problems]

The present inventors discovered that not only the LGR7 gene but also the LGR7 protein are highly expressed in clear cell adenocarcinoma cells of ovarian cancer. There has been no report that LGR7 is closely related to only one type of carcinoma among ovarian cancers - clear cell adenocarcinoma.

Furthermore, the present inventors produced monoclonal antibodies against the LGR7 protein.

The present inventors measured the antibody-dependent cell-mediated cytotoxicity (ADCC) activities of the anti-LGR7 antibodies, and discovered that the anti-LGR7 antibodies have ADCC activity against LGR7-expressing cells. The present inventors also measured the complement-dependent cell-mediated cytotoxicity (CDC) activities, and discovered that the anti-LGR7 antibodies have CDC activity against LGR7-expressing cells. In addition, the tumor regression effect of the anti-LGR7 antibodies was demonstrated by administering them to xenograft tumor model mice. Based on the above findings, the present inventors discovered that the anti-LGR7 antibodies are effective for diagnosis, prevention, and treatment of primary or metastatic ovarian clear cell adenocarcinoma, and thus completed the present invention. More specifically, the present inventors discovered that the anti-LGR7 antibodies are useful as tools for treatment and diagnosis of cancers highly expressing LGR7 such as ovarian clear cell adenocarcinoma, and thus completed the present invention.

Specifically, the present invention provides monoclonal LGR7 protein-binding antibodies. Furthermore, the present invention provides LGR7 protein-binding monoclonal antibodies that have cytotoxic activity against cells expressing the LGR7 protein. Preferably, the cytotoxic activity is ADCC activity. The present invention also provides monoclonal anti-LGR7 antibodies bound with a cytotoxic substance.

Furthermore, the present invention provides monoclonal LGR7 protein-binding antibodies for use in the treatment of cancer.

Specifically, the present invention provides the inventions of [1] to [9] directly below:

[1] A monoclonal antibody of any one of (1) to (2) below that binds to an LGR7 protein, wherein the antibody has internalization activity against cells expressing the LGR7 protein:

(1) an antibody comprising an H chain having the amino acid sequence of SEQ ID NO: 25 as CDR1, the amino acid sequence of SEQ ID NO: 26 as CDR2, and the amino acid sequence of SEQ ID NO: 27 as CDR3 and comprising an L chain having the amino acid sequence of SEQ ID NO: 30 as CDR1, the amino acid sequence of SEQ ID NO: 31 as CDR2, and the amino acid sequence of SEQ ID NO: 32 as CDR3;
(2) an antibody that competes with the antibody of (1) for binding to the epitope that the antibody of (1) binds to.

[2] The antibody of (2) in [1], wherein the antibody is selected from any one of (a) to (g):

(a) an antibody comprising an H chain having the amino acid sequence of SEQ ID NO: 5 as CDR1, the amino acid sequence of SEQ ID NO: 6 as CDR2, and the amino acid sequence of SEQ ID NO: 7 as CDR3 and comprising an L chain having the amino acid sequence of SEQ ID NO: 10 as CDR1, the amino acid sequence of SEQ ID NO: 11 as CDR2, and the amino acid sequence of SEQ ID NO: 12 as CDR3;
(b) an antibody comprising an H chain having the amino acid sequence of SEQ ID NO: 15 as CDR1, the amino acid sequence of SEQ ID NO: 16 as CDR2, and the amino acid sequence of SEQ ID NO: 17 as CDR3 and comprising an L chain having the amino acid sequence of SEQ ID NO: 20 as CDR1, the amino acid sequence of SEQ ID NO: 21 as CDR2, and the amino acid sequence of SEQ ID NO: 22 as CDR3;
(c) an antibody comprising an H chain having the amino acid sequence of SEQ ID NO: 45 as CDR1, the amino acid sequence of SEQ ID NO: 46 as CDR2, and the amino acid sequence of SEQ ID NO: 47 as CDR3 and comprising an L chain having the amino acid sequence of SEQ ID NO: 50 as CDR1, the amino acid sequence of SEQ ID NO: 51 as CDR2, and the amino acid sequence of SEQ ID NO: 52 as CDR3;
(d) an antibody comprising an H chain having the amino acid sequence of SEQ ID NO: 55 as CDR1, the amino acid sequence of SEQ ID NO: 56 as CDR2, and the amino acid sequence of SEQ ID NO: 57 as CDR3 and comprising an L chain having the amino acid sequence of SEQ ID NO: 60 as CDR1, the amino acid sequence of SEQ ID NO: 61 as CDR2, and the amino acid sequence of SEQ ID NO: 62 as CDR3;
(e) an antibody comprising an H chain having the amino acid sequence of SEQ ID NO: 65 as CDR1, the amino acid sequence of SEQ ID NO: 66 as CDR2, and the amino acid sequence of SEQ ID NO: 67 as CDR3 and comprising an L chain having the amino acid sequence of SEQ ID NO: 70 as CDR1, the amino acid sequence of SEQ ID NO: 71 as CDR2, and the amino acid sequence of SEQ ID NO: 72 as CDR3;
(f) an antibody comprising an H chain having the amino acid sequence of SEQ ID NO: 75 as CDR1, the amino acid sequence of SEQ ID NO: 76 as CDR2, and the amino acid sequence of SEQ ID NO: 77 as CDR3 and comprising an L chain having the amino acid sequence of SEQ ID NO: 80 as CDR1, the amino acid sequence of SEQ ID NO: 81 as CDR2, and the amino acid sequence of SEQ ID NO: 82 as CDR3;
(g) an antibody comprising an H chain having the amino acid sequence of SEQ ID NO: 85 as CDR1, the amino acid sequence of SEQ ID NO: 86 as CDR2, and the amino acid sequence of SEQ ID NO: 87 as CDR3 and comprising an L chain having the amino acid sequence of SEQ ID NO: 90 as CDR1, the amino acid sequence of SEQ ID NO: 91 as CDR2, and the amino acid sequence of SEQ ID NO: 92 as CDR3.

[3] The antibody of any one of [1] to [2], which is an antibody that has cytotoxic activity; particularly wherein the cytotoxic activity is antibody-dependent cell-mediated cytotoxicity activity or wherein the cytotoxic activity is complement-dependent cytotoxicity activity.
[4] The antibody of any one of [1] to [3], which is an antibody to which a cytotoxic substance is bound.
[5] The antibody of any one of [1] to [4], which is an antibody that suppresses the growth of cancer cells, particularly wherein the cancer cells are clear cell ovarian cancer cells.
[6] The antibody of any one of [1] to [5], wherein the antibody has a human constant region, particularly wherein the antibody is a chimeric antibody, humanized antibody, or human antibody.
[7] The antibody of any one of [1] to [6], wherein the antibody is a fucose-deficient antibody.
[8] The antibody of any one of [1] to [7] for use in the treatment of cancer.
[9] The antibody for use of [8], wherein the cancer is ovarian cancer; particularly wherein the ovarian cancer is clear cell adenocarcinoma.

Disclosed are

[1] An antibody that binds to an LGR7 protein and which has cell growth inhibitory activity against cells expressing the LGR7 protein.
[2] The antibody of [1], wherein the cell growth inhibitory activity is cytotoxic activity.
[3] The antibody of [2], wherein the cytotoxic activity is antibody-dependent cell-mediated cytotoxicity activity.
[4] The antibody of [2], wherein the cytotoxic activity is complement-dependent cytotoxicity activity.
[5] The antibody of any one of [1] to [4], which is an antibody to which a cytotoxic substance is bound.
[6] The antibody of [5], which is an antibody having an internalizing activity.
[7] The antibody of any one of [1] to [6], which is an antibody that suppresses the growth of cancer cells.
[8] The antibody of [7], wherein the cancer cells are clear cell ovarian cancer cells.
[9] An antibody of any one of (1) to (29) below:

(1) an antibody comprising an H chain having the amino acid sequence of SEQ ID NO: 5 as CDR1, the amino acid sequence of SEQ ID NO: 6 as CDR2, and the amino acid sequence of SEQ ID NO: 7 as CDR3 (22DA6 heavy chain);
(2) an antibody comprising an L chain having the amino acid sequence of SEQ ID NO: 10 as CDR1, the amino acid sequence of SEQ ID NO: 11 as CDR2, and the amino acid sequence of SEQ ID NO: 12 as CDR3 (22DA6 light chain);
(3) an antibody comprising the H chain of (1) and the L chain of (2) (22DA6);
(4) an antibody comprising an H chain having the amino acid sequence of SEQ ID NO: 15 as CDR1, the amino acid sequence of SEQ ID NO: 16 as CDR2, and the amino acid sequence of SEQ ID NO: 17 as CDR3 (22DA7 heavy chain);
(5) an antibody comprising an L chain having the amino acid sequence of SEQ ID NO: 20 as CDR1, the amino acid sequence of SEQ ID NO: 21 as CDR2, and the amino acid sequence of SEQ ID NO: 22 as CDR3 (22DA7 light chain);
(6) an antibody comprising the H chain of (4) and the L chain of (5) (22DA7);
(7) an antibody comprising an H chain having the amino acid sequence of SEQ ID NO: 25 as CDR1, the amino acid sequence of SEQ ID NO: 26 as CDR2, and the amino acid sequence of SEQ ID NO: 27 as CDR3 (22DA17 heavy chain);
(8) an antibody comprising an L chain having the amino acid sequence of SEQ ID NO: 30 as CDR1, the amino acid sequence of SEQ ID NO: 31 as CDR2, and the amino acid sequence of SEQ ID NO: 32 as CDR3 (22DA17 light chain);
(9) an antibody comprising the H chain of (7) and the L chain of (8) (22DA17);
(10) an antibody comprising an H chain having the amino acid sequence of SEQ ID NO: 35 as CDR1, the amino acid sequence of SEQ ID NO: 36 as CDR2, and the amino acid sequence of SEQ ID NO: 37 as CDR3 (22DA22 heavy chain);
(11) an antibody comprising an L chain having the amino acid sequence of SEQ ID NO: 40 as CDR1, the amino acid sequence of SEQ ID NO: 41 as CDR2, and the amino acid sequence of SEQ ID NO: 42 as CDR3 (22DA22 light chain);
(12) an antibody comprising the H chain of (10) and the L chain of (11) (22DA22);
(13) an antibody comprising an H chain having the amino acid sequence of SEQ ID NO: 45 as CDR1, the amino acid sequence of SEQ ID NO: 46 as CDR2, and the amino acid sequence of SEQ ID NO: 47 as CDR3 (22DA23 heavy chain);
(14) an antibody comprising an L chain having the amino acid sequence of SEQ ID NO: 50 as CDR1, the amino acid sequence of SEQ ID NO: 51 as CDR2, and the amino acid sequence of SEQ ID NO: 52 as CDR3 (22DA23 light chain);
(15) an antibody comprising the H chain of (13) and the L chain of (14) (22DA23);
(16) an antibody comprising an H chain having the amino acid sequence of SEQ ID NO: 55 as CDR1, the amino acid sequence of SEQ ID NO: 56 as CDR2, and the amino acid sequence of SEQ ID NO: 57 as CDR3 (22DA24 heavy chain);
(17) an antibody comprising an L chain having the amino acid sequence of SEQ ID NO: 60 as CDR1, the amino acid sequence of SEQ ID NO: 61 as CDR2, and the amino acid sequence of SEQ ID NO: 62 as CDR3 (22DA24 light chain);
(18) an antibody comprising the H chain of (16) and the L chain of (17) (22DA24);
(19) an antibody comprising an H chain having the amino acid sequence of SEQ ID NO: 65 as CDR1, the amino acid sequence of SEQ ID NO: 66 as CDR2, and the amino acid sequence of SEQ ID NO: 67 as CDR3 (22SD7 heavy chain);
(20) an antibody comprising an L chain having the amino acid sequence of SEQ ID NO: 70 as CDR1, the amino acid sequence of SEQ ID NO: 71 as CDR2, and the amino acid sequence of SEQ ID NO: 72 as CDR3 (22SD7 light chain);
(21) an antibody comprising the H chain of (19) and the L chain of (20) (22SD7);
(22) an antibody comprising an H chain having the amino acid sequence of SEQ ID NO: 75 as CDR1, the amino acid sequence of SEQ ID NO: 76 as CDR2, and the amino acid sequence of SEQ ID NO: 77 as CDR3 (22SD11 heavy chain);
(23) an antibody comprising an L chain having the amino acid sequence of SEQ ID NO: 80 as CDR1, the amino acid sequence of SEQ ID NO: 81 as CDR2, and the amino acid sequence of SEQ ID NO: 82 as CDR3 (22SD11 light chain);
(24) an antibody comprising the H chain of (22) and the L chain of (23) (22SD11);
(25) an antibody comprising an H chain having the amino acid sequence of SEQ ID NO: 85 as CDR1, the amino acid sequence of SEQ ID NO: 86 as CDR2, and the amino acid sequence of SEQ ID NO: 87 as CDR3 (22SD48 heavy chain);
(26) an antibody comprising an L chain having the amino acid sequence of SEQ ID NO: 90 as CDR1, the amino acid sequence of SEQ ID NO: 91 as CDR2, and the amino acid sequence of SEQ ID NO: 92 as CDR3 (22SD48 light chain);
(27) an antibody comprising the H chain of (25) and the L chain of (26) (22SD48);
(28) an antibody that has equivalent activity as the antibody of any one of (1) to (27);
(29) an antibody that recognizes the same epitope recognized by the antibody of any one of (1) to (27).

[10] The antibody of [1] to [9], which has a human constant region.
[11] The antibody of [10], which is a chimeric antibody, humanized antibody, or human antibody.
[12] The antibody of any one of [1] to [11], which is a fucose-deficient antibody.
[13] A pharmaceutical composition comprising the antibody of any one of [1] to [12] as an active ingredient.
[14] A cell growth inhibitor comprising the antibody of any one of [1] to [12] as an active ingredient.
[15] An anticancer agent comprising the antibody of any one of [1] to [12] as an active ingredient.
[16] The anticancer agent of [15], wherein the cancer to be treated is ovarian cancer.
[17] The anticancer agent of [16], wherein the ovarian cancer is clear cell adenocarcinoma.
[18] A method for diagnosing cancer, which comprises detecting an LGR7 protein or a gene encoding an LGR7 protein.
[19] A method for diagnosing cancer, which comprises detecting an LGR7 protein.
[20] The diagnostic method of [19], wherein the LGR7 protein is detected using an antibody that binds to the LGR7 protein.
[21] A method for diagnosing cancer, which comprises the steps of:

(a) providing a sample collected from a subject; and
(b) detecting an LGR7 protein contained in the sample of (a) using an antibody that binds to the LGR7 protein.

[22] A method for diagnosing cancer, which comprises the steps of:

(a) administering to a subject an antibody that has binding activity towards an LGR7 protein and which is labeled with a radioisotope; and

(b) detecting accumulation of the radioisotope.

[23] The diagnostic method of any one of [18] to [22], wherein the cancer to be diagnosed is ovarian cancer.
[24] The diagnostic method of [23], wherein the ovarian cancer is clear cell adenocarcinoma.

## Brief Description of the Drawings

Fig. 1-1 shows the expression profile of LGR7 (1552715_a_at) in ten cases of ovarian cancers including the following tissue types: clear cell carcinoma (four cases), serous adenocarcinoma (two cases), endometrioid adenocarcinoma (three cases), and carcinosarcoma (one case), as well as ten types of normal tissues, four types of fetal tissues, four types of ovarian cancer cell lines, and 87 ovarian cancer cases including three clear cell cancer cases from the U133 Plus 2.0 expression data published by the International Genomics Consortium (IGC). The letters indicated under the IGC sample names respectively refer to the following: C, clear cell adenocarcinoma; E, endometrioid adenocarcinoma; S, serous adenocarcinoma; M, mucinous adenocarcinoma; O, other cancers; and B, benign serous cystadenoma of borderline malignancy. JHOC-5, MCAS, RMG-1, RMUG-S, and TKY-nu are ovarian cancer cell lines, and among them, RMG-1 is a cell line derived from clear cell adenocarcinoma.
Fig. 1-2 shows the expression profile of LGR7 (1552715_a_at).
Fig. 1-3 shows the expression profile of LGR7 (1552715_a_at).
Fig. 2 presents a photograph showing the result of detecting LGR7 expression in HA-LGR7/BaF3#48 and HA-LGR7/DG44#24 by subjecting cell lysates to SDS-PAGE electrophoresis, and Western blotting using an anti-HA antibody (HA-7). Lane 1: BaF3; lane 2: HA-LGR7/BaF3#48; lane 3: DG44; and lane 4: HA-LGR7/DG44#24.
Fig. 3 shows the results of measuring ADCC activity by Cr release by using HA-LGR7/DG44 as the target cell and NK92mFcR3 as the effector cell.
Fig. 4 shows the results of measuring complement-dependent cytotoxicity by using HA-LGR7/DG44 as the target cell, and reacting it with an anti-LGR7 antibody together with a baby rabbit complement. The intensity of CDC activity was defined by the proportion of 7-AAD-incorporating cells to cells reacted with each antibody.
Fig. 5 shows the results of determining the number of viable cells by using HA-LGR7/DG44 as the target cell, and reacting it with Mab-ZAP (Saporin-labeled anti-mouse antibody) and each monoclonal antibody, and then performing WST8 assay. A higher value on the vertical axis indicates a larger number of viable cells.
Fig. 6 shows that 22DA17 and 22DA23 cross-react with mouse LGR7 based on the FACS analysis of HA-mLGR7/BaF3. The vertical axis indicates geometric mean values.
Fig. 7 shows the results of competition FACS analysis using the biotinylated antibodies Bio-22DA17 and Bio-22DA22. Whether antibodies recognize different epitopes was determined by observing the competition for the epitope by an unlabeled antibody. It was found that 22DA12 and 22DA22 are antibodies that recognize different epitopes from those of other antibodies. The solid lines indicate the results of reacting the biotinylated antibodies, and the shaded peaks indicate the results of reacting an FITC-recognizing anti-mouse antibody.
Fig. 8 shows the result of a drug efficacy test using a mouse xenograft model. In the graph, the tumor volume after antibody administration is plotted against the days post-tumor transplantation. The solid line shows the result of the PBS-administered group (negative control). The dashed line with squares shows the result of the group administered with 10 mg/kg of the FTKODA23 antibody, and the dashed line with triangles shows the result of the group administered with 2 mg/kg of the FTKODA23 antibody.

## Mode for Carrying Out the Invention

### LGR7

In the present invention, LGR7 is a member protein of the seven-transmembrane LGR family. The amino acid sequence of human LGR7 and the gene sequence encoding it are disclosed in NCBI accession numbers NP_067647 (SEQ ID NO: 1) and NM_021634 (SEQ ID NO: 2), respectively. LGR7 used in the present invention may be splice variants or mutants. In the present invention, "LGR7 protein" refers to both the full-length protein and a fragment thereof. Herein, "fragment" refers to a polypeptide comprising any region of the LGR7 protein, and it does not necessarily have the function of the naturally-occurring LGR7 protein. An example of the fragment is a fragment comprising an extracellular domain of the LGR7 protein. The extracellular domains of the LGR7 protein correspond to positions 1 to 404, 462 to 485, 549 to 581, and 648 to 661 in the amino acid sequence of SEQ ID NO: 1. The transmembrane regions correspond to positions 405 to 427, 439 to 461, 486 to 508, 529-548, 582 to 604, 625-647, and 662 to 681 in the amino acid sequence of SEQ ID NO: 1.

Preparation of the anti-LGR7 antibodies

The origin, type, form, and such of an anti-LGR7 antibody used in the present invention are not limited as long as it binds to the LGR7 protein. More specifically, known antibodies such as nonhuman animal antibodies (for example, mouse antibodies, rat antibodies, and camel antibodies), human antibodies, chimeric antibodies, humanized antibodies, and the like can be used. In the present invention, a monoclonal antibody is used. The binding between an antibody and the LGR7 protein is preferably specific. An antibody that recognizes human LGR7 may specifically recognize human LGR7, or it may at the same time recognize LGR7 derived from another animal (for example, mouse LGR7).

An anti-LGR7 antibody used in the present invention is obtained as a monoclonal antibody using known means. The anti-LGR7 antibody used in the present invention is preferably a mammal-derived monoclonal antibody in particular. The mammal-derived monoclonal antibodies include antibodies produced by a hybridoma and antibodies produced by a host transformed with an expression vector containing an antibody gene using a genetic engineering method.

Basically, monoclonal antibody-producing hybridomas can be prepared using known techniques as follows. First, immunization is carried out by a conventional immunization method using the LGR7 protein as the sensitizing antigen. Hybridomas are prepared by fusing immune cells obtained from an immunized animal with a known parental cell using a conventional cell fusion method. Next, a hybridoma producing an anti-LGR7 antibody can be selected from the hybridomas by screening for a cell producing the antibody of interest using a conventional screening method.

Specifically, monoclonal antibodies are prepared, for example, as shown below. First, the LGR7 protein to be used as the sensitizing antigen for producing an antibody can be obtained by expressing the LGR7 gene. The nucleotide sequence of the LGR7 gene is disclosed in NCBI accession number NM_021634 (SEQ ID NO: 2), etc. That is, a gene sequence encoding LGR7 is inserted into a known expression vector, and suitable host cells are transformed with this vector, and then the human LGR7 protein of interest can be purified from the host cells or culture supernatants using a known method. A purified naturally-occurring LGR7 protein can also be used. Furthermore, as utilized in the present invention, a fusion protein obtained by fusing a desired partial polypeptide of the LGR7 protein with a different polypeptide can also be used as the immunogen. For example, an antibody Fc fragment, peptide tag, or such can be used to produce a fusion protein as the immunogen. A vector expressing the fusion protein can be prepared by fusing genes encoding two or more desired polypeptide fragments in-frame, and inserting the fusion gene into an expression vector. Methods for preparing fusion proteins are described in Molecular Cloning, 2nd ed. (Sambrook J et al., Molecular Cloning 2nd ed., 9.47-9.58, Cold Spring Harbor Lab. Press, 1989).

An LGR7 protein thus purified can be used as a sensitizing antigen for use in immunizing a mammal. An LGR7 partial peptide can also be used as the sensitizing antigen. For example, the following peptides can be used as the sensitizing antigen:

a peptide obtained by chemical synthesis based on the amino acid sequence of human LGR7;
a peptide obtained by inserting a portion of the LGR7 gene into an expression vector and expressing it; and
a peptide obtained by protease degradation of the LGR7 protein.

There is no particular limitation on the region and size of LGR7 to be used as the partial peptide. A preferred region can be selected from the amino acid sequences constituting the extracellular domains of LGR7 (positions 1 to 404, 462 to 485, 549 to 581, and 648 to 661 in the amino acid sequence of SEQ ID NO: 1). The number of amino acids constituting a peptide to be used as the sensitizing antigen is preferably at least three or more, for example, five or more, or six or more. More specifically, a peptide having eight to 50 residues, preferably ten to 30 residues can be used as the sensitizing antigen.

There is no particular limitation on the mammal to be immunized with the sensitizing antigen. To obtain a monoclonal antibody by a cell fusion method, the animal to be immunized is preferably selected by considering the compatibility with the parental cell used for cell fusion. In general, rodents are preferable animals for immunization. More specifically, a mouse, rat, hamster, or rabbit can be used as the animal to be immunized. Alternatively, a monkey or such can be used as the animal to be immunized.

The above-mentioned animals can be immunized with the sensitizing antigen using a known method. In a general method, for example, a mammal can be immunized by intraperitoneal or subcutaneous injection of a sensitizing antigen. More specifically, the sensitizing antigen is administered to the mammal several times every four to 21 days. The sensitizing antigen is diluted at a suitable dilution ratio in phosphate-buffered saline (PBS), physiological saline, or such, and used for immunization. The sensitizing antigen can be administered together with an adjuvant. For example, the sensitizing antigen can be prepared by mixing and emulsifying with Freund's complete adjuvant. Furthermore, a suitable carrier can be used in immunization with the sensitizing antigen. In particular, when a partial peptide of low molecular weight is used as the sensitizing antigen, it is desirable to attach the sensitizing antigen peptide to a carrier protein such as albumin, keyhole limpet hemocyanin, and use this for immunization.

On the other hand, monoclonal antibodies can be obtained by DNA immunization. DNA immunization is a method that confers immune stimulation by administering into an immunized animal a vector DNA constructed in a form that allows an antigen protein-encoding gene (e.g., SEQ ID NO: 2) to be expressed in the immunized animal, and expressing the immunizing antigen in the body of the immunized animal. Compared to general immunization methods by which a protein antigen is administered, the following advantages can be expected from DNA immunization:

- immune stimulation can be provided while retaining the structure of a membrane protein such as LGR7; and
- there is no need to purify the immunizing antigen.

On the other hand, however, it is difficult to combine DNA immunization with an immune stimulatory means such as an adjuvant. It was predicted that since LGR7 has the structural feature of a seven-transmembrane conformation, it would be difficult to induce an immune response to LGR7 *in vivo* while retaining its naturally-occurring structure. Because of such structural feature, it was an unexpected result to actually have obtained by DNA immunization monoclonal antibodies that bind to LGR7, which is a protein belonging to the LGR family for which antibody production is difficult.

To obtain a monoclonal antibody by DNA immunization, a DNA expressing the LGR7 protein is first administered to an animal to be immunized. The LGR7-encoding DNA can be synthesized by known methods such as PCR. The obtained DNA is inserted into a suitable expression vector and administered to the animal to be immunized. A commercially available expression vector such as pcDNA3.1 can be used as the expression vector. Methods generally used can be employed to administer the vector into the body. For example, DNA immunization can be carried out by using a gene gun to shoot gold particles adsorbed with the expression vector into cells.

A mammal is thus immunized, and an increase in the level of a desired antibody in the serum is observed. Then, immune cells are collected from the mammal and subjected to cell fusion. In particular, spleen cells can be preferably used as the immune cells.

A mammalian myeloma cell is used as a cell to be fused with the above-mentioned immunocyte. The myeloma cells preferably comprise a suitable selection marker for screening. A selection marker confers characteristics to cells for their survival (or failure to survive) under a specific culturing condition. Hypoxanthine-guanine phosphoribosyltransferase deficiency (hereinafter abbreviated as HGPRT deficiency), and thymidine kinase deficiency (hereinafter abbreviated as TK deficiency) are known as selection markers. Cells having HGPRT or TK deficiency have hypoxanthine-aminopterin-thymidine sensitivity (hereinafter abbreviated as HAT sensitivity). HAT-sensitive cells cannot carry out DNA synthesis in a HAT selection medium, and are thus killed. However, when the cells are fused with normal cells, they can continue to synthesize DNA using the salvage pathway of the normal cells, and therefore they can grow in the HAT selection medium.

HGPRT-deficient and TK-deficient cells can be selected in a medium containing 6-thioguanine or 8-azaguanine (hereinafter abbreviated as 8AG), and 5'-bromodeoxyuridine, respectively. Normal cells are killed since they incorporate these pyrimidine analogs into their DNA. On the other hand, cells that are deficient in these enzymes can survive in the selection medium, since they cannot incorporate these pyrimidine analogs. Alternatively, a selection marker referred to as G418 resistance provides resistance to 2-deoxystreptamine-type antibiotics (gentamycin analogs) from the neomycin-resistance gene. Various types of myeloma cells that are suitable for cell fusion are known. For example, myeloma cells including the following cells can be used to produce the monoclonal antibodies of the present invention or as disclosed:

P3 (P3x63Ag8.653) (J. Immunol. (1979) 123, 1548-1550);
P3x63Ag8U.1 (Current Topics in Microbiology and Immunology (1978) 81, 1-7);
NS-1 (Kohler. G. and Milstein, C. Eur. J. Immunol. (1976) 6, 511-519);
MPC-11 (Margulies. D.H. et al., Cell (1976) 8, 405-415);
SP2/0 (Shulman, M. et al., Nature (1978) 276, 269-270);
FO (de St. Groth, S. F. et al., J. Immunol. Methods (1980) 35, 1-21);
S194 (Trowbridge, I. S. J. Exp. Med. (1978) 148, 313-323); and
R210 (Galfre, G. et al., Nature (1979) 277, 131-133).

Cell fusion of the above-mentioned immunocytes with myeloma cells is essentially performed according to a known method, for example, the method of Kohler and Milstein *et al.* (Kohler. G. and Milstein, C., Methods Enzymol. (1981) 73, 3-46).

More specifically, the above-mentioned cell fusion can be performed in a standard nutritional culture medium in the presence of, for example, a cell-fusion accelerator. A cell-fusion accelerator may be, for example, polyethylene glycol (PEG), Sendai virus (HVJ), or the like. If desired, an auxiliary agent such as dimethylsulfoxide can be added to further enhance fusion efficiency.

The ratio of immunocytes to myeloma cells used can be established at one's discretion. For example, the number of immunocytes is preferably set to one to ten times of that of myeloma cells. As a medium to be used for the above-mentioned cell fusion, for example, RPMI1640 medium and MEM medium, which are appropriate for the growth of the above-mentioned myeloma cell line, or other standard media that are used for this type of cell culture can be used. Moreover, a serum supplement solution such as fetal calf serum (FCS) can be added to the media.

Cell fusion is performed by thoroughly mixing predetermined amounts of the above-mentioned immunocytes and myeloma cells in the above-mentioned medium, adding and mixing with a PEG solution pre-heated to approximately 37°C, so as to form the desired fused cells (hybridomas). In the cell fusion method, for example, PEG with an average molecular weight of approximately 1000 to 6000 can generally be added at a concentration of 30 to 60% (w/v). Subsequently, the agent for cell fusion or the like which is unfavorable for the growth of hybridomas can be removed by successively adding an appropriate medium such as those listed above, removing the supernatant after centrifugation, and repeating these steps.

Hybridomas obtained in this manner can be selected using a selection medium appropriate for the selection markers carried by myelomas used for cell fusion. For example, cells that have HGPRT and TK deficiencies can be selected by culturing them in a HAT medium (a medium containing hypoxanthine, aminopterin, and thymidine). More specifically, when HAT-sensitive myeloma cells are used for cell fusion, cells that successfully fuse with normal cells can be selectively grown in the HAT medium. Culturing using the above-mentioned HAT medium is continued for a sufficient period of time to kill the cells other than the hybridoma of interest (non-fused cells). More specifically, the hybridoma of interest can be selected, typically by culturing for several days to several weeks. Subsequently, hybridomas that produce the antibody of interest can be screened and single isolated by carrying out a standard limiting dilution method. Alternatively, a LGR7-recognizing antibody can be prepared using the method described in International Patent Publication No. WO 03/104453.

Screening for an antibody of interest and cloning can be suitably carried out by a screening method based on known antigen-antibody reactions. For example, an antigen is bound to a carrier such as beads made of polystyrene or the like, or a commercially available 96-well microplate or such, and reacted with the culture supernatant of a hybridoma. Next, after rinsing away the carrier, a secondary antibody labeled with an enzyme, or such is reacted. If the antibody of interest that reacts with the sensitizing antigen is contained in the culture supernatant, the secondary antibody will bind to the carrier via the antibody. Finally, whether or not the antibody of interest is present in the culture supernatant can be determined by detecting the secondary antibody bound to the carrier. A hybridoma that produces a desired antibody having binding ability towards the antigen can be cloned using the limiting dilution method or such. Herein, as an antigen, the antigen used for immunization, or an LGR7 protein that is substantially identical thereto can be suitably used. For example, a cell line expressing LGR7, an extracellular domain of LGR7, or an oligopeptide that comprises a partial amino acid sequence constituting the domain can be used as the antigen.

Alternatively, instead of using the above method of obtaining the hybridoma by immunizing a non-human animal with an antigen, the antibody of interest can be obtained by sensitizing human lymphocytes with the antigen. More specifically, first, human lymphocytes are sensitized by the LGR7 protein *in vitro.* Then, the immunosensitized lymphocytes are fused with a suitable fusion partner. For example, human-derived myeloma cells that can divide indefinitely can be used as the fusion partner (see Japanese Patent Application Kokoku Publication No. (JP-B) H01-59878 (examined, approved Japanese patent application published for opposition)). The anti-LGR7 antibody obtained by this method is a human antibody having the activity to bind to the LGR7 protein.

Alternatively, an anti-LGR7 human antibody can be obtained by administering the LGR7 protein which serves as an antigen to a transgenic animal having the complete repertoire of human antibody genes, or by immunizing such an animal with a DNA constructed to express LGR7 in the animal. Antibody-producing cells from the immunized animal can be immortalized by cell fusion with a suitable fusion partner or treatment such as Epstein-Barr virus infection. A human antibody against the LGR7 protein can be isolated from the immortalized cells obtained in this manner (see International Publications WO94/25585, WO93/12227, WO92/03918, and WO94/02602). By further cloning the immortalized cells, it is possible to clone cells that produce an antibody having the reaction specificity of interest. When using a transgenic animal as the immunized animal, the immune system of the animal recognizes human LGR7 as a foreign substance. Thus, a human antibody against human LGR7 can be easily obtained.

The monoclonal antibody-producing hybridomas produced in this manner can be passaged and cultured in a standard medium. Alternatively, the hybridomas can be stored for a long period in liquid nitrogen.

The hybridomas can be cultured according to a standard method, and the monoclonal antibody of interest can be obtained from the culture supernatants. Alternatively, the hybridomas can be grown by administering them to a compatible mammal, and monoclonal antibodies can be obtained as its ascites. The former method is suitable for obtaining highly purified antibodies.

In the present invention, an antibody encoded by an antibody gene cloned from antibody-producing cells can be used. The cloned antibody gene can be incorporated into a suitable vector and then introduced into a host to express the antibody. Methods for isolating an antibody gene, introducing the gene into a vector, and transforming host cells have been established (see for example, Vandamme, A. M. et al., Eur. J. Biochem. (1990) 192, 767-775).

For example, a cDNA encoding the variable region (V region) of an anti-LGR7 antibody can be obtained from hybridoma cells producing the anti-LGR7 antibody. Usually, in order to accomplish this, first, total RNA is extracted from the hybridoma. For example, the following methods can be used as methods for extracting mRNA from cells:

the guanidine ultracentrifugation method (Chirgwin, J. M. et al., Biochemistry (1979) 18, 5294-5299); and
the AGPC method (Chomczynski, P. et al., Anal. Biochem. (1987) 162, 156-159).

The extracted mRNA can be purified using an mRNA purification kit (manufactured by GE Healthcare Biosciences) or the like. Alternatively, kits such as the QuickPrep mRNA Purification Kit (manufactured by GE Healthcare Biosciences) for direct extraction of total mRNA from cells are commercially available. Total mRNA can be obtained from a hybridoma using such a kit. A cDNA encoding the antibody V region can be synthesized from the obtained mRNA using a reverse transcriptase. An arbitrary sequence of 15 to 30 bases selected from a sequence common to the mouse antibody genes can be used as a primer. Specifically, a cDNA encoding the antibody V region can be obtained using primers having the DNA sequences of SEQ ID NOs: 97 to 100. A cDNA can be synthesized using the AMV Reverse Transcriptase First-strand cDNA Synthesis Kit (manufactured by Seikagaku Corporation), etc. Alternatively, the 5'-Ampli FINDER RACE Kit (manufactured by Clontech) and the 5'-RACE method which utilizes PCR (Frohman, M. A. et al., Proc. Natl. Acad. Sci. USA (1988) 85, 8998-9002; Belyavsky, A. et al., Nucleic Acids Res. (1989) 17, 2919-2932) can be used for synthesizing and amplifying cDNA. Additionally, during the process of cDNA synthesis, suitable restriction enzyme sites mentioned below can be introduced into both ends of the cDNA.
The cDNA fragment of interest is purified from the obtained PCR product and then ligated to a vector DNA. The recombinant vector is thus prepared, and introduced into *Escherichia coli* (*E. coli*) and the like, and colonies are selected. Then, a desired recombinant vector can be prepared from the colony-forming *E. coli*. The nucleotide sequence of the cDNA can be verified by a known method such as the dideoxynucleotide chain termination method.
Furthermore, a cDNA library can be used to obtain a gene encoding an antibody variable region. First, cDNAs are synthesized using mRNAs extracted from antibody-producing cells as a template to obtain a cDNA library. A commercially available kit is conveniently used for synthesis of the cDNA library. In practice, the quantity of mRNAs obtained from only few cells is very small; thus, the yield is low if the mRNAs are purified directly. Therefore, purification is usually carried out after addition of a carrier RNA which clearly does not contain an antibody gene. Alternatively, when a certain quantity of RNAs can be extracted, the RNAs of antibody-producing cells themselves can be efficiently extracted. For example, addition of carrier RNA is not necessary in some cases when RNAs are extracted from 10 or more, or 30 or more, or preferably 50 or more antibody-producing cells.
The antibody gene is amplified by the PCR method using the obtained cDNA library as a template. Primers for amplifying an antibody gene by the PCR method are known. For example, it is possible to design primers for amplifying a human antibody gene based on the disclosure of the article (J. Mol. Biol. (1991), 222, 581-597), and the like. These primers have different nucleotide sequences depending on the immunoglobulin subclass. Therefore, all possibilities should be taken into consideration when the PCR method is carried out using a cDNA library of unknown subclass as a template.
Specifically, for example, when the objective is to obtain a gene encoding human IgG, one can use primers capable of amplifying genes encoding $\gamma$1 to y5 as the heavy chain, and $\kappa$ and $\lambda$ chains as the light chain. To amplify an IgG variable region gene, a primer that anneals to a portion corresponding to the hinge region is generally used as the 3'-end primer. Meanwhile, a primer for the individual subclass can be used as the 5'-end primer.
The PCR products produced by gene amplification primers for each heavy- or light-chain subclass are made into independent libraries. Using the libraries thus synthesized, immunoglobulins comprising a combination of heavy and light chains can be reconstructed. The antibody of interest can be screened using the binding activity of a reconstructed immunoglobulin toward LGR7 as an indicator.
For example, when the objective is to obtain an antibody against LGR7, it is more preferable that the binding between the antibody and LGR7 is specific. An LGR7-binding antibody may be screened, for example, by the following steps:

(1) contacting LGR7 with an antibody comprising a V region encoded by an obtained cDNA;
(2) detecting the binding between LGR7 and the antibody; and
(3) selecting an antibody that binds to LGR7.

A method for detecting the binding between an antibody and LGR7 is known. More specifically, a test antibody is reacted with LGR7 immobilized onto a carrier, and then a labeled antibody that recognizes the antibody is reacted. If the labeled antibody on the carrier is detected after washing, then the binding between the test antibody and LGR7 can be verified. Enzymatically active proteins such as peroxidase and $\beta$-galactosidase, or fluorescent substances such as FITC can be used for the label. A fixed specimen of LGR7-expressing cells can be used to evaluate the binding activity of the antibody.
Alternatively, for an antibody screening method based on the binding activity, a phage vector-based panning method may be used. When the antibody genes are obtained as libraries of the heavy-chain and light-chain subclasses from polyclonal antibody-expressing cells as mentioned above, phage displaying methods are advantageous. Genes encoding variable regions of the heavy and light chains can be made into a single-chain Fv (scFv) gene by linking the genes via suitable linker sequences. Phages expressing an scFv on their surface can be obtained by inserting a gene encoding the scFv into a phagemid vector. DNA encoding an scFv having the binding activity of interest can be collected by contacting the phage with an antigen of interest, and then collecting antigen-bound phage. scFv having the binding activity of interest can be enriched by repeating this operation as necessary.

An antibody-encoding polynucleotide may encode a full-length antibody or a portion of the antibody. "A portion of an antibody" refers to any portion of an antibody molecule. Hereinafter, the term "antibody fragment" may be used to refer to a portion of an antibody. A preferred antibody fragment of the present invention comprises the complementarity determination region (CDR) of an antibody. More preferably, an antibody fragment of the present invention comprises all of the three CDRs that constitute a variable region.

Once a cDNA encoding the V region of an anti-LGR7 antibody of interest is obtained, this cDNA is digested with restriction enzymes that recognize the restriction enzyme sites inserted to both ends of the cDNA. A preferred restriction enzyme recognizes and digests a nucleotide sequence that is less likely to appear in the nucleotide sequence constituting the antibody gene. Furthermore, to insert a single copy of the digested fragment into a vector in the correct direction, a restriction enzyme that provides sticky ends is preferable. A cDNA encoding the anti-LGR7 antibody V region, which has been digested as described above, is inserted into a suitable expression vector to obtain the antibody expression vector. In this step, a chimeric antibody can be obtained by fusing a gene encoding the antibody constant region (C region) with the above-mentioned gene encoding the V region in frame. Herein, "chimeric antibody" refers to an antibody whose constant and variable regions are derived from different origins. Therefore, in addition to interspecies chimeric antibodies such as mouse-human chimeric antibodies, human-human intraspecies chimeric antibodies are also included in the chimeric antibodies of the present invention. A chimeric antibody expression vector can also be constructed by inserting the V-region gene into an expression vector into which a constant region-encoding gene has been introduced.

More specifically, for example, the restriction enzyme recognition sequence for a restriction enzyme that digests the V-region gene can be placed at the 5' end of a DNA encoding a desired antibody constant region (C region) in an expression vector. The chimeric antibody expression vector is constructed by digesting the two vectors using the same combination of restriction enzymes, and fusing them in frame.

To produce an anti-LGR7 antibody, the antibody gene can be incorporated into an expression vector so that it is expressed under the regulation of an expression control region. The expression regulatory region for antibody expression includes, for example, an enhancer or a promoter. Then, by transforming suitable host cells with this expression vector, recombinant cells that carry the DNA expressing the anti-LGR7 antibody can be obtained.

To express an antibody gene, a DNA encoding the antibody heavy chain (H-chain) and a DNA encoding the antibody light chain (L-chain) can be incorporated separately into expression vectors. An antibody molecule comprising the H-chain and L-chain can be expressed by simultaneously transfecting (co-transfecting) the H-chain and L-chain-incorporated vectors into the same host cell. Alternatively, DNAs encoding the H-chain and L-chain can be incorporated into a single expression vector to transform a host cell with the vector (see International Patent Publication No. WO 94/11523).

Many combinations of hosts and expression vectors for introducing an isolated antibody gene into an appropriate host to produce the antibody are known. Any of these expression systems can be applied. When using eukaryotic cells as a host, animal cells, plant cells, and fungal cells can be used. More specifically, animal cells that may be used are, for example, the following cells:

(1) mammalian cells such as CHO, COS, myeloma, baby hamster kidney (BHK), HeLa, Vero, HEK293, Ba/F3, HL-60, Jurkat, and SK-HEP1 cells;
(2) amphibian cells such as Xenopus oocytes; and
(3) insect cells such as sf9, sf21, Tn5.

In addition, as a plant cell system, an antibody gene expression system using cells derived from the *Nicotiana* genus such as *Nicotiana tabacum* is known. Callus-cultured cells can be used to transform plant cells.

Furthermore, the following cells can be used as fungal cells; yeasts: the *Saccharomyces* genus, for example, *Saccharomyces cerevisiae,* and the *Pichia* genus, for example, *Pichia pastoris*; and filamentous fungi: the *Aspergillus* genus, for example, *Aspergillus niger.*

Antibody gene expression systems that utilize prokaryotic cells are also known. For example, when using bacterial cells, *E. coli* cells, *Bacillus subtilis* cells, and such may be used.

For mammalian cells, the antibody genes can be expressed by operatively placing the antibody gene just behind a commonly used effective promoter, and a polyA signal on the 3' downstream side of the antibody gene. An example of such promoter/enhancer is human cytomegalovirus immediate early promoter/enhancer.

Other promoters/enhancers that can be used for antibody expression include viral promoters/enhancers, or mammalian cell-derived promoters/enhancers such as human elongation factor 1α (HEF1α). Specific examples of viruses whose promoters/enhancers may be used include retrovirus, polyoma virus, adenovirus, and simian virus 40 (SV40).

When an SV40 promoter/enhancer is used, the method of Mulligan et al. (Nature (1979) 277, 108) may be utilized. An HEF1α promoter/enhancer can be readily used for expressing a gene of interest by the method of Mizushima et al. (Nucleic Acids Res. (1990) 18, 5322).

In the case of *E. coli,* the antibody can be expressed by operatively placing the antibody gene with a signal sequence for secretion at downstream of a commonly used effective promoter . Examples of such promoter include the lacZ promoter and araB promoter. For the lacZ promoter, the method of Ward et al., (Nature (1989) 341, 544-546; FASEB J. (1992) 6, 2422-2427) may be used. Alternatively, the araB promoter can be used for expressing a gene of interest by the method of Better et al. (Science (1988) 240, 1041-1043).

The pelB signal sequence for secretion (Lei, S. P. et al., J. Bacteriol. (1987) 169, 4379) may be used for antibody production in the periplasm of *E. coli.* After isolation of the antibody produced in the periplasm, the antibody can be refolded by using a protein denaturant like guanidine hydrochloride or urea so that the antibody will have the desired binding activity.

When the antibody is produced using animal cells, it is desirable to use a signal sequence of an antibody heavy- or light-chain gene as the signal sequence necessary for secretion to the outside of the cells. Alternatively, the signal sequences possessed by secretory proteins such as IL-3 and IL-6 can be used.

A replication origin derived from SV40, a polyomavirus, adenovirus, bovine papilloma virus (BPV), or such can be used and inserted into an expression vector. Additionally, a selection marker can be inserted into the expression vector for increasing the gene copy number in the host cell system. More specifically, the following selection markers can be used:

aminoglycoside transferase (APH) gene;
thymidine kinase (TK) gene;
*E. coli* xanthine guanine phosphoribosyl transferase (Ecogpt) gene; and
dihydrofolate reductase (dhfr) gene, etc.

The antibody of interest is produced by introducing these expression vectors into host cells, and then culturing the transformed host cells either *in vitro* or *in vivo.* The host cells are cultured according to known methods. For example, DMEM, MEM, RPMI 1640, or IMDM can be used as the liquid culture medium, and a serum-based supplement such as fetal calf serum (FCS) can be used in combination.

The antibody expressed and produced as described above can be purified by using known methods conventionally used for protein purification either singly or in a suitable combination. For example, the antibody can be separated and purified by suitably selecting and combining an affinity column such as a protein A column, a chromatography column, a filter, ultrafiltration, salting-out, dialysis, and such (Antibodies: A Laboratory Manual. Ed Harlow, David Lane, Cold Spring Harbor Laboratory, 1988).

In addition to the above host cells, transgenic animals can also be used to produce a recombinant antibody. That is, the antibody can be obtained from an animal into which the gene encoding the antibody of interest is introduced. For example, the antibody gene can be inserted in frame into a gene that encodes a protein produced inherently in milk to construct a fused gene. Goat β-casein or such can be used, for example, as the protein secreted in milk. A DNA fragment containing the fused gene inserted with the antibody gene is injected into a goat embryo, and then this embryo is introduced into a female goat. Desired antibodies can be obtained as a protein fused with the milk protein from milk produced by the transgenic goat born from the goat that received the embryo (or progeny thereof). To increase the volume of milk containing the desired antibody produced by the transgenic goat, hormones can be used on the transgenic goat as necessary (Ebert, K. M. et al., Bio/Technology (1994) 12, 699-702).

Non-human animal-derived antibody C regions can be used for the C regions of a recombinant antibody. For example, Cγ1, Cγ2a, Cγ2b, Cγ3, Cμ, C8, Cα1, Cα2, and Cε can be used for the mouse antibody H-chain C-region, and Cκ and Cλ can be used for the L-chain C-region. In addition, antibodies of animals other than mice such as rats, rabbits, goat, sheep, camels, and monkeys can be used. Their sequences are known. Furthermore, the C region can be modified to improve the stability of the antibodies or their production.

When administering antibodies to humans, genetically recombinant antibodies that have been artificially modified for the purpose of reducing xenoantigenicity against humans, or the like can be used. Examples of the genetically recombinant antibodies include chimeric antibodies and humanized antibodies. These modified antibodies can be produced using known methods.

A chimeric antibody is an antibody whose variable regions and constant regions are of different origins. For example, an antibody comprising the heavy-chain and light-chain variable regions of a mouse antibody and the heavy-chain and light-chain constant regions of a human antibody is a mouse-human interspecies chimeric antibody. A recombinant vector expressing a chimeric antibody can be produced by ligating a DNA encoding a mouse antibody variable region to a DNA encoding a human antibody constant region, and then inserting it into an expression vector. The recombinant cells that have been transformed with the vector are cultured, and the incorporated DNA is expressed to obtain the chimeric antibody produced in the culture. Human C regions are used for the C regions of chimeric antibodies and humanized antibodies.

For example, Cγ1, Cγ2, Cγ3, Cγ4, Cμ, Cδ, Cα1, Cα2, and Cε can be used as an H-chain C region. Cκ and Cλ can be used as an L-chain C region. The amino acid sequences of these C regions and the nucleotide sequences encoding them are known. Furthermore, the human antibody C region can be modified to improve the stability of an antibody or its production.

Generally, a chimeric antibody consists of the V region of an antibody derived from a non-human animal, and a C region derived from a human antibody. On the other hand, a humanized antibody consists of the complementarity determining region (CDR) of an antibody derived from a non-human animal, and the framework region (FR) and C region derived from a human antibody. Since the antigenicity of a humanized antibody in human body is reduced, a humanized antibody is useful as an active ingredient for the therapeutic agents.

The antibody variable region generally comprises three complementarity-determining regions (CDRs) separated by four framework regions (FRs). CDR is a region that substantially determines the binding specificity of an antibody. The amino acid sequences of CDRs are highly diverse. On the other hand, the FR-constituting amino acid sequences are often highly homologous even among antibodies with different binding specificities. Therefore, generally, it is said that the binding specificity of a certain antibody can be transferred to another antibody by CDR grafting.

A humanized antibody is also called a reshaped human antibody. Specifically, humanized antibodies prepared by grafting the CDR of a non-human animal antibody such as a mouse antibody to a human antibody and such are known. Common genetic engineering technologies for obtaining humanized antibodies are also known.

Specifically, for example, overlap extension PCR is known as a method for grafting a mouse antibody CDR to a human FR. In overlap extension PCR, a nucleotide sequence encoding a mouse antibody CDR to be grafted is added to the primers for synthesizing a human antibody FR. Primers are prepared for each of the four FRs. It is generally said that when grafting a mouse CDR to a human FR, selecting a human FR that is highly homologous to a mouse FR is advantageous for maintaining the CDR function. That is, it is generally preferable to use a human FR comprising an amino acid sequence highly homologous to the amino acid sequence of the FR adjacent to the mouse CDR to be grafted.

Nucleotide sequences to be ligated are designed so that they will be connected to each other in frame. Human FRs are individually synthesized using the respective primers. As a result, products in which the mouse CDR-encoding DNA is attached to the individual FR-encoding DNAs are obtained. Nucleotide sequences encoding the mouse CDR of each product are designed so that they overlap with each other. Then, overlapping CDR regions of the products synthesized using a human antibody gene as the template are annealed for complementary strand synthesis reaction. By this reaction, human FRs are ligated through the mouse CDR sequences.

The full length of the V-region gene, in which three CDRs and four FRs are ultimately ligated, is amplified using primers that anneal to its 5' and 3' ends and which have suitable restriction enzyme recognition sequences. A vector for human antibody expression can be produced by inserting the DNA obtained as described above and a DNA that encodes a human antibody C region into an expression vector so that they will ligate in frame. After transfecting this vector into a host to establish recombinant cells, the recombinant cells are cultured, and the DNA encoding the humanized antibody is expressed to produce the humanized antibody in the culture of the cells (see, European Patent Publication No. EP 239,400, and International Patent Publication No. WO 96/02576).

By qualitatively or quantitatively measuring and evaluating the antigen-binding activity of the humanized antibody produced as described above, one can suitably select human antibody FRs that allow CDRs to form a favorable antigen-binding site when ligated through the CDRs. As necessary, amino acid residues in an FR may be substituted so that the CDRs of a reshaped human antibody form an appropriate antigen-binding site. For example, amino acid sequence mutations can be introduced into FRs by applying the PCR method used for fusing a mouse CDR with a human FR. More specifically, partial nucleotide sequence mutations can be introduced into primers that anneal to the FR sequence. Nucleotide sequence mutations are introduced into the FRs synthesized using such primers. Mutant FR sequences having the desired characteristics can be selected by measuring and evaluating the activity of the amino acid-substituted mutant antibody to bind to the antigen by the above-mentioned method (Sato, K. et al., Cancer Res. 1993, 53, 851-856).

Methods for obtaining human antibodies are also known. For example, human lymphocytes are sensitized *in vitro* with a desired antigen or cells expressing a desired antigen. Next, the desired human antibody which has the activity to bind to the antigen can be obtained by fusing a sensitized lymphocyte with a human myeloma cell (see JP-B H01-59878). For example, U266 and the like can be used as the human myeloma cell which serves as the fusion partner.

In addition, a desired human antibody can be obtained by immunizing a transgenic animal having the complete repertoire of human antibody genes with a desired antigen (see International Publication Nos. WO 93/12227, WO 92/03918, WO 94/02602, WO 94/25585, WO 96/34096, and WO 96/33735). Furthermore, technologies for obtaining a human antibody by panning using a human antibody library are known. For example, a human antibody V region can be expressed on the surface of a phage as a single-chain antibody (scFv) by the phage display method, and a phage that binds to the antigen can be selected. The DNA sequence encoding the V region of the human antibody that binds to the antigen can be determined by analyzing the genes of the selected phage. After the DNA sequence of the scFv that binds to the antigen is determined, an expression vector can be prepared by fusing the V-region sequence in-frame with the sequence of a desired human antibody C region, and then inserting this into a suitable expression vector. The expression vector is introduced into suitable expression cells such as those described above, and the human antibody can be obtained by expressing the human antibody-encoding gene. These methods are already known (International Publication Nos. WO 92/01047, WO 92/20791, WO 93/06213, WO 93/11236, WO 93/19172, WO 95/01438, and WO 95/15388).

Therefore, in a preferred embodiment, the antibodies used may include an antibody comprising a human constant region.

The antibodies of the present invention or as disclosed are not limited to bivalent antibodies represented by IgG, but include monovalent antibodies and multivalent antibodies represented by IgM, as long as it binds to the LGR7 protein. The multivalent antibody includes a multivalent antibody that has the same antigen binding sites, and a multivalent antibody that has partially or completely different antigen binding sites. The antibody is not limited to the whole antibody molecule, but includes low-molecular-weight antibodies (minibodies) and modified products thereof, as long as they bind to the LGR7 protein.

A low-molecular-weight antibody contains an antibody fragment lacking a portion of a whole antibody (for example, whole IgG). As long as it has the ability to bind the LGR7 antigen, partial deletions of an antibody molecule are permissible. Antibody fragments of the present invention preferably contain a heavy-chain variable region (VH) and/or a light-chain variable region (VL). Furthermore, antibody fragments of the present invention preferably contain CDR. The amino acid sequence of VH or VL may have substitutions, deletions, additions, and/or insertions. Furthermore, as long as it has the ability to bind the LGR7 antigen, VH and/or VL can be partially deleted. The variable region may be chimerized or humanized. Specific examples of the antibody fragments include Fab, Fab', F(ab')2, and Fv. Specific examples of low-molecular-weight antibodies include Fab, Fab', F(ab')2, Fv, scFv (single chain Fv), diabody, sc(Fv)2 (single chain (Fv)2), and scFv-Fc. Multimers of these antibodies (for example, dimers, trimers, tetramers, and polymers) are also included in the low-molecular-weight antibodies.

Fragments of antibodies can be obtained by treating an antibody with an enzyme to produce antibody fragments. Known enzymes that produce antibody fragments are, for example, papain, pepsin, and plasmin. Alternatively, genes encoding these antibody fragments can be constructed, introduced into expression vectors, and then expressed in appropriate host cells (see, for example, Co, M. S. et al., J. Immunol. (1994) 152, 2968-2976; Better, M. and Horwitz, A. H., Methods in Enzymology (1989) 178, 476-496; Plueckthun, A. and Skerra, A., Methods in Enzymology (1989) 178, 476-496; Lamoyi, E., Methods in Enzymology (1989) 121, 652-663; Rousseaux, J. et al., Methods in Enzymology (1989) 121, 663-669; and Bird, R. E. et al., TIBTECH (1991) 9, 132-137).

Digestive enzymes cleave specific sites of an antibody fragment, and yield antibody fragments with the following specific structures. When genetic engineering technologies are used on such enzymatically obtained antibody fragments, any portion of the antibody can be deleted.

Papain digestion: F(ab)2 or Fab
Pepsin digestion: F(ab')2 or Fab'
Plasmin digestion: Facb

Therefore, low-molecular-weight antibodies may be antibody fragments lacking any region, as long as they have binding affinity to LGR7. Furthermore, according to the present invention, the antibodies desirably maintain their effector activity, particularly in the treatment of cell proliferative diseases such as cancer. More specifically, preferred low-molecular-weight antibodies have both binding affinity to LGR7 and effector function. The antibody effector function includes ADCC activity and CDC activity. Particularly preferably, therapeutic antibodies of the present invention have ADCC activity and/or CDC activity as effector function.

A diabody refers to a bivalent antibody fragment constructed by gene fusion (Hollinger P. et al., Proc. Natl. Acad. Sci. USA 90: 6444-6448 (1993); EP 404,097; WO 93/11161; and such). A diabody is a dimer composed of two polypeptide chains. Generally, in each polypeptide chain constituting the dimer, VL and VH are linked by a linker within the same chain. The linker in a diabody is generally short enough to prevent binding between VL and VH. Specifically, the amino acid residues constituting the linker are, for example, five residues or so. Therefore, VL and VH that are encoded by the same polypeptide chain cannot form a single-chain variable region fragment, and form a dimer with another single chain variable region fragment. As a result, diabodies have two antigen binding sites.

scFv can be obtained by ligating the H-chain V region and L-chain V region of an antibody. In scFv, the H-chain V region and L-chain V region are ligated via a linker, preferably a peptide linker (Huston, J. S. et al., Proc. Natl. Acad. Sci. U.S.A., 1988, 85, 5879-5883). The H-chain V region and L-chain V region of scFv may be derived from any of the antibodies described herein. The peptide linker for ligating the V regions is not particularly limited. For example, any single-chain peptide consisting of 3 to 25 residues or so can be used as the linker. More specifically, for example, peptide linkers described below or such can be used.

PCR methods such as those described above can be used for ligating the V regions of both chains. For ligation of the V regions by PCR methods, first, a whole DNA or a DNA encoding a desired partial amino acid sequence selected from the following DNAs can be used as a template:

a DNA sequence encoding the H chain or the H-chain V region of the antibody; and
a DNA sequence encoding the L chain or the L-chain V region of the antibody.

DNAs encoding the H-chain and L-chain V regions are individually amplified by PCR methods using a pair of primers that have sequences corresponding to the sequences of both ends of the DNA to be amplified. Then, a DNA encoding the peptide linker portion is prepared. The DNA encoding the peptide linker can also be synthesized using PCR. To the 5' end of the primers used, nucleotide sequences that can be ligated to each of the individually synthesized V-region amplification products are added. Then, PCR reaction is carried out using the "H-chain V region DNA", "peptide linker DNA", and "L-chain V region DNA", and the primers for assembly PCR.

The primers for assembly PCR consist of the combination of a primer that anneals to the 5' end of the "H-chain V region DNA" and a primer that anneals to the 3' end of the "L-chain V region DNA". That is, the primers for assembly PCR are a primer set that can amplify a DNA encoding the full-length sequence of scFv to be synthesized. On the other hand, nucleotide sequences that can be ligated to each V-region DNA are added to the "peptide linker DNA". Thus, these DNAs are ligated, and the full-length scFv is ultimately produced as an amplification product using the primers for assembly PCR. Once the scFv-encoding DNA is constructed, expression vectors containing the DNA, and recombinant cells transformed by these expression vectors can be obtained according to conventional methods. Furthermore, the scFvs can be obtained by culturing the resulting recombinant cells and expressing the scFv-encoding DNA.

scFv-Fc is a low-molecular-weight antibody produced by fusing an Fc domain to an scFV comprising the H-chain V region and L-chain V region of an antibody (Cellular & Molecular Immunology 2006; 3: 439-443). While there is no particular limitation on the origin of the scFv used for scFv-Fc, for example, scFv derived from IgM may be used. Furthermore, while there is no particular limitation on the origin of Fc, for example, mouse IgG (mouse IgG2a and such) and human IgG (human IgG1 and such) may be used. Therefore, in a preferred embodiment, examples of scFv-Fc include an scFv-Fc produced by linking the scFv fragment of an IgM antibody to CH2 (for example, Cγ2) and CH3 (for example, Cγ3) of mouse IgG2a with the hinge region (Hγ) of mouse IgG2a, and an scFv-Fc produced by linking the scFv fragment of an IgM antibody to CH2 and CH3 of human IgG1 with the hinge region of human IgG1.

sc(Fv)2 is a minibody prepared by ligating two VHs and two VLs with linkers or such to form a single chain (Hudson et al., J. Immunol. Methods 1999; 231: 177-189). sc(Fv)2 can be produced, for example, by joining scFvs with a linker.

Moreover, antibodies in which two VHs and two VLs are arranged in the order of VH, VL, VH, and VL ([VH]-linker-[VL]-linker-[VH]-linker-[VL]), starting from the N-terminal side of a single chain polypeptide, are preferred.

The order of the two VHs and the two VLs is not particularly limited to the above-mentioned arrangement, and they may be placed in any order. Examples include the following arrangements:

[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

Any arbitrary peptide linker can be introduced by genetic engineering, and synthetic linkers (see, for example, those disclosed in Protein Engineering, 9(3), 299-305, 1996) or such can be used as linkers for linking the antibody variable regions. In the present invention, peptide linkers are preferable. The length of the peptide linkers is not particularly limited, and can be suitably selected by those skilled in the art according to the purpose. The length of amino acid residues composing a peptide linker is generally 1 to 100 amino acids, preferably 3 to 50 amino acids, more preferably 5 to 30 amino acids, and particularly preferably 12 to 18 amino acids (for example, 15 amino acids).

Any amino acid sequences composing peptide linkers can be used, as long as they do not inhibit the binding activity of scFv. Examples of the amino acid sequences used in peptide linkers include:

Ser
Gly-Ser
Gly-Gly-Ser

Ser-Gly-Gly
Gly-Gly-Gly-Ser (SEQ ID NO: 101)
Ser-Gly-Gly-Gly (SEQ ID NO: 102)
Gly-Gly-Gly-Gly-Ser (SEQ ID NO: 103)
Ser-Gly-Gly-Gly-Gly (SEQ ID NO: 104)
Gly-Gly-Gly-Gly-Gly-Ser (SEQ ID NO: 105)
Ser-Gly-Gly-Gly-Gly-Gly (SEQ ID NO: 106)
Gly-Gly-Gly-Gly-Gly-Gly-Ser (SEQ ID NO: 107)
Ser-Gly-Gly-Gly-Gly-Gly-Gly (SEQ ID NO: 108)
(Gly-Gly-Gly-Gly-Ser (SEQ ID NO: 101))n
(Ser-Gly-Gly-Gly-Gly (SEQ ID NO: 102))n

in which n is an integer of 1 or larger.

The amino acid sequences of the peptide linkers can be selected appropriately by those skilled in the art according to the purpose. For example, n, which determines the length of the peptide linkers, is generally 1 to 5, preferably 1 to 3, more preferably 1 or 2.

Therefore, a particularly preferred embodiment of sc(Fv)2 in the present invention is, for example, the following sc(Fv)2:

[VH]-peptide linker (15 amino acids)-[VL]-peptide linker (15 amino acids)-[VH]-peptide linker (15 amino acids)-[VL]

Alternatively, synthetic chemical linkers (chemical crosslinking agents) can be used to link the V regions. Crosslinking agents routinely used to crosslink peptide compounds and such can be used in the present invention. For example, the following chemical crosslinking agents are known. These crosslinking agents are commercially available:

N-hydroxy succinimide (NHS);
disuccinimidyl suberate (DSS);
bis(sulfosuccinimidyl) suberate (BS3);
dithiobis(succinimidyl propionate) (DSP);
dithiobis(sulfosuccinimidyl propionate) (DTSSP);
ethylene glycol bis(succinimidyl succinate) (EGS);
ethylene glycol bis(sulfosuccinimidyl succinate) (sulfo-EGS);
disuccinimidyl tartrate (DST);
disulfosuccinimidyl tartrate (sulfo-DST);
bis[2-(succinimidoxycarbonyloxy)ethyl] sulfone (BSOCOES); and
bis[2-(sulfosuccinimidoxycarbonyloxy)ethyl] sulfone (sulfo-BSOCOES).

Usually, three linkers are required to link four antibody variable regions. The multiple linkers to be used may all be of the same type or different types. In the present invention, a preferred minibody is a diabody or an sc(Fv)2. Such minibody can be obtained by treating an antibody with an enzyme, such as papain or pepsin, to generate antibody fragments, or by constructing DNAs that encode these antibody fragments, introducing them into expression vectors, and then expressing them in appropriate host cells (see, for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

The antibodies of the present invention or as disclosed include not only monovalent antibodies but also multivalent antibodies. Multivalent antibodies include multivalent antibodies whose antigen binding sites are all the same and multivalent antibodies whose antigen binding sites are partially or completely different.

Antibodies bound to various types of molecules such as polyethylene glycol (PEG) can also be used as modified antibodies. Moreover, chemotherapeutic agents, toxic peptides, or cytotoxic substances such as radioactive chemical substances can be bound to the antibodies. Such modified antibodies (hereinafter referred to as antibody conjugates) can be obtained by subjecting the obtained antibodies to chemical modification. Methods for modifying antibodies are already established in this field. Furthermore, as described below, such antibodies can also be obtained in the molecular form of a bispecific antibody designed using genetic engineering technologies to recognize not only LGR7 proteins, but also chemotherapeutic agents, toxic peptides, cytotoxic substances such as radioactive chemical substances, or such. These antibodies are included in the "antibodies" of the present invention.

Chemotherapeutic agents that are bound to the anti-LGR7 antibodies to drive the cytotoxic activity include the following:

azaribine, anastrozole, azacytidine, bleomycin, bortezomib,

bryostatin-1, busulfan, camptothecin, 10-hydroxycamptothecin, carmustine, celebrex, chlorambucil, cisplatin, irinotecan, carboplatin, cladribine, cyclophosphamide, cytarabine, dacarbazine, docetaxel, dactinomycin, daunomycin glucuronide, daunorubicin, dexamethasone, diethylstilbestrol, doxorubicin, doxorubicin glucuronide, epirubicin, ethinyl estradiol, estramustine, etoposide, etoposide glucuronide, floxuridine, fludarabine, flutamide, fluorouracil, fluoxymesterone, gemcitabine, hydroxyprogesterone caproate, hydroxyurea, idarubicin, ifosfamide, leucovorin, lomustine, mechlorethamine, medroxyprogesterone acetate, megestrol acetate, melphalan, mercaptopurine, methotrexate, mitoxantrone, mithramycin, mitomycin, mitotane, phenylbutyrate, prednisone, procarbazine, paclitaxel, pentostatin, semustine streptozocin, tamoxifen, taxanes, taxol, testosterone propionate, thalidomide, thioguanine, thiotepa, teniposide, topotecan, uracil mustard, vinblastine, vinorelbine, and vincristine.

In the present invention, preferred chemotherapeutic agents are low-molecular-weight chemotherapeutic agents. Low-molecular-weight chemotherapeutic agents are unlikely to interfere with antibody function even after binding to antibodies. In the present invention, low-molecular-weight chemotherapeutic agents usually have a molecular weight of 100 to 2000, preferably 200 to 1000. Examples of the chemotherapeutic agents demonstrated herein are all low-molecular-weight chemotherapeutic agents. The chemotherapeutic agents of the present invention include prodrugs that are converted to active chemotherapeutic agents *in vivo.* Prodrug activation may be enzymatic conversion or non-enzymatic conversion.

Moreover, the antibody can be modified with a toxic peptide. Examples of the toxic peptides include the following: Diphtheria toxin A Chain (Langone J. J., et al., Methods in Enzymology, 93,307-308,1983); Pseudomonas Exotoxin (Nature Medicine, 2, 350-353, 1996); Ricin A Chain (Fulton R. J., et al., J. Biol. Chem., 261, 5314-5319, 1986; Sivam G., et al., Cancer Res., 47, 3169-3173, 1987; Cumber A. J. et al., J. Immunol. Methods, 135, 15-24, 1990; Wawrzynczak E. J., et al., Cancer Res., 50, 7519-7562, 1990; Gheeite V., et al., J. Immunol. Methods, 142, 223-230, 1991); Deglicosylated Ricin A Chain (Thorpe P. E., et al., Cancer Res., 47, 5924-5931, 1987); Abrin A Chain (Wawrzynczak E. J., et al., Br. J. Cancer, 66, 361-366, 1992; Wawrzynczak E. J., et al., Cancer Res., 50, 7519-7562, 1990; Sivam G., et al., Cancer Res., 47, 3169-3173, 1987; Thorpe P. E., et al., Cancer Res., 47, 5924-5931, 1987); Gelonin (Sivam G., et al., Cancer Res., 47, 3169-3173, 1987; Cumber A. J. et al., J. Immunol. Methods, 135, 15-24, 1990; Wawrzynczak E. J., et al., Cancer Res., 50, 7519-7562, 1990; Bolognesi A., et al., Clin. exp. Immunol., 89, 341-346, 1992); Pokeweed anti-viral protein from seeds (PAP-s) (Bolognesi A., et al.,Clin. exp. Immunol., 89, 341-346, 1992); Briodin (Bolognesi A., et al., Clin. exp. Immunol., 89, 341-346, 1992); Saporin (Bolognesi A., et al., Clin. exp. Immunol., 89, 341-346, 1992); Momordin (Cumber A. J., et al., J. Immunol. Methods, 135, 15-24, 1990; Wawrzynczak E. J., et al., Cancer Res., 50, 7519-7562, 1990; Bolognesi A., et al., Clin. exp. Immunol., 89, 341-346, 1992); Momorcochin (Bolognesi A., et al., Clin. exp. Immunol., 89, 341-346, 1992); Dianthin 32 (Bolognesi A., et al., Clin. exp. Immunol., 89, 341-346, 1992); Dianthin 30 (Stirpe F., Barbieri L., FEBS letter 195,1-8,1986); Modeccin (Stirpe F., Barbieri L., FEBS letter 195,1-8,1986); Viscumin (Stirpe F., Barbieri L., FEBS letter 195,1-8,1986); Volkesin (Stirpe F., Barbieri L., FEBS letter 195,1-8,1986); Dodecandrin (Stirpe F., Barbieri L., FEBS letter 195, 1-8, 1986); Tritin (Stirpe F., Barbieri L., FEBS letter 195,1-8,1986); Luffin (Stirpe F., Barbieri L., FEBS letter 195, 1-8, 1986); and Trichokirin (Casellas P., et al., Eur. J. Biochem. 176, 581-588, 1988; Bolognesi A., et al., Clin. exp. Immunol., 89, 341-346, 1992).

In the present invention, "radioactive chemical substance" refers to a chemical substance containing a radioisotope. There is no particular limitation on the radioisotope. Any radioisotope may be used, and for example, $^{32}P$, $^{14}C$, $^{125}I$, $^{3}H$, $^{131}I$, $^{186}Re$, and $^{188}Re$ may be utilized.

In another embodiment or as disclosed, one or two or more of the low-molecular-weight chemotherapeutic agents and toxic peptides can be combined and used for antibody modification. The bonding between an anti-LGR7 antibody and the above-mentioned low-molecular weight chemotherapeutic agent may be covalent bonding or non-covalent bonding. Methods for producing antibodies bound to these chemotherapeutic agents are known.

Furthermore, pharmacologically active proteins or peptide toxins can be bound to antibodies by gene recombination technologies. Specifically, for example, it is possible to construct a recombinant vector by fusing a DNA encoding the above-mentioned toxic peptide with a DNA encoding an anti-LGR7 antibody in frame, and inserting this into an expression vector. This vector is introduced into suitable host cells, the obtained transformed cells are cultured, and the incorporated DNA is expressed. Thus, an anti-LGR7 antibody bound to the toxic peptide can be obtained as a fusion protein. When obtaining an antibody as a fusion protein, the pharmacologically active protein or toxin is generally fused at the C terminus of the antibody. A peptide linker can be inserted between the antibody and the pharmacologically active protein or toxin.

Furthermore, the antibody of the present invention or as disclosed may be a bispecific antibody. A bispecific antibody refers to an antibody that carries variable regions that recognize different epitopes within the same antibody molecule. The bispecific antibody may have antigen-binding sites that recognize different epitopes on an LGR7 molecule. Two molecules of such a bispecific antibody can bind to one molecule of LGR7. As a result, stronger cytotoxic action can be expected.

Alternatively, the bispecific antibody may be an antibody in which one antigen-binding site recognizes LGR7, and the other antigen-binding site recognizes a cytotoxic substance. Specifically, cytotoxic substances include chemotherapeutic agents, toxic peptides, and radioactive chemical substances. Such a bispecific antibody binds to LGR7-expressing cells, and at the same time, captures cytotoxic substances. This enables the cytotoxic substances to directly act on LGR7-expressing cells. Therefore, bispecific antibodies that recognize cytotoxic substances specifically injure tumor cells and suppress tumor cell proliferation.

Furthermore, in the present invention, bispecific antibodies that recognize antigens other than LGR7 may be combined. For example, it is possible to combine bispecific antibodies that recognize non-LGR7 antigens that are specifically expressed on the surface of target cancer cells like LGR7.

Methods for producing bispecific antibodies are known. For example, two types of antibodies recognizing different antigens may be linked to prepare a bispecific antibody. The antibodies to be linked may be half molecules each having an H chain or an L chain, or may be quarter molecules consisting of only an H chain. Alternatively, hybrid cells producing a bispecific antibody can be prepared by fusing hybridomas producing different monoclonal antibodies. Bispecific antibodies can also be prepared by genetic engineering technologies.

Known methods can be used to measure the antigen-binding activity of the antibodies (Antibodies A Laboratory Manual. Ed Harlow, David Lane, Cold Spring Harbor Laboratory, 1988). For example, an enzyme linked immunosorbent assay (ELISA), an enzyme immunoassay (EIA), a radioimmunoassay (RIA), or a fluoroimmunoassay can be used.

The antibodies of the present invention or as disclosed may be antibodies with a modified sugar chain. It is known that the cytotoxic activity of an antibody can be increased by modifying its sugar chain. Known antibodies having modified sugar chains include the following:

antibodies with modified glycosylation (for example, WO 99/54342);
antibodies deficient in fucose attached to sugar chains (for example, WO 00/61739, WO 02/31140, WO2006/067913, etc.);
antibodies having a sugar chain with bisecting GlcNAc (for example, WO 02/79255), etc.

When the antibodies of the present invention or as disclosed are used for therapy, they are preferably antibodies having cytotoxic activity.

In the present invention, the cytotoxic activity includes, for example, antibody-dependent cell-mediated cytotoxicity (ADCC) activity and complement-dependent cytotoxicity (CDC) activity. In the present invention, CDC activity refers to complement system-mediated cytotoxic activity. ADCC activity refers to the activity of injuring a target cell when a specific antibody attaches to its cell surface antigen. An Fcγ receptor-carrying cell (immune cell, or such) binds to the Fc portion of the antibody via the Fcγ receptor and the target cell is damaged.

An anti-LGR7 antibody of the present invention or as disclosed can be tested to see whether it has ADCC activity or CDC activity using known methods (for example, Current Protocols in Immunology, Chapter 7. Immunologic studies in humans, Editor, John E. Coligan et al., John Wiley & Sons, Inc., (1993) and the like).

First, specifically, effector cells, complement solution, and target cells are prepared.

(1) Preparation of effector cells

Spleen is removed from a CBA/N mouse or the like, and spleen cells are isolated in RPMI1640 medium (manufactured by Invitrogen). After washing in the same medium containing 10% fetal bovine serum (FBS, manufactured by HyClone), the cell concentration is adjusted to 5 x $10^6$ /mL to prepare the effector cells.

(2) Preparation of complement solution

Baby Rabbit Complement (manufactured by CEDARLANE) is diluted 10-fold in a culture medium (manufactured by Invitrogen) containing 10% FBS to prepare a complement solution.

(3) Preparation of target cells

The target cells can be radioactively labeled by incubating cells expressing the LGR7 protein with 0.2 mCi of sodium chromate-$^{51}$Cr (manufactured by GE Healthcare Bio-Sciences) in a DMEM medium containing 10% FBS for one hour at 37°C. For LGR7 protein-expressing cells, one may use transformed cells with a LGR7 gene, ovarian cancer cells, or such. After radioactive labeling, cells are washed three times in RPMI1640 medium with 10% FBS, and the target cells can be prepared by adjusting the cell concentration to 2 x $10^5$ /mL.

ADCC activity or CDC activity can be measured by the method described below. In the case of ADCC activity measurement, the target cell and anti-LGR7 antibody (50 μL each) are added to a 96-well U-bottom plate (manufactured by Becton Dickinson), and reacted for 15 minutes on ice. Thereafter, 100 μL of effector cells are added and incubated in a carbon dioxide incubator for four hours. The final concentration of the antibody is adjusted to 0 or 10 μg/mL. After culturing, 100 μL of the supernatant is collected, and the radioactivity is measured with a gamma counter (COBRAII AUTO-GAMMA, MODEL D5005, manufactured by Packard Instrument Company). The cytotoxic activity (%) can be calculated using the measured values according to the equation: (A - C) / (B - C) x 100, wherein A represents the radioactivity (cpm) in each sample, B represents the radioactivity (cpm) in a sample where 1% NP-40 (manufactured by Nacalai Tesque) has been added, and C represents the radioactivity (cpm) of a sample containing the target cells only.

Meanwhile, in the case of CDC activity measurement, 50 μL of target cell and 50 μL of an anti-LGR7 antibody are added to a 96-well flat-bottomed plate (manufactured by Becton Dickinson), and reacted for 15 minutes on ice. Thereafter, 100 μL of the complement solution is added, and incubated in a carbon dioxide incubator for four hours. The final concentration of the antibody is adjusted to 0 or 3 μg/mL. After incubation, 100 μL of supernatant is collected, and the radioactivity is measured with a gamma counter. The cytotoxic activity can be calculated in the same way as in the ADCC activity determination.

On the other hand, in the case of measuring the cytotoxic activity of an antibody conjugate, 50 μL of target cell and 50 μL of an anti-LGR7 antibody conjugate are added to a 96-well flat-bottomed plate (manufactured by Becton Dickinson), and reacted for 15 minutes on ice. This is then incubated in a carbon dioxide incubator for one to four hours. The final concentration of the antibody is adjusted to 0 or 3 μg/mL. After culturing, 100 μL of supernatant is collected, and the radioactivity is measured with a gamma counter. The cytotoxic activity can be calculated in the same way as in the ADCC activity determination.

The antibody of the present invention is an antibody having internalization activity. In the present invention, "antibody having internalization activity" refers to an antibody that is transported into a cell (into the cytoplasm, vesicles, other organelles, and such) upon binding to LGR7 on the cell surface.

Whether or not an antibody has internalization activity can be confirmed using methods known to those skilled in the art. For example, the internalization activity can be confirmed by the method of contacting an anti-LGR7 antibody bound by a labeled substance with LGR7-expressing cells and determining whether the labeled substance is incorporated into the cells, or the method of contacting an anti-LGR7 antibody bound by a cytotoxic substance with LGR7-expressing cells and determining whether or not cell death is induced in the LGR7-expressing cells. More specifically, whether or not an antibody has internalization activity can be checked, for example, by the method described in the Examples below.

An antibody having internalization activity can be used for a pharmaceutical composition such as an anticancer agent, for example, by binding it with the above-mentioned cytotoxic substance.

The monoclonal antibodies of the present invention are referred to below as antibodies (a) to (i). These antibodies can be, for example, full-length antibodies, low-molecular-weight antibodies, animal antibodies, chimeric antibodies, humanized antibodies, or human antibodies.

(a) A monoclonal antibody comprising an H chain having the amino acid sequence of SEQ ID NO: 25 as CDR1, the amino acid sequence of SEQ ID NO: 26 as CDR2, and the amino acid sequence of SEQ ID NO: 27 as CDR3 and comprising an L chain having the amino acid sequence of SEQ ID NO: 30 as CDR1, the amino acid sequence of SEQ ID NO: 31 as CDR2, and the amino acid sequence of SEQ ID NO: 32 as CDR3;

(b) a monoclonal antibody comprising an H chain having the amino acid sequence of SEQ ID NO: 5 as CDR1, the amino acid sequence of SEQ ID NO: 6 as CDR2, and the amino acid sequence of SEQ ID NO: 7 as CDR3 and comprising an L chain having the amino acid sequence of SEQ ID NO: 10 as CDR1, the amino acid sequence of SEQ ID NO: 11 as CDR2, and the amino acid sequence of SEQ ID NO: 12 as CDR3;

(c) a monoclonal antibody comprising an H chain having the amino acid sequence of SEQ ID NO: 15 as CDR1, the amino acid sequence of SEQ ID NO: 16 as CDR2, and the amino acid sequence of SEQ ID NO: 17 as CDR3 and comprising an L chain having the amino acid sequence of SEQ ID NO: 20 as CDR1, the amino acid sequence of SEQ ID NO: 21 as CDR2, and the amino acid sequence of SEQ ID NO: 22 as CDR3;

(d) a monoclonal antibody comprising an H chain having the amino acid sequence of SEQ ID NO: 45 as CDR1, the amino acid sequence of SEQ ID NO: 46 as CDR2, and the amino acid sequence of SEQ ID NO: 47 as CDR3 and comprising an L chain having the amino acid sequence of SEQ ID NO: 50 as CDR1, the amino acid sequence of SEQ ID NO: 51 as CDR2, and the amino acid sequence of SEQ ID NO: 52 as CDR3;

(e) a monoclonal an antibody comprising an H chain having the amino acid sequence of SEQ ID NO: 55 as CDR1, the amino acid sequence of SEQ ID NO: 56 as CDR2, and the amino acid sequence of SEQ ID NO: 57 as CDR3 and comprising an L chain having the amino acid sequence of SEQ ID NO: 60 as CDR1, the amino acid sequence of SEQ ID NO: 61 as CDR2, and the amino acid sequence of SEQ ID NO: 62 as CDR3;

(f) a monoclonal an antibody comprising an H chain having the amino acid sequence of SEQ ID NO: 65 as CDR1, the amino acid sequence of SEQ ID NO: 66 as CDR2, and the amino acid sequence of SEQ ID NO: 67 as CDR3 and comprising an L chain having the amino acid sequence of SEQ ID NO: 70 as CDR1, the amino acid sequence of SEQ ID NO: 71 as CDR2, and the amino acid sequence of SEQ ID NO: 72 as CDR3;

(g) a monoclonal an antibody comprising an H chain having the amino acid sequence of SEQ ID NO: 75 as CDR1, the amino acid sequence of SEQ ID NO: 76 as CDR2, and the amino acid sequence of SEQ ID NO: 77 as CDR3 and comprising an L chain having the amino acid sequence of SEQ ID NO: 80 as CDR1, the amino acid sequence of SEQ ID NO: 81 as CDR2, and the amino acid sequence of SEQ ID NO: 82 as CDR3;

(h) a monoclonal an antibody comprising an H chain having the amino acid sequence of SEQ ID NO: 85 as CDR1, the amino acid sequence of SEQ ID NO: 86 as CDR2, and the amino acid sequence of SEQ ID NO: 87 as CDR3 and comprising an L chain having the amino acid sequence of SEQ ID NO: 90 as CDR1, the amino acid sequence of SEQ ID NO: 91 as CDR2, and the amino acid sequence of SEQ ID NO: 92 as CDR3;

(i) a monoclonal antibody that competes with antibody of (a) for binding to the epitope that the antibody of (a) binds to.

Furthermore, disclosed herein are further antibodies that recognize LGR7. Examples are the antibodies of (1) to (29) described below. These antibodies can be, for example, full-length antibodies, low-molecular-weight antibodies, animal antibodies, chimeric antibodies, humanized antibodies, or human antibodies. Antibodies of (3), (6), (9), (15), (18), (21), (24), and (27) are comprised by the present invnetion.,

(1) an antibody comprising an H chain having the amino acid sequence of SEQ ID NO: 5 as CDR1, the amino acid sequence of SEQ ID NO: 6 as CDR2, and the amino acid sequence of SEQ ID NO: 7 as CDR3 (22DA6 heavy chain);

(2) an antibody comprising an L chain having the amino acid sequence of SEQ ID NO: 10 as CDR1, the amino acid sequence of SEQ ID NO: 11 as CDR2, and the amino acid sequence of SEQ ID NO: 12 as CDR3 (22DA6 light chain);

(3) an antibody comprising the H chain of (1) and the L chain of (2) (22DA6);

(4) an antibody comprising an H chain having the amino acid sequence of SEQ ID NO: 15 as CDR1, the amino acid sequence of SEQ ID NO: 16 as CDR2, and the amino acid sequence of SEQ ID NO: 17 as CDR3 (22DA7 heavy chain);

(5) an antibody comprising an L chain having the amino acid sequence of SEQ ID NO: 20 as CDR1, the amino acid sequence of SEQ ID NO: 21 as CDR2, and the amino acid sequence of SEQ ID NO: 22 as CDR3 (22DA7 light chain);

(6) an antibody comprising the H chain of (4) and the L chain of (5) (22DA7);

(7) an antibody comprising an H chain having the amino acid sequence of SEQ ID NO: 25 as CDR1, the amino acid sequence of SEQ ID NO: 26 as CDR2, and the amino acid sequence of SEQ ID NO: 27 as CDR3 (22DA17 heavy chain);

(8) an antibody comprising an L chain having the amino acid sequence of SEQ ID NO: 30 as CDR1, the amino acid sequence of SEQ ID NO: 31 as CDR2, and the amino acid sequence of SEQ ID NO: 32 as CDR3 (22DA17 light chain);

(9) an antibody comprising the H chain of (7) and the L chain of (8) (22DA17);

(10) an antibody comprising an H chain having the amino acid sequence of SEQ ID NO: 35 as CDR1, the amino acid sequence of SEQ ID NO: 36 as CDR2, and the amino acid sequence of SEQ ID NO: 37 as CDR3 (22DA22 heavy chain);

(11) an antibody comprising an L chain having the amino acid sequence of SEQ ID NO: 40 as CDR1, the amino acid sequence of SEQ ID NO: 41 as CDR2, and the amino acid sequence of SEQ ID NO: 42 as CDR3 (22DA22 light chain);

(12) an antibody comprising the H chain of (10) and the L chain of (11) (22DA22);

(13) an antibody comprising an H chain having the amino acid sequence of SEQ ID NO: 45 as CDR1, the amino acid sequence of SEQ ID NO: 46 as CDR2, and the amino acid sequence of SEQ ID NO: 47 as CDR3 (22DA23 heavy chain);

(14) an antibody comprising an L chain having the amino acid sequence of SEQ ID NO: 50 as CDR1, the amino acid sequence of SEQ ID NO: 51 as CDR2, and the amino acid sequence of SEQ ID NO: 52 as CDR3 (22DA23 light chain);

(15) an antibody comprising the H chain of (13) and the L chain of (14) (22DA23);

(16) an antibody comprising an H chain having the amino acid sequence of SEQ ID NO: 55 as CDR1, the amino acid sequence of SEQ ID NO: 56 as CDR2, and the amino acid sequence of SEQ ID NO: 57 as CDR3 (22DA24 heavy chain);

(17) an antibody comprising an L chain having the amino acid sequence of SEQ ID NO: 60 as CDR1, the amino acid sequence of SEQ ID NO: 61 as CDR2, and the amino acid sequence of SEQ ID NO: 62 as CDR3 (22DA24 light chain);

(18) an antibody comprising the H chain of (16) and the L chain of (17) (22DA24);

(19) an antibody comprising an H chain having the amino acid sequence of SEQ ID NO: 65 as CDR1, the amino acid sequence of SEQ ID NO: 66 as CDR2, and the amino acid sequence of SEQ ID NO: 67 as CDR3 (22SD7 heavy chain);
(20) an antibody comprising an L chain having the amino acid sequence of SEQ ID NO: 70 as CDR1, the amino acid sequence of SEQ ID NO: 71 as CDR2, and the amino acid sequence of SEQ ID NO: 72 as CDR3 (22SD7 light chain);
(21) an antibody comprising the H chain of (19) and the L chain of (20) (22SD7);
(22) an antibody comprising an H chain having the amino acid sequence of SEQ ID NO: 75 as CDR1, the amino acid sequence of SEQ ID NO: 76 as CDR2, and the amino acid sequence of SEQ ID NO: 77 as CDR3 (22SD11 heavy chain);
(23) an antibody comprising an L chain having the amino acid sequence of SEQ ID NO: 80 as CDR1, the amino acid sequence of SEQ ID NO: 81 as CDR2, and the amino acid sequence of SEQ ID NO: 82 as CDR3 (22SD11 light chain);
(24) an antibody comprising the H chain of (22) and the L chain of (23) (22SD11);
(25) an antibody comprising an H chain having the amino acid sequence of SEQ ID NO: 85 as CDR1, the amino acid sequence of SEQ ID NO: 86 as CDR2, and the amino acid sequence of SEQ ID NO: 87 as CDR3 (22SD48 heavy chain);
(26) an antibody comprising an L chain having the amino acid sequence of SEQ ID NO: 90 as CDR1, the amino acid sequence of SEQ ID NO: 91 as CDR2, and the amino acid sequence of SEQ ID NO: 92 as CDR3 (22SD48 light chain);
(27) an antibody comprising the H chain of (25) and the L chain of (26) (22SD48);
(28) an antibody that has equivalent activity as the antibody of any one of (1) to (27);
(29) an antibody that recognizes the same epitope recognized by the antibody of any one of (1) to (27).

"Having equivalent activity" means having binding activity towards LGR7 and/or cytotoxic activity against LGR7-expressing cells that are equivalent to those of an antibody of the present invention or as disclosed.

A method of introducing mutations into polypeptides is one of the methods well known to those skilled in the art for preparing polypeptides that are functionally equivalent to a certain polypeptide. For example, those skilled in the art can prepare an antibody functionally equivalent to an antibody of the present invention or as disclosed by introducing appropriate mutations into the antibody using site-directed mutagenesis (Hashimoto-Gotoh, T. et al. (1995) Gene 152, 271-275; Zoller, MJ, and Smith, M. (1983) Methods Enzymol. 100, 468-500; Kramer, W. et al. (1984) Nucleic Acids Res. 12, 9441-9456; Kramer W, and Fritz HJ (1987) Methods. Enzymol. 154, 350-367; Kunkel, TA (1985) Proc. Natl. Acad. Sci. USA. 82, 488-492; Kunkel (1988) Methods Enzymol. 85, 2763-2766) and such. Amino acid mutations may also occur naturally. In this way, the antibodies of the present invention or as disclosed also comprise antibodies comprising amino acid sequences with one or more amino acid mutations in the amino acid sequences of the antibodies of the present invention or as disclosed, respectively, and which are functionally equivalent to the antibodies of the present invention or as disclosed, respectively.

The number of amino acids that are mutated in such mutants is generally considered to be 50 amino acids or less, preferably 30 amino acids or less, and more preferably 10 amino acids or less (for example, 5 amino acids or less).

It is desirable that the amino acid residues are mutated into amino acids in which the properties of the amino acid side chains are conserved. For example, the following categories have been established depending on the amino acid side chain properties:

hydrophobic amino acids (A, I, L, M, F, P, W, Y, and V);
hydrophilic amino acids (R, D, N, C, E, Q, G, H, K, S, and T);
amino acids having aliphatic side chains (G, A, V, L, I, and P);
amino acids having hydroxyl-containing side chains (S, T, and Y);
amino acids having sulfur-containing side chains (C and M);
amino acids having carboxylic acid- and amide-containing side chains (D, N, E, and Q);
amino acids having basic side chains (R, K, and H); and
amino acids having aromatic ring-containing side chains (H, F, Y, and W)
(amino acids are represented by one-letter codes in parentheses).

Polypeptides comprising a modified amino acid sequence, in which one or more amino acid residues in a certain amino acid sequence is deleted, added, and/or substituted with other amino acids, are known to retain their original biological activities (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413). That is, it is generally said that, in an amino acid sequence constituting a certain polypeptide, the activity of the polypeptide is highly likely to be maintained when amino acids classified into the same group are mutually substituted. In the present invention, the above-mentioned substitution between amino acids within the same amino acid group is referred to as conservative substitution.

Disclosed are antibodies that bind to the same epitope bound by an anti-LGR7 antibody as disclosed. Thus, antibodies may recognize the same epitope recognized by the 22DA6, 22DA7, 22DA17, 22DA22, 22DA23, 22DA24, 22SD7, 22SD11, or 22SD48 antibody, and to the use of such antibodies. These antibodies can be obtained, for example, by the methods described below.

Whether a test antibody shares an epitope with a certain antibody can be determined by their competition for the same epitope. The competition between antibodies is detected by cross-blocking assay and the like. For example, competitive ELISA assay is a preferred cross-blocking assay. Comprised by the present invention is an antibody that competes with the antibody 22DA17 for binding to the epitope that the antibody 22DA17 binds to.

More specifically, in cross-blocking assay, microtiter plate wells coated with an LGR7 protein are preincubated either in the presence or absence of a candidate competing antibody, and an anti-LGR7 antibody is added thereto. The quantity of the anti-LGR7 antibody that binds to the LGR7 protein in a well correlates indirectly with the binding ability of the candidate competing antibody (test antibody) that competes for binding to the same epitope. In other words, the higher the affinity of a test antibody to the same epitope, the lower the binding of the anti-LGR7 antibody to a well coated with the LGR7 protein, and the higher the binding of the test antibody to a well coated with the LGR7 protein.

The quantity of the antibody bound to the well can be easily measured by labeling the antibody in advance. For example, a biotin-labeled antibody can be measured using an avidin/peroxidase conjugate and a suitable substrate. Cross-blocking assay using an enzyme label such as peroxidase is particularly referred to as competitive ELISA assay. The antibody can be labeled with another labeling substance that can be detected or measured. More specifically, radiolabels, fluorescent labels and the like are known.

Alternatively, competitive FACS assay is also a preferable cross-blocking assay.

Specifically, in competitive ELISA assay, instead of using the LGR7 protein to coat the wells of a microtiter plate, LGR7 protein-expressing cells are used. This is pre-incubated in the presence or absence of a candidate competitive antibody, then a biotin-labeled anti-LGR7 antibody is added thereto, and a streptavidin/fluorescein conjugate can be used to detect the competition between antibodies. Cross-blocking assay that uses flow cytometry is particularly referred to as competitive FACS assay. The antibody can be labeled with another fluorescent labeling substance that can be detected or measured.

Alternatively, when a test antibody has a constant region derived from a species other than that of the anti-LGR7 antibody, each antibody bound to the well can be measured by a labeled antibody that recognizes its constant region. Alternatively, even when the antibodies are derived from the same species, if their classes are different, each antibody bound to the well can be measured with an antibody that recognizes its class.

If a candidate antibody can block the binding of the anti-LGR7 antibody by at least 20%, preferably at least 30%, and more preferably at least 50% compared to the binding activity obtained in a control test performed in the absence of the candidate competing antibody, then the candidate competing antibody either binds to essentially the same epitope as the anti-LGR7 antibody, or competes with the anti-LGR7 antibody for binding to the same epitope. When determining the epitope, the constant region of an antibody can be substituted with the same constant region as the test antibody.

## Pharmaceutical Compositions

Disclosed are pharmaceutical compositions comprising an LGR7 protein-binding antibody as an active ingredient. Furthermore, disclosed are cell growth inhibitors, in particular anticancer agents, which comprise an LGR7 protein-binding antibody as an active ingredient. The cell growth inhibitors and anticancer agents may be administered to a subject who suffers or may suffer from cancer. Since the level of LGR7 expression is very low in normal cells except in the brain but is enhanced in cancer cells, it is thought that cancer cell-specific cytotoxic effect can be obtained by administration of an anti-LGR7 antibody. Comprised by the present invention is the antibody of the present invention for use in the treatment of cancer, wherein the cancer may be ovarian cancer; particularly wherein the ovarian cancer is clear cell adenocarcinoma.

There is no particular limitation on the anti-LGR7 antibodies used in the pharmaceutical compositions (for example, anticancer agents) as disclosed, and they may be any anti-LGR7 antibodies. For example, the anti-LGR7 antibodies described above may be used.

"Comprising an LGR7-binding antibody as an active ingredient" means comprising an anti-LGR7 antibody as a primary active ingredient, and there is no limitation on the content ratio of the anti-LGR7 antibody.

When the disease targeted by a pharmaceutical composition is cancer, the targeted cancer is not particularly limited; however, ovarian cancer is preferred, and ovarian clear cell adenocarcinoma is particularly preferred. The cancer may be either primary or metastatic lesions.

The pharmaceutical compositions or the anti-LGR7 antibody can be administered orally or parenterally to a patient. Preferably, the administration is parenteral administration. Specifically, the method of administration is, for example, administration by injection, transnasal administration, transpulmonary administration, or transdermal administration. Examples of administration by injection include systemic and local administrations of the pharmaceutical composition or the anti-LGR7 antibody by intravenous injection, intramuscular injection, intraperitoneal injection, subcutaneous injection, or such. A suitable administration method may be selected according to the age of the patient and symptoms. The dosage may be selected, for example, within the range of 0.0001 mg to 1000 mg per kg body weight in each administration. Alternatively, for example, the dosage for each patient may be selected within the range of 0.001 to 100,000 mg/body. However, the pharmaceutical composition or anti-LGR7 antibody is not limited to these doses.

The pharmaceutical compositions or anti-LGR7 antibody can be formulated according to conventional methods (for example, Remington's Pharmaceutical Science, latest edition, Mark Publishing Company, Easton, U.S.A), and may also contain pharmaceutically acceptable carriers and additives. Examples include, but are not limited to, surfactants, excipients, coloring agents, perfumes, preservatives, stabilizers, buffers, suspending agents, isotonization agents, binders, disintegrants, lubricants, fluidity promoting agents, and flavoring agents; and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium chain fatty acid triglyceride, polyoxyethylene hardened castor oil 60, saccharose, carboxymethyl cellulose, corn starch, inorganic salt, and such.

Disclosed are methods for damaging or inhibiting proliferation of LGR7-expressing cells by contacting the LGR7-expressing cells with an antibody that binds to the LGR7 protein.

There is no particular limitation on the antibodies used in the methods as disclosed, but for example, the antibodies of the present invention or described above may be used. The cells bound by the anti-LGR7 antibodies are not particularly limited as long as they are cells expressing LGR7. The LGR7-expressing cells are preferably cancer cells. More preferably, they are ovarian cancer cells. The methods can be applied to both primary and metastatic lesions of these cancers. Even more preferable cancer cells are primary and metastatic ovarian cancer cells.

"Contact" is carried out, for example, by adding the antibody to the culture medium of LGR7-expressing cells cultured *in vitro.* Alternatively, "contact" is carried out by administering to a non-human animal into which cells expressing LGR7 have been transplanted, or to an animal that endogenously has LGR7-expressing cancer cells.

The following method is suitably used as a method for evaluating or measuring cell damage induced in LGR7-expressing cells by contacting them with an anti-LGR7 antibody. Examples of a method for evaluating or measuring the cytotoxic activity in a test tube include methods for measuring the above-mentioned antibody-dependent cell-mediated cytotoxicity (ADCC) activity, complement-dependent cytotoxicity (CDC) activity, and such. Whether or not an anti-LGR7 antibody has ADCC activity or CDC activity can be measured by known methods (for example, Current protocols in Immunology, Chapter 7. Immunologic studies in humans, Editor, John E. Coligan et al., John Wiley & Sons, Inc., (1993) and the like). For activity measurements, an binding antibody having the same isotype as an anti-LGR7 antibody but not having any cytotoxic activity can be used as a control antibody in the same manner as the anti-LGR7 antibody, and it can be determined that the activity is present when the anti-LGR7 antibody shows a stronger cytotoxic activity than the control antibody.

The isotype of an antibody is defined by the sequence of its H chain constant region in the antibody amino acid sequence. The isotype of an antibody is ultimately determined *in vivo* by class switching that arises from genetic recombinations in chromosomes which occur during maturation of antibody-producing B-cells. Difference in isotype is reflected in the difference of physiological and pathological functions of antibodies. Specifically, for example, the strength of cytotoxic activity is known to be influenced by antibody isotype in addition to the expression level of the antigen. Therefore, when measuring the above-described cell damaging activity, an antibody of the same isotype as the test antibody is preferably used as the control.

To evaluate or measure cell damaging activity *in vivo,* for example, LGR7-expressing cancer cells are intradermally or subcutaneously transplanted to a non-human test animal, and then a test antibody is intravenously or intraperitoneally administered daily or at the interval of few days, starting from the day of transplantation or the following day. Cytotoxicity can be determined by daily measurement of tumor size. In a similar manner to the evaluation in a test tube, cytotoxicity can be determined by administering a control antibody having the same isotype, and observing that the tumor size in the anti-LGR7 antibody-administered group is significantly smaller than the tumor size in the control antibody-administered group. When using a mouse as the non-human test animal, it is suitable to use a nude (nu/nu) mouse whose thymus has been made genetically defective so that its T lymphocyte function is lost. The use of such a mouse can eliminate the participation of T lymphocytes in the test animals when evaluating or measuring the cytotoxicity of the administered antibody.

Furthermore, disclosed are cancer diagnostic methods that comprise detecting an LGR7 protein or a gene encoding the LGR7 protein. While LGR7 expression is significantly enhanced in various cancer tissues or cancer cell lines, LGR7 expression in normal cells is very low. Therefore, LGR7 is useful as a marker for specific detection of cancer.

Cancer may be diagnosed by detecting the LGR7 protein in a sample. Thereby, an extracellular domain of the LGR7 protein may be detected. Detection of the LGR7 protein may be carried out using an antibody that recognizes the LGR7 protein.

A specific example of the diagnostic methods is a cancer diagnostic method comprising the following steps:

(a) providing a sample collected from a subject; and
(b) detecting an LGR7 protein contained in the collected sample using an LGR7 protein-binding antibody.

"Detection" includes both quantitative and qualitative detection. The following measurements are examples of qualitative detection:

measurement to simply determine whether or not the LGR7 protein is present;
measurement to determine whether or not a predetermined amount or more of LGR7 protein is present; and
measurement to compare the quantity of LGR7 protein with that of another sample (for example, a control sample, etc.).

On the other hand, quantitative detection includes measurement of the concentration of LGR7 protein, the quantity of LGR7 protein, and such.

The test sample is not particularly limited as long as it is a sample that possibly contains the LGR7 protein. The sample may be collected from the body of an organism such as a mammal. The sample may be collected from a human. Specific examples of the test sample include blood, interstitial fluid, plasma, extravascular fluid, cerebrospinal fluid, synovial fluid, pleural fluid, serum, lymph, saliva, urine, tissue, ascites, and intraperitoneal lavage. The sample may be obtained from a test sample such as a specimen of fixed tissues or cells collected from the body of an organism, or liquid culture medium of cells.

The cancer to be diagnosed is not particularly limited and can be any kind of cancer. Specific examples include ovarian cancer. Both primary and metastatic lesions of these cancers can be diagnosed. The cancers may be primary ovarian cancer and metastatic ovarian cancer.

If the LGR7 protein is detected in a test sample, its level is used as an indicator for cancer diagnosis. Specifically, if the quantity of the LGR7 protein detected in a test sample is greater than that in a negative control or healthy individual, it indicates that the patient has cancer or is highly likely to suffer from cancer in the future. The cancer diagnostic method may comprise the following steps:

(1) detecting the level of LGR7 expression in a test sample collected from a subject; and
(2) indicating that the subject has cancer when the level of LGR7 expression detected in (1) is higher than that in a control.

"Control" refers to a sample that serves as a standard for comparison, and it includes both a negative control and a biological sample from a healthy individual. A negative control can be obtained by collecting biological samples from healthy individuals and mixing them as necessary. The level of LGR7 expression in the control can be detected in parallel with the level of LGR7 expression in a biological sample from a subject. Alternatively, the level of LGR7 expression in biological samples from a plurality of healthy individuals can be detected in advance, and the standard expression level in healthy individuals can be determined statistically. More specifically, for example, a standard value of mean $\pm$ 2 standard deviations (S.D.) or mean $\pm$ 3 standard deviations (S.D.) can be used. Statistically, the mean $\pm$ 2 standard deviation (S.D.) includes values of 80% of the healthy individuals, and the mean $\pm$ 3 standard deviation (S.D.) includes values of 90% of the healthy individuals.

Alternatively, the level of LGR7 expression in the control can be established using a receiver operating characteristic (ROC) curve. An ROC curve is a graph that shows the detection sensitivity on the vertical axis and the false positive rate (that is, "1-specificity") on the horizontal axis. An ROC curve can be obtained by plotting changes in the sensitivity and false positive rate when continuously varying the standard value for determining the level of LGR7 expression in a biological sample.

The "standard value" for obtaining an ROC curve is a numerical value temporarily used for statistical analysis. Generally, the "standard value" for obtaining an ROC curve is varied continuously within a range that covers all the standard values that can be selected. For example, the standard value can be varied between the maximum and minimum of the measured values of LGR7 in the population to be analyzed.

Based on the obtained ROC curve, expected standard values for the desired detection sensitivity and accuracy can be selected. A standard value statistically established by an ROC curve and such is also called the "cut-off value". In cut-off value-based cancer detection methods, in step (2) mentioned above, the LGR7 expression level detected in (1) is compared with the cut-off value. Furthermore, the subject is detected of cancer when the LGR7 expression level detected in (1) is higher than the cut-off value.

The LGR7 expression level can be determined by any method. It is possible to determine the LGR7 expression level by evaluating the LGR7 mRNA quantity, LGR7 protein quantity, and biological activity of the LGR7 protein. The quantity of LGR7 mRNA or protein can be determined by the methods described herein.

Any animal species expressing an LGR7 protein can be the subject. For example, it is known that many nonhuman mammals such as chimpanzee (*Pan troglodytes*) (ENSPTRG00000016551), rhesus monkey (*Macaca mulatta*) (ENSMMUG00000004647), mouse (*Mus musculus*) (ENSMUSG00000034009), rat (*Rattus norvegicus*) (ENSRNOG00000024120), guinea pig (*Cavia porcellus*) (ENSCPOG00000015517), dog (*Canis familiaris*) (ENSCAFG00000008672), chicken (*Gallus gallus*) (ENSGALG00000009429) and the like express the LGR7 protein. Thus, these animals are included in the subjects. The subject may be a human. Needless to say, when a nonhuman animal is used as a subject, the LGR7 protein of the animal species will be detected.

There is no particular limitation on the method for detecting the LGR7 protein contained in a test sample; however, detection by an immunological method exemplified below using the anti-LGR7 antibody is preferable:

radioimmunoassay (RIA);
enzyme immunoassay (EIA);
fluoroimmunoassay (FIA);
luminescence immunoassay (LIA);
immunoprecipitation (IP);
turbidimetric immunoassay (TIA);
Western blotting (WB);
immunohistochemistry (IHC); and
single radial immunodiffusion (SRID).

Of these techniques, the immunohistochemistry (IHC) method comprises the step of detecting the LGR7 protein on a section of fixed tissues or cells collected from a patient suffering from cancer, and it is one of the preferable immunological assays for cancer diagnostic methods. The above immunological methods such as the immunohistochemistry (IHC) method are known to those skilled in the art.

That is, because LGR7 is a membrane protein whose expression is specifically increased in cancer cells, cancer cells or tissues can be detected by an anti-LGR7 antibody. Cancer cells contained in cells or tissues collected from the body are detected by the above-mentioned immunohistological analysis.

It is also possible to detect cancer tissues *in vivo* using an anti-LGR7 antibody. Disclosed is a method for detecting cancer which comprises the steps of: (1) administering to a subject an LGR7 protein-binding antibody labeled with a labeling substance such as a radioisotope; and (2) detecting accumulation of the labeling substance. To trace the antibody administered into the body, the antibody can be labeled so that it can be detected. For example, it is possible to monitor the *in vivo* behavior of an antibody labeled with a fluorescent or luminescent substance, or a radioisotope. An antibody labeled with a fluorescent or luminescent substance can be observed using an endoscope or laparoscope. With radioisotope, the localization of the antibody can be imaged by tracing radioactivity. The localization of the anti-LGR7 antibody in *vivo* represents the presence of cancer cells.

A positron-emitting nuclide can be used as a radioisotope for labeling the antibody for cancer detection *in vivo.* For example, the antibody can be labeled with a positron-emitting nuclide such as $^{18}$F, $^{55}$Co, $^{64}$Cu, $^{66}$Ga, $^{68}$Ga, $^{76}$Br, $^{89}$Zr and $^{124}$I. A known method (Acta Oncol. 32, 825-830, 1993) can be used for labeling the anti-LGR7 antibody with such a positron-emitting nuclide.

After administering the anti-LGR7 antibody labeled with a positron-emitting nuclide to a human or animal, the radiation emitted by the radionuclide is measured from outside the body by a positron emission tomography (PET) device, and this is converted to an image using a computerized tomography technique. PET is a device for non-invasive collection of data on the *in vivo* behavior and such of a drug. The radiation intensity can be quantitatively imaged by PET as signal strength. Using PET as described above, it is possible to detect an antigen molecule that is highly expressed in a specific cancer without collecting a sample from the patient. The anti-LGR7 antibody can be labeled with a short-lived nuclide using a positron-emitting nuclide such as $^{11}$C, $^{13}$N, $^{15}$O, $^{18}$F, $^{45}$Ti, and such, in addition to the above-mentioned nuclides.

Research and development of the production of short-lived nuclides using the above nuclides by a medical cyclotron, manufacturing techniques for compounds labeled with short-lived nuclides, and the like are progressing. An anti-LGR7 antibody can be labeled with various radioisotopes using these techniques. The anti-LGR7 antibody administered to a patient accumulates in primary and metastatic lesions in accordance with the anti-LGR7 antibody specificity for each site of pathological tissue. If the anti-LGR7 antibody is labeled with a positron-emitting nuclide, by determining radioactivity, the presence of the primary and metastatic lesions can be detected based on the localization of the radioactivity. For the diagnostic applications, the activity value of a gamma-particle or positron emission level of 25 to 4000 keV can be suitably used. In addition, if a suitable nuclide is selected and administered in a large quantity, therapeutic effects can also be expected. To obtain an anticancer effect by radiation, a nuclide providing a gamma-particle or positron emission level of 70 to 700 keV can be used.

In the methods as disclosed, LGR7 gene expression may be detected. There is no particular limitation on the gene detected; however, mRNA is preferable. "Detection" includes both quantitative and qualitative detection. Examples of qualitative detection include the following measuring operations:

measurement to simply determine whether or not LGR7 mRNA is present;
measurement to determine whether or not a predetermined amount or more of LGR7 mRNA is present; and
measurement to comparing the quantity of LGR7 mRNA with that of another sample (for example, a control sample, and such)

On the other hand, quantitative detection includes measurement of the concentration of LGR7 mRNA, the quantity of LGR7 mRNA, and such.

Any sample that possibly contains LGR7 mRNA can be used as a test sample. The sample may be a sample collected from the body of an organism such as a mammal. The sample may be a sample collected from a human. Specific examples of the test sample include blood, interstitial fluid, plasma, extravascular fluid, cerebrospinal fluid, synovial fluid, pleural fluid, serum, lymph, saliva, urine, tissues, ascites, and intraperitoneal lavage. The test sample may include a sample obtained from a test sample such as a specimen of fixed tissues or cells collected from the body of an organism, or a culture medium of cells.

When using samples obtained from test samples such as a specimen of fixed tissues or cells collected from the body of an organism, or a culture medium of cells, the *in situ* hybridization method can be suitably used. The *in situ* hybridization method has been developed as a means for determining the presence or distribution of specific DNA and RNA molecules in cells and tissues, and their expression intensity. The principle is that the method utilizes the property of specific complex formation of a specified nucleic acid sequence in a cell by a probe nucleic acid having a nucleotide sequence complementary thereto. The *in situ* hybridization method is used for detection of DNA, RNA, and such in cells, because if the probe is labeled with a radioisotope (RI) or an antigenic substance (hapten) in advance, then the site of hybridization can be distinguished by detecting the label. An RI label can be suitably used as the probe label. A more suitable example is the use of fluorescent labels utilizing non-radioactive substances such as haptens including biotin, digoxigenin, and the like. A particularly suitable example is the use of a detection method by fluorescence *in situ* hybridization called FISH.

Examples of cancers to be diagnosed include clear cell adenocarcinoma of ovarian cancer. Both primary and metastatic lesions of these cancers can be diagnosed.

Any animal species expressing the LGR7 protein can be used as a subject. For example, it is known that various mammals other than humans such as mice, rats, rhesus monkeys, and chimpanzees express LGR7. The subject may be human. When using a non-human animal species as the subject, LGR7 mRNA of the animal species will be detected.

A specific detection method is described below. First, a sample is prepared from a subject. Then, LGR7 mRNA contained in the sample is detected. cDNA synthesized from mRNA can also be detected. When an LGR7 mRNA or LGR7-encoding cDNA is detected in the test sample, it is determined that cancer may be possible. For example, when the quantity of LGR7 mRNA or LGR7-encoding cDNA detected in the test sample is greater than that in a negative control or healthy individual, this indicates that the subject has cancer or high possibility of suffering from cancer in the future.

Methods for detecting mRNA are known. Thus, methods such as northern blotting, RT-PCR, and DNA array can be used.

The above detection methods can be automated using various automatic detection devices. With automation, it is possible to test a large number of samples in a short time.

Disclosed is a diagnostic agent or kit for cancer diagnosis which comprises a reagent for detecting the LGR7 protein in a test sample. The diagnostic agent contains at least an anti-LGR7 antibody.

The kit for cancer diagnosis can be prepared by combining the cancer diagnostic agent with another component used for detecting LGR7. The kit for cancer diagnosis may contain an LGR7-binding antibody and a reagent that detects the binding between the antibody and LGR7. The kit may also include a control sample comprising a biological sample containing LGR7. The kit of the present invention may also be accompanied by instructions explaining the measurement procedure.

## Examples

Herein below, the present invention will be specifically described with reference to the Examples, but the technical scope of the present invention is not to be construed as being limited thereto.

[Example 1] Expression Analysis of Human LGR7 mRNA by Affymetrix U133 Plus 2.0 Array

Total RNA was extracted from surgical specimens of ten ovarian cancer cases that were collected after obtaining written consents at the University of Tokyo Hospital (Japan), and stored by freezing. Herein, the surgical samples were embedded in an OCT compound, and this was sliced and dissolved in TRIZOL (Invitrogen), and then total RNA was extracted according to the method described in the manual attached to the product. At the same time, HE-stained specimens were prepared to confirm that a cancerous part is included. The tissue types of the ten ovarian cancer cases are as follows: clear cell carcinomas (four cases), serous adenocarcinoma (two cases), endometrioid adenocarcinoma (three cases), and carcinosarcoma (one case). Expression analysis was performed by Affymetrix U-133 Plus 2.0 Array using these total RNAs, and genes showing high expression specifically in ovarian clear cell adenocarcinoma were selected. As controls, total RNAs derived from normal tissues (Clontech) and ovarian cancer cell lines (purchased from ATCC, JCRB, and Riken) were used.

The probe sets were narrowed down to 11761 sets that have an expression level of 200 or more in at least one out of four ovarian clear cell adenocarcinoma cases, which was used as a standard for selecting target molecules suitable for treatment of clear cell adenocarcinoma. Next, by comparing the third highest expression level among the four ovarian clear cell adenocarcinomas cases, and the highest expression level among normal ovary, peripheral blood, bone marrow, and major organs (liver, kidney, lung, stomach, intestine, and pancreas), the probe sets were further narrowed down to 197 sets showing a ratio of 1.8 or more. Of them, LGR7 was selected as the molecule that showed the highest value of the above ratio and which had not been reported to be related to ovarian clear cell adenocarcinoma. When making this selection, expression data of 87 ovarian cancer cases including three clear cell adenocarcinoma cases disclosed by the International Genomics Consortium (IGC) were taken into consideration. Fig. 1 shows a graph indicating the signal levels of LGR7 probe 1552715_a_at in ovarian cancers, ovarian cancer cell lines, and normal tissues. The meaning of the sample names are shown in Table 1.

Among ovarian cancers, LGR7 is expressed specifically in clear cell adenocarcinoma, and thus antitumor agents that target human LGR7 are expected to be effective for this type of cancer.

Table 1

| | | |
|---|---|---|
| Brain | normal tissue | brain |
| Muscle | normal tissue | skeletal muscle |
| Heart | normal tissue | heart |
| Skin | normal tissue | skin |
| Lung | normal tissue | lung |
| Liver | normal tissue | liver |
| Stomach | normal tissue | stomach |
| Colon | normal tissue | colon |
| Pancreas | normal tissue | pancreas |
| Kidney | normal tissue | kidney |
| Bone Marrow | normal tissue | bone marrow |
| Peripheral Blood | normal tissue | peripheral blood |
| Ovary | normal tissue | ovary |
| Testis | normal tissue | testis |
| Fetal Brain | normal tissue | fetal brain |
| Fetal Lung | normal tissue | fetal lung |
| Fetal Liver | normal tissue | fetal liver |
| Fetal Colon | normal tissue | fetal colon |
| Ovary3Ca | tissue isolated from ovarian cancer | ovarian clear cell adenocarcinoma |
| Ovary5Ca | tissue isolated from ovarian cancer | ovarian clear cell adenocarcinoma |
| Ovary7Ca | tissue isolated from ovarian cancer | ovarian clear cell adenocarcinoma |

(continued)

| | | |
|---|---|---|
| Ovary 19Ca | tissue isolated from ovarian cancer | ovarian clear cell adenocarcinoma |
| Ovary9Ca | tissue isolated from ovarian cancer | ovarian endometrioid adenocarcinoma |
| Ovary 13Ca | tissue isolated from ovarian cancer | ovarian endometrioid adenocarcinoma |
| Ovary 17Ca | tissue isolated from ovarian cancer | ovarian endometrioid adenocarcinoma |
| Ovary 1Ca | tissue isolated from ovarian cancer | ovarian serous adenocarcinoma |
| Ovary 15Ca | tissue isolated from ovarian cancer | ovarian serous adenocarcinoma |
| Ovary 11Ca | tissue isolated from ovarian cancer | ovarian carcinosarcoma |
| CP_JHOC_5 | ovarian cancer cell line | ovarian clear cell adenocarcinoma |
| CP_MCAS | ovarian cancer cell line | ovarian mucinous adenocarcinoma |
| CP_PMG_1 | ovarian cancer cell line | ovarian clear cell adenocarcinoma |
| CP_RMUG_S | ovarian cancer cell line | ovarian mucinous adenocarcinoma |
| CP_TKY_nu | ovarian cancer cell line | ovarian undifferentiated adenocarcinoma |

[Example 2] Establishment of Cells Expressing Full-length Human LGR7

Full-length human LGR7 cDNA was isolated by the PCR method using Human Uterus QUICK-CLONE cDNA (Clontech) based on NCBI Accession Nos. NP_067647 (SEQ ID NO: 1, amino acid sequence) and NM_021634 (SEQ ID NO: 2, nucleotide sequence). This was cloned into pGEM-T Easy (Promega), and an HA tag sequence was added to the N terminus. Then, this was cloned into the pMCN2i vector for expression in mammalian cells.

Gene introduction into the Chinese hamster ovary-derived DG44 cell line was carried out using the BioRad GenePulser to obtain the HA-LGR7-expressing cell line HA-LGR7/DG#24. Introduction into Ba/F3 which is a mouse pro-B cell was performed to obtain the HA-LGR7-expressing cell line HA-LGR/BaF3#48. LGR7 expression was confirmed by Western blotting using the HA-7 antibody (Sigma) against the HA tag (Fig. 2).

In addition, a vector into which the LGR7 gene is inserted was constructed for DNA immunization. The expression vector pMCN allows expression of an inserted gene under the mouse CMV promoter (Accession No. U68299), and it has a neomycin resistance gene incorporated as a drug resistance marker. The LGR7 gene was cloned into pMCN using a conventional method to prepare the LGR7 expression vector pMCN-LGR7.

[Example 3] Preparation of Anti-LGR7 Monoclonal Antibodies by DNA Immunization

DNA immunization by gene introduction into mice was carried out using the GeneGun Particle method. The method was performed according to the manual by Bio-Rad. The bullets for DNA immunization were prepared by mixing 1-mm gold particles (Bio-Rad) and pMCN-LGR7 DNA, and coating the inside of a tube with the mixture. Gene introduction was carried out by shooting the bullets coated with pMCN-LGR7 DNA into the abdominal skin of six-week-old female MRL/1pr mice using a Helios Gene Gun (Bio-Rad) at a pressure of 200 to 300 psi. It is thought that the gene introduced into keratinocytes, Langerhans cells, and dermal dendritic cells in the skin expresses the LGR7 protein, and thus the cells become antigen-presenting cells (APC) and induce immunity (Methods 31, 232-242 (2003); Immunization with DNA through the skin). DNA immunization was carried out six times at one-week intervals. For the final immunization, 1 x $10^6$ cells of the BaF3 cell line HA-LGR/BaF3#48 expressing LGR7 were diluted in PBS and then administered into the tail vein. Measurement of the antibody titer was performed by FACS analysis using HA-LGR7/DG#24 cells. The sera from the immunized mice were compared based on their reactivity to the LGR7 protein expressed on the cell membrane surface of HA-LGR7/DG#24 cells. The mouse showing the highest reactivity was subjected to final immunization and cell fusion. The spleen cells were isolated three days after the final immunization, and mixed at 2:1 ratio with P3-X63Ag8U1 mouse myeloma cells (P3U1, purchased from ATCC). Cell fusion was carried out by gradually adding PEG1500 (Roche Diagnostics), and hybridoma cells were prepared. The PEG1500 concentration was diluted by carefully adding RPMI 1640 medium (Gibco BRL), and then PEG1500 was removed by centrifugation. Next, the hybridoma cells were suspended in an RPMI 1640 medium containing 10% FBS, 1x HAT media supplement (SIGMA), and 0.5x BM-Condimed H1 Hybridoma cloning supplement (Roche Diagnostics) (hereinafter, HAT medium), and inoculated into a 96-well culture plate at 200 $\mu$L/well. The cell concentration at the time of inoculation was diluted according to the number of P3U1 cells used, and the hybridoma cells were cultured for about one week in HAT medium in the 96-well culture plate at 37°C under 5% $CO_2$. Screening of hybridomas that secrete an antibody into the culture supernatant was performed by flow cytometry.

[Example 4] Preparation of sLGR7Fc

A fragment containing the amino acids of positions 1 to 555 of the LGR7 protein was amplified by PCR, and a vector was constructed to express this fragment as a fusion protein with a human Fc protein (nucleotide sequence, SEQ ID NO: 95; amino acid sequence, SEQ ID NO: 96). The constructed vector was introduced into DG44 cells, and cells that can express the sLGR7Fc fusion protein were selected as a neomycin-resistant strain. The obtained cell strain was subjected to large-scale culture, and the culture supernatant was collected, and the sLGR7Fc protein was purified. The sLGR7Fc protein which was affinity-purified as an Fc fusion protein using an rProtein A column was used as an antigen for protein immunization and screening of hybridomas.

[Example 5] Production of Anti-LGR7 Antibodies by sLGR7Fc Protein Immunization

50 μg of the affinity-purified sLGR7Fc protein was mixed with Freund's complete adjuvant, and this was subcutaneously immunized into mice. Then, antibodies were induced by subcutaneous immunization of mice twice with a mixture of 50 μg of the sLGR7Fc protein and Freund's incomplete adjuvant. 25 μg of the sLGR7Fc protein was injected into the tail vein of the mouse showing the highest reactivity to the LGR7 protein. After three days, the spleen was removed from the mouse, and subjected to cell fusion with the mouse myeloma cell line P3 X63Ag8U.1, and hybridomas were prepared as in Example 3.

[Example 6] Evaluation of the Binding Activity by FACS (Flow Cytometry)

The binding of the obtained hybridomas to human LGR7/DG44 cells was evaluated by flow cytometry. The cell line expressing human LGR7 suspended in FACS buffer (2% FBS/PBS/0.05% $NaN_3$) was diluted to 1 x $10^6$ cells/mL using FACS buffer, and this was aliquoted into a Falcon 353910 round-bottom 96-well plate at 50 μL/well. The hybridoma culture supernatant diluted to a suitable concentration was added to the wells containing the cells, and this was reacted for 60 minutes on ice. Next, the cells were rinsed once with FACS buffer. As a secondary antibody, a goat $F(ab')_2$ fragment anti-mouse IgG Fcy-FITC (Beckman Coulter) was added to the wells containing the cells, and this was reacted for 30 minutes on ice. After the reaction, the supernatant was removed by centrifugation, and the cells were suspended in 100 μL of FACS buffer, and subjected to flow cytometry. The FACS Calibur (Becton Dickinson) was used for the flow cytometry. A gate for the viable cell population was established using a forward scatter/side scatter dot blot, and an FL1 histogram was produced for the cells included in the population, and their binding activity was evaluated.

The hybridoma supernatants were reacted with DG44 cells induced to express LGR7 and the parental DG44 cells, respectively. Thus, hybridomas that specifically react with LGR7-expressing cells were obtained. The hybridomas from the wells were made into single clones by the limiting dilution method. The isotype of each antibody was analyzed using an IsoStrip® mouse monoclonal antibody isotyping kit (Roche Diagnostics). As a result, the isotype of 22DA6, 22DA7, 22DA11, 22DA12, 22DA23, and 22DA24 is IgG1; and the isotype of 22DA4, 22DA10, 22SD7, 22SD25, 22SD31, and 22SD48 is IgG2a; and the isotype of 22DA17 and 22DA20 is IgG2b. Expansion culture of a single-clone hybridoma was performed, and then the antibody was purified from the culture supernatant using a protein G column according to the manual. The purified antibody was subjected to protein quantification by DC Protein Assay, etc.

[Example 7] Measurement of the ADCC Activity of the Anti-LGR7 Monoclonal Antibodies

The ADCC activities of the anti-human LGR7 monoclonal antibodies against DG44 cells forcedly expressing LGR7 were evaluated by the chromium release method. Target cells were cultured for a few hours in a culture solution supplemented with Chromium-51 (CHO-S SFM II, manufactured by Invitrogen). Then, the culture solution was removed, and the cells were rinsed with a culture medium. The cells were suspended in a fresh culture medium, and this was added to a 96-well round-bottom plate at 1 x $10^4$ cells per well. Next, the antibody was added at final concentrations of 1 μg/mL or 0.1 μg/mL, and effector cells (recombinant cells (WO2008/093688) produced by forced expression of a chimeric protein comprising the extracellular domain of mouse Fc-gamma receptor 3 (NM_010188) and the transmembrane and intracellular domains of the human gamma chain (NM_004106) in NK-92 (ATCC, CRL-2407)), were added to each well at approximately five times the amount of the target cells. The plate was left to stand for four hours at 37°C in a 5% $CO_2$ incubator. Subsequently, the plate was centrifuged, and a fixed amount of the supernatant was collected from each well. The radioactivity was measured using a Wallac 1480 gamma counter, and the specific chromium release rate (%) was determined using the following equation:

$$\text{Specific chromium release rate (\%)} = (\text{A-C}) \times 100 / (\text{B-C})$$

where A represents the radioactivity in each well; B represents the mean value of radioactivity released into the medium upon cell lysis with Nonidet P-40 at a final concentration of 1%; and C represents the mean value of radioactivity when the medium alone is added.

As a result, as indicated in Fig. 3, of the anti-human LGR7 monoclonal antibodies used in the test, in particular 22DA6, 22DA7, 22DA17, 22DA22, 22DA23, 22DA24, 22SD7, 22SD25, and 22SD38 induced very strong ADCC activities against human LGR7-expressing cells. The result indicates that anti-tumor antibody therapy that targets human LGR7 is very useful.

[Example 8] Measurement of the CDC Activity of the Anti-LGR7 Monoclonal Antibodies

The CDC activity was measured using the extent of 7-AAD uptake by cells in which cell injury has occurred as an indicator.

LGR7-expressing DG44 cells were reacted with the monoclonal antibodies at a concentration of 10 μg/mL at 4°C for 30 minutes. Next, Baby Rabbit Complement (Cedarlane Laboratories) was added thereto at a final concentration of 10%, and the reaction was further performed for 90 minutes at 37°C. After adding 7-AAD (Beckman Coulter) at a final concentration of 1 μg/mL, this was left to stand for 10 minutes at room temperature. Thereafter, the cells were rinsed with FACS buffer, and then the ratio of cells in which cell injury has occurred was evaluated by FACS Calibur. The value of "% FL3" shows the ratio of 7-AAD-stained cells in which cell injury has occurred. As indicated in Fig. 4, multiple anti-LGR7 antibodies showed complement-dependent cytotoxicity (CDC) activity against DG44 cells expressing HA-LGR7.

[Example 9] Cloning of the Antigen Genes

Sequences of the antibody variable region genes of the nine hybridomas, 22DA6, 22DA7, 22DA17, 22DA22, 22DA23, 22DA24, 22SD7, 22SD11, and 22SD48, which showed ADCC activity and CDC activity, were determined. The antibody genes were amplified by the RT-PCR method using total RNAs extracted from the hybridomas producing the anti-LGR7 antibodies. Total RNA was extracted from 1 x $10^7$ hybridoma cells using the RNeasy Plant Mini Kit (QIAGEN). A RACE library was constructed using 1 μg of total RNA and the SMART RACE cDNA Amplification Kit (Clontech). Using synthetic oligonucleotides complementary to the mouse IgG1 constant region sequences, MHC-IgG1 (SEQ ID NO: 97, GGGCCAGTGGATAGACAGATG), MHC-IgG2a (SEQ ID NO: 98, CAGGGGCCAGTGGATAGACCGATG), MHC-IgG2b (SEQ ID NO: 99, CAGGGGCCAGTGGATAGACTGATG), or a synthetic oligonucleotide complementary to the mouse κ chain constant region nucleotide sequence, kappa (SEQ ID NO: 100, GCTCACTGGATGGTGGGAAGATG), a 5'-end gene fragment was amplified from the gene encoding the antibody produced by the hybridoma. Reverse transcription reaction was carried out at 42°C for 1.5 hours. 50 μL of the PCR solution contained 5 μL of 10x Advantage 2 PCR Buffer, 5 μL of 10x Universal Primer A Mix, 0.2 mM dNTPs (dATP, dGTP, dCTP, and dTTP), 1 μL of Advantage 2 Polymerase Mix (all manufactured by Clontech), 2.5 μL of reverse transcription reaction product, and 10 pmol of the synthetic oligonucleotide MHC-IgG1, MHC-IgG2a, MHC-IgG2b, or kappa. PCR reaction was carried out as follows: reaction at a starting temperature of 94°C for 30 seconds; five cycles of 94°C for five seconds and 72°C for three minutes; five cycles of 94°C for five seconds, 70°C for ten seconds, and 72°C for three minutes; and then 25 cycles of 94°C for five seconds, 68°C for ten seconds, and 72°C for three minutes. Finally, the reaction product was heated at 72°C for seven minutes. Each PCR product was purified from agarose gel using the QIAquick Gel Extraction Kit (manufactured by QIAGEN). Then, the PCR product was cloned into the pGEM-T Easy vector (manufactured by Promega), and its nucleotide sequence was determined.

For 22DA6, the nucleotide and amino acid sequences of the H-chain variable region are shown in SEQ ID NO: 3 and SEQ ID NO: 4, respectively; and the nucleotide and amino acid sequences of the L-chain variable region are shown in SEQ ID NO: 8 and SEQ ID NO: 9, respectively. Furthermore, for 22DA6, SEQ ID NO: 5 shows the amino acid sequence of heavy-chain CDR1, SEQ ID NO: 6 shows the amino acid sequence of heavy-chain CDR2, SEQ ID NO: 7 shows the amino acid sequence of heavy-chain CDR3, SEQ ID NO: 10 shows the amino acid sequence of light-chain CDR1, SEQ ID NO: 11 shows the amino acid sequence of light-chain CDR2, and SEQ ID NO: 12 shows the amino acid sequence of light-chain CDR3.

For 22DA7, the nucleotide and amino acid sequences of the H-chain variable region are shown in SEQ ID NO: 13 and SEQ ID NO: 14, respectively; and the nucleotide and amino acid sequences of the L-chain variable region are shown in SEQ ID NO: 18 and SEQ ID NO: 19, respectively. Furthermore, for 22DA7, SEQ ID NO: 15 shows the amino acid sequence of heavy-chain CDR1, SEQ ID NO: 16 shows the amino acid sequence of heavy-chain CDR2, SEQ ID NO: 17 shows the amino acid sequence of heavy-chain CDR3, SEQ ID NO: 20 shows the amino acid sequence of light-chain CDR1, SEQ ID NO: 21 shows the amino acid sequence of light-chain CDR2, and SEQ ID NO: 22 shows the amino acid sequence of light-chain CDR3.

For 22DA17, the nucleotide and amino acid sequences of the H-chain variable region are shown in SEQ ID NO: 23 and SEQ ID NO: 24, respectively; and the nucleotide and amino acid sequences of the L-chain variable region are shown in SEQ ID NO: 28 and SEQ ID NO: 29, respectively. Furthermore, for 22DA17, SEQ ID NO: 25 shows the amino acid sequence of heavy-chain CDR1, SEQ ID NO: 26 shows the amino acid sequence of heavy-chain CDR2, SEQ ID NO: 27 shows the amino acid sequence of heavy-chain CDR3, SEQ ID NO: 30 shows the amino acid sequence of light-chain CDR1, SEQ ID NO: 31 shows the amino acid sequence of light-chain CDR2, and SEQ ID NO: 32 shows the amino acid sequence of light-chain CDR3.

For 22DA22, the nucleotide and amino acid sequences of the H-chain variable region are shown in SEQ ID NO: 33 and SEQ ID NO: 34, respectively; and the nucleotide and amino acid sequences of the L-chain variable region are shown in SEQ ID NO: 38 and SEQ ID NO: 39, respectively. Furthermore, for 22DA22, SEQ ID NO: 35 shows the amino acid sequence of heavy-chain CDR1, SEQ ID NO: 36 shows the amino acid sequence of heavy-chain CDR2, SEQ ID NO: 37 shows the amino acid sequence of heavy-chain CDR3, SEQ ID NO: 40 shows the amino acid sequence of light-chain CDR1, SEQ ID NO: 41 shows the amino acid sequence of light-chain CDR2, and SEQ ID NO: 42 shows the amino acid sequence of light-chain CDR3.

For 22DA23, the nucleotide and amino acid sequences of the H-chain variable region are shown in SEQ ID NO: 43 and SEQ ID NO: 44, respectively; and the nucleotide and amino acid sequences of the L-chain variable region are shown in SEQ ID NO: 48 and SEQ ID NO: 49, respectively. Furthermore, for 22DA23, SEQ ID NO: 45 shows the amino acid sequence of heavy-chain CDR1, SEQ ID NO: 46 shows the amino acid sequence of heavy-chain CDR2, SEQ ID NO: 47 shows the amino acid sequence of heavy-chain CDR3, SEQ ID NO: 50 shows the amino acid sequence of light-chain CDR1, SEQ ID NO: 51 shows the amino acid sequence of light-chain CDR2, and SEQ ID NO: 52 shows the amino acid sequence of light-chain CDR3.

For 22DA24, the nucleotide and amino acid sequences of the H-chain variable region are shown in SEQ ID NO: 53 and SEQ ID NO: 54, respectively; and the nucleotide and amino acid sequences of the L-chain variable region are shown in SEQ ID NO: 58 and SEQ ID NO: 59, respectively. Furthermore, for 22DA24, SEQ ID NO: 55 shows the amino acid sequence of heavy-chain CDR1, SEQ ID NO: 56 shows the amino acid sequence of heavy-chain CDR2, SEQ ID NO: 57 shows the amino acid sequence of heavy-chain CDR3, SEQ ID NO: 60 shows the amino acid sequence of light-chain CDR1, SEQ ID NO: 61 shows the amino acid sequence of light-chain CDR2, and SEQ ID NO: 62 shows the amino acid sequence of light-chain CDR3.

For 22SD7, the nucleotide and amino acid sequences of the H-chain variable region are shown in SEQ ID NO: 63 and SEQ ID NO: 64, respectively; and the nucleotide and amino acid sequences of the L-chain variable region are shown in SEQ ID NO: 68 and SEQ ID NO: 69, respectively. Furthermore, for 22SD7, SEQ ID NO: 65 shows the amino acid sequence of heavy-chain CDR1, SEQ ID NO: 66 shows the amino acid sequence of heavy-chain CDR2, SEQ ID NO: 67 shows the amino acid sequence of heavy-chain CDR3, SEQ ID NO: 70 shows the amino acid sequence of light-chain CDR1, SEQ ID NO: 71 shows the amino acid sequence of light-chain CDR2, and SEQ ID NO: 72 shows the amino acid sequence of light-chain CDR3.

For 22SD11, the nucleotide and amino acid sequences of the H-chain variable region are shown in SEQ ID NO: 73 and SEQ ID NO: 74, respectively; and the nucleotide and amino acid sequences of the L-chain variable region are shown in SEQ ID NO: 78 and SEQ ID NO: 79, respectively. Furthermore, for 22SD11, SEQ ID NO: 75 shows the amino acid sequence of heavy-chain CDR1, SEQ ID NO: 76 shows the amino acid sequence of heavy-chain CDR2, SEQ ID NO: 77 shows the amino acid sequence of heavy-chain CDR3, SEQ ID NO: 80 shows the amino acid sequence of light-chain CDR1, SEQ ID NO: 81 shows the amino acid sequence of light-chain CDR2, and SEQ ID NO: 82 shows the amino acid sequence of light-chain CDR3.

For 22SD48, the nucleotide and amino acid sequences of the H-chain variable region are shown in SEQ ID NO: 83 and SEQ ID NO: 84, respectively; and the nucleotide and amino acid sequences of the L-chain variable region are shown in SEQ ID NO: 88 and SEQ ID NO: 89, respectively. Furthermore, for 22SD48, SEQ ID NO: 85 shows the amino acid sequence of heavy-chain CDR1, SEQ ID NO: 86 shows the amino acid sequence of heavy-chain CDR2, SEQ ID NO: 87 shows the amino acid sequence of heavy-chain CDR3, SEQ ID NO: 90 shows the amino acid sequence of light-chain CDR1, SEQ ID NO: 91 shows the amino acid sequence of light-chain CDR2, and SEQ ID NO: 92 shows the amino acid sequence of light-chain CDR3.

[Example 10] <u>Cell-killing Effect by Internalization of Mab-Zap</u>

Mab-Zap (manufactured by Advanced Targeting Systems) which is a secondary antibody bound to a toxin called saporin was used to evaluate the cell killing ability against LGR7-expressing cells. For the development model of an antibody pharmaceutical, the mechanism of action is that an antibody bound to a toxin or such binds to a target cell, and is incorporated into the cell, and then kills the target cell by the action of the conjugated toxin. BaF3 cells made to express HA-LGR7 were used. 100 ng of the antibody and 100 ng of Mab-Zap were added to each well. After incubation at 37°C in a 5% $CO_2$ incubator for three days, the number of viable cells was determined by WST8 assay using the cell viability assay reagent SF (Nakalai Tesque). The results are shown in Fig. 5. Compared to a control antibody of the same isotype, the values of WST8 assay were clearly lower for the cells co-treated with Mab-ZAP, and the cell-killing effect can be confirmed in all of the analyzed antibodies. This shows that antibodies labeled with a toxin or radioisotope can be incorporated into cells and kill these cells.

[Example 11] Reactivity of anti-LGR7 monoclonal antibodies to mouse LGR7

Mouse LGR7 (nucleotide sequence, SEQ ID NO: 93; amino acid sequence, SEQ ID NO: 94) was inserted into an expression vector. Cross-reactivity towards mouse LGR7 was evaluated by flow cytometry using the cell line of forced expression HA-mLGR7/BaF3 obtained by gene introduction into BaF3 cells. As shown in Fig. 6, 22DA17 and 22DA23 were found to exhibit cross-reactivity towards mouse LGR7.

[Example 12] Epitope Classification by Competition FACS Analysis

The epitopes were classified by competition FACS analysis. Antibodies were biotinylated using the Biotin Protein Labeling Kit (Roche) according to the manual. Competition FACS analysis is a method in which an excess amount of unlabeled antibody is reacted in advance, a biotinylated antibody is reacted, and the biotinylated antibody is subsequently detected with an FITC-labeled streptavidin. When the antibodies recognize the same epitope, the biotinylated antibody is unable to access the antigen since the unlabeled antibody masks the epitope, and the peak shifts to the left in FACS analysis. When the unlabeled antibody and biotinylated antibody of the same antibody are reacted in order, competition occurs and the shift moves to the left as compared to when an FITC-labeled anti-mouse antibody is reacted. On the other hand, when the antibodies recognize different epitopes, the binding can take place without competition with the unlabeled antibody, and thus the shift to the left is small. Several antibodies were biotinylated and competition assay was carried out. Some of the results are shown in Fig. 7. From competition FACS analysis using the biotinylated Bio-22DA17 and Bio-22DA22 antibodies, it was found that 22DA12 and 22DA22 are antibodies that recognize epitopes different from those of other antibodies.

[Example 13] Production of a Mouse IgG2a Chimeric Antibody with Mouse IgG2a CH and CL

As methods for enhancing the ADCC activity of an antibody, methods of modifying antibody sugar chains are known. WO2006/067913 and such describe the production of antibodies carrying a sugar chain that does not include $\alpha$-1,6-core fucose by using CHO cells in which the fucose transporter gene is knocked out (CHO_FTKO).

The H-chain and L-chain variable regions of the antibody gene of the anti-human LGR7 monoclonal antibody 22DA23 cloned as described in Example 9 were respectively amplified by PCR. They were respectively ligated to the H-chain C region $C\gamma 2a$ and L-chain C region $C\kappa$ of a mouse antibody, and this was inserted into an expression vector for mammalian cells so that they can be expressed as a mouse IgG2a chimeric molecule. The obtained vector was transfected into the fucose transporter-deficient CHO cell CHO_FTKO to establish a neomycin-resistant strain.

The cells were cultured in RPMI-1640/10% Ultra-low IgG FBS (Invitrogen)/500 $\mu$g/mL Geneticin in the presence of penicillin/streptomycin, and the mouse IgG2a chimeric antibody was purified from the culture supernatant using a Protein A column according to the manual. The purified 22DA23-mIgG2a/FTPKO antibody was subjected to a drug efficacy test using a mouse xenograft model.

[Example 14] Drug Efficacy Test Using a Mouse Xenograft Model

The purified 22DA23-mIgG2a/FTPKO (FTKODA23) antibody was subjected to a drug efficacy test in mice. An approximately 3-mm-square piece of RMG-1 *in vivo* passaged tumor was transplanted subcutaneously into seven-week-old Scid female mice (Clea Japan). Ten days after transplantation, groups were formed according to the tumor volume and body weight, and they were subjected to the examination. For the RMG-1 *in vivo* passaged tumor, a tumor isolated 42 days after subcutaneous transplantation of cultured RMG-1 cells at 1e7 cells/body was used. A group included six mice, and the FTKODA23 antibody was administered to the mice once a week at 2 mg/kg or 10 mg/kg. PBS(-) was administered to the control group. The first administration was carried out ten days after transplantation, the second administration 17 days after transplantation, the third administration 24 days after transplantation, and the fourth administration 31 days after transplantation. The tumor volume was measured twice a week, and the final measurement was performed one week after the fourth administration. A graph showing the changes in the tumor volume is presented in Fig. 8. The tumor volumes of the control group and the antibody administration group were compared. TGI (Tumor Growth Inhibition) was 37% on average in the 10 mg/kg administration group and 33% on average in the 2 mg/kg administration group, and the tumor regression effect was confirmed.

Industrial Applicability

The anti-LGR7 antibodies of the present invention or as disclosed can exhibit anticancer effects by antibody-dependent cell-mediated cytotoxicity activity and complement-dependent cytotoxicity activity towards LGR7-expressing cells. Furthermore, when conjugated to a toxin (cell-damaging substance), they can cause cell injury in LGR7-expressing cells, thus they are useful for diagnosis, prevention, and treatment of various primary and metastatic cancers.
The LGR7 protein-specific antibodies of the present invention or as disclosed are specifically expressed, in particular, in clear cell adenocarcinoma of ovarian cancer, and thus can be used as agents for diagnosing clear cell adenocarcinoma. The diagnostic agents are useful for diagnosis of primary and metastatic cancers. More specifically, the possibility of cancer can be evaluated by detecting the LGR7 protein contained in a biological sample collected from a patient. Alternatively, the presence of ovarian clear cell adenocarcinoma can be determined *in vivo* by detecting the localization of LGR7-expressing cells *in vivo.*
In addition, the anti-LGR7 antibodies having cytotoxic activity of the present invention or as disclosed are useful for prevention and treatment of cancers expressing the LGR7 protein. Disclosed are cytotoxic agents and cell growth inhibitors against cancer cells of ovarian clear cell adenocarcinoma. The cytotoxic agents and cell growth inhibitors against cancer cells can be applied to both primary and metastatic cancers.
Furthermore, the anti-LGR7 antibodies having cytotoxic activity of the present invention or as disclosed can be used as therapeutic drugs for ovarian clear cell carcinoma. In the present invention, the anti-LGR7 antibodies are useful as therapeutic drugs against both primary and metastatic cancers.
Additionally, genes encoding the antibodies of the present invention or as disclosed and recombinant cells transformed with the genes can be used to prepare recombinant antibodies that have the above effects or more preferable effects.

## Claims

**1.** A monoclonal antibody of any one of (1) to (2) below that binds to an LGR7 protein, wherein the antibody has internalization activity against cells expressing the LGR7 protein:

(1) an antibody comprising an H chain having the amino acid sequence of SEQ ID NO: 25 as CDR1, the amino acid sequence of SEQ ID NO: 26 as CDR2, and the amino acid sequence of SEQ ID NO: 27 as CDR3 and comprising an L chain having the amino acid sequence of SEQ ID NO: 30 as CDR1, the amino acid sequence of SEQ ID NO: 31 as CDR2, and the amino acid sequence of SEQ ID NO: 32 as CDR3;
(2) an antibody that competes with the antibody of (1) for binding to the epitope that the antibody of (1) binds to.

**2.** The antibody of (2) in claim 1, wherein the antibody is selected from any one of (a) to (g):

(a) an antibody comprising an H chain having the amino acid sequence of SEQ ID NO: 5 as CDR1, the amino acid sequence of SEQ ID NO: 6 as CDR2, and the amino acid sequence of SEQ ID NO: 7 as CDR3 and comprising an L chain having the amino acid sequence of SEQ ID NO: 10 as CDR1, the amino acid sequence of SEQ ID NO: 11 as CDR2, and the amino acid sequence of SEQ ID NO: 12 as CDR3;
(b) an antibody comprising an H chain having the amino acid sequence of SEQ ID NO: 15 as CDR1, the amino acid sequence of SEQ ID NO: 16 as CDR2, and the amino acid sequence of SEQ ID NO: 17 as CDR3 and comprising an L chain having the amino acid sequence of SEQ ID NO: 20 as CDR1, the amino acid sequence of SEQ ID NO: 21 as CDR2, and the amino acid sequence of SEQ ID NO: 22 as CDR3;
(c) an antibody comprising an H chain having the amino acid sequence of SEQ ID NO: 45 as CDR1, the amino acid sequence of SEQ ID NO: 46 as CDR2, and the amino acid sequence of SEQ ID NO: 47 as CDR3 and comprising an L chain having the amino acid sequence of SEQ ID NO: 50 as CDR1, the amino acid sequence of SEQ ID NO: 51 as CDR2, and the amino acid sequence of SEQ ID NO: 52 as CDR3;
(d) an antibody comprising an H chain having the amino acid sequence of SEQ ID NO: 55 as CDR1, the amino acid sequence of SEQ ID NO: 56 as CDR2, and the amino acid sequence of SEQ ID NO: 57 as CDR3 and comprising an L chain having the amino acid sequence of SEQ ID NO: 60 as CDR1, the amino acid sequence of SEQ ID NO: 61 as CDR2, and the amino acid sequence of SEQ ID NO: 62 as CDR3;
(e) an antibody comprising an H chain having the amino acid sequence of SEQ ID NO: 65 as CDR1, the amino acid sequence of SEQ ID NO: 66 as CDR2, and the amino acid sequence of SEQ ID NO: 67 as CDR3 and comprising an L chain having the amino acid sequence of SEQ ID NO: 70 as CDR1, the amino acid sequence of SEQ ID NO: 71 as CDR2, and the amino acid sequence of SEQ ID NO: 72 as CDR3;
(f) an antibody comprising an H chain having the amino acid sequence of SEQ ID NO: 75 as CDR1, the amino acid sequence of SEQ ID NO: 76 as CDR2, and the amino acid sequence of SEQ ID NO: 77 as CDR3 and comprising an L chain having the amino acid sequence of SEQ ID NO: 80 as CDR1, the amino acid sequence of SEQ ID NO: 81 as CDR2, and the amino acid sequence of SEQ ID NO: 82 as CDR3;
(g) an antibody comprising an H chain having the amino acid sequence of SEQ ID NO: 85 as CDR1, the amino acid sequence of SEQ ID NO: 86 as CDR2, and the amino acid sequence of SEQ ID NO: 87 as CDR3 and comprising an L chain having the amino acid sequence of SEQ ID NO: 90 as CDR1, the amino acid sequence of SEQ ID NO: 91 as CDR2, and the amino acid sequence of SEQ ID NO: 92 as CDR3.

**3.** The antibody of any one of claims 1 to 2, which is an antibody that has cytotoxic activity; particularly wherein the cytotoxic activity is antibody-dependent cell-mediated cytotoxicity activity or wherein the cytotoxic activity is complement-dependent cytotoxicity activity.

**4.** The antibody of any one of claims 1 to 3, which is an antibody to which a cytotoxic substance is bound.

**5.** The antibody of any one of claims 1 to 4, which is an antibody that suppresses the growth of cancer cells, particularly wherein the cancer cells are clear cell ovarian cancer cells.

**6.** The antibody of any one of claims 1 to 5, wherein the antibody has a human constant region, particularly wherein the antibody is a chimeric antibody, humanized antibody, or human antibody.

**7.** The antibody of any one of claims 1 to 6, wherein the antibody is a fucose-deficient antibody.

**8.** The antibody of any one of claims 1 to 7 for use in the treatment of cancer.

**9.** The antibody for use of claim 8, wherein the cancer is ovarian cancer; particularly wherein the ovarian cancer is clear cell adenocarcinoma.

## Patentansprüche

**1.** Monoklonaler Antikörper nach einem beliebigen von (1) bis (2) nachstehend, der an ein LGR7-Protein bindet, wobei der Antikörper Internalisierungsaktivität gegen Zellen aufweist, die das LGR7-Protein exprimieren:

(1) ein Antikörper, umfassend eine H-Kette, die die Aminosäuresequenz von SEQ ID NR: 25 als CDR1, die Aminosäuresequenz von SEQ ID NR: 26 als CDR2 und die Aminosäuresequenz von SEQ ID NR: 27 als CDR3 aufweist, und umfassend eine L-Kette, die die Aminosäuresequenz von SEQ ID NR: 30 als CDR1, die Aminosäuresequenz von SEQ ID NR: 31 als CDR2 und die Aminosäuresequenz von SEQ ID NR: 32 als CDR3 aufweist;
(2) ein Antikörper, der mit dem Antikörper von (1) um das Binden an das Epitop kompetitiert, an das der Antikörper von (1) bindet.

**2.** Antikörper von (2) in Anspruch 1, wobei der Antikörper ausgewählt ist von einem beliebigen von (a) bis (g):

(a) einem Antikörper, umfassend eine H-Kette, die die Aminosäuresequenz von SEQ ID NR: 5 als CDR1, die Aminosäuresequenz von SEQ ID NR: 6 als CDR2 und die Aminosäuresequenz von SEQ ID NR. 7 als CDR3 aufweist, und umfassend eine L-Kette, die die Aminosäuresequenz von SEQ ID NR: 10 als CDR1, die Aminosäuresequenz von SEQ ID NR: 11 als CDR2 und die Aminosäuresequenz von SEQ ID NR: 12 als CDR3 aufweist;
(b) einem Antikörper, umfassend eine H-Kette, die die Aminosäuresequenz von SEQ ID NR: 15 als CDR1, die Aminosäuresequenz von SEQ ID NR: 16 als CDR2 und die Aminosäuresequenz von SEQ ID NR. 17 als CDR3 aufweist, und umfassend eine L-Kette, die die Aminosäuresequenz von SEQ ID NR: 20 als CDR1, die Aminosäuresequenz von SEQ ID NR: 21 als CDR2 und die Aminosäuresequenz von SEQ ID NR: 22 als CDR3 aufweist;
(c) einem Antikörper, umfassend eine H-Kette, die die Aminosäuresequenz von SEQ ID NR: 45 als CDR1, die Aminosäuresequenz von SEQ ID NR: 46 als CDR2 und die Aminosäuresequenz von SEQ ID NR. 47 als CDR3 aufweist, und umfassend eine L-Kette, die die Aminosäuresequenz von SEQ ID NR: 50 als CDR1, die Aminosäuresequenz von SEQ ID NR: 51 als CDR2 und die Aminosäuresequenz von SEQ ID NR: 52 als CDR3 aufweist;
(d) einem Antikörper, umfassend eine H-Kette, die die Aminosäuresequenz von SEQ ID NR: 55 als CDR1, die Aminosäuresequenz von SEQ ID NR: 56 als CDR2 und die Aminosäuresequenz von SEQ ID NR. 57 als CDR3 aufweist, und umfassend eine L-Kette, die die Aminosäuresequenz von SEQ ID NR: 60 als CDR1, die Aminosäuresequenz von SEQ ID NR: 61 als CDR2 und die Aminosäuresequenz von SEQ ID NR: 62 als CDR3 aufweist;
(e) einem Antikörper, umfassend eine H-Kette, die die Aminosäuresequenz von SEQ ID NR: 65 als CDR1, die Aminosäuresequenz von SEQ ID NR: 66 als CDR2 und die Aminosäuresequenz von SEQ ID NR. 67 als CDR3 aufweist, und umfassend eine L-Kette, die die Aminosäuresequenz von SEQ ID NR: 70 als CDR1, die Aminosäuresequenz von SEQ ID NR: 71 als CDR2 und die Aminosäuresequenz von SEQ ID NR: 72 als CDR3 aufweist;
(f) einem Antikörper, umfassend eine H-Kette, die die Aminosäuresequenz von SEQ ID NR: 75 als CDR1, die Aminosäuresequenz von SEQ ID NR: 76 als CDR2 und die Aminosäuresequenz von SEQ ID NR. 77 als CDR3 aufweist, und umfassend eine L-Kette, die die Aminosäuresequenz von SEQ ID NR: 80 als CDR1, die Aminosäuresequenz von SEQ ID NR: 81 als CDR2 und die Aminosäuresequenz von SEQ ID NR: 82 als CDR3 aufweist;
(g) einem Antikörper, umfassend eine H-Kette, die die Aminosäuresequenz von SEQ ID NR: 85 als CDR1, die Aminosäuresequenz von SEQ ID NR: 86 als CDR2 und die Aminosäuresequenz von SEQ ID NR. 87 als CDR3 aufweist, und umfassend eine L-Kette, die die Aminosäuresequenz von SEQ ID NR: 90 als CDR1, die Aminosäuresequenz von SEQ ID NR: 91 als CDR2 und die Aminosäuresequenz von SEQ ID NR: 92 als CDR3 aufweist.

**3.** Antikörper nach einem beliebigen der Ansprüche 1 bis 2, der ein Antikörper ist, der zytotoxische Aktivität aufweist; insbesondere wobei die zytotoxische Aktivität antikörperabhängige zellvermittelte Zytotoxizitätsaktivität ist oder wobei die zytotoxische Aktivität komplementabhängige Zytotoxizitätsaktivität ist.

**4.** Antikörper nach einem beliebigen der Ansprüche 1 bis 3, der ein Antikörper ist, an den eine zytotoxische Substanz gebunden ist.

**5.** Antikörper nach einem beliebigen der Ansprüche 1 bis 4, der ein Antikörper ist, der das Wachstum von Krebszellen unterdrückt, insbesondere wobei die Krebszellen klarzellige Eierstockkrebszellen sind.

**6.** Antikörper nach einem beliebigen der Ansprüche 1 bis 5, wobei der Antikörper eine humane konstante Region aufweist, insbesondere wobei der Antikörper ein chimärer Antikörper, humanisierter Antikörper oder humaner Antikörper ist.

**7.** Antikörper nach einem beliebigen der Ansprüche 1 bis 6, wobei der Antikörper ein Fucose-defizienter Antikörper ist.

**8.** Antikörper nach einem beliebigen der Ansprüche 1 bis 7 zur Verwendung bei der Behandlung von Krebs.

**9.** Antikörper zur Verwendung nach Anspruch 8, wobei der Krebs Eierstockkrebs ist; insbesondere wobei der Eierstockkrebs klarzelliges Adenokarzinom ist.

**Revendications**

**1.** Anticorps monoclonal selon l’un quelconque de (1) à (2) ci-dessous qui se lie à une protéine LGR7, où l’anticorps a une activité d’internalisation contre les cellules exprimant la protéine LGR7 :

(1) anticorps comprenant une chaîne H ayant la séquence d’acides aminés de la SEQ ID No: 25 à titre de CDR1, la séquence d’acides aminés de la SEQ ID No: 26 à titre de CDR2, et la séquence d’acides aminés de la SEQ ID No: 27 à titre de CDR3 et comprenant une chaîne L ayant la séquence d’acides aminés de la SEQ ID No: 30 à titre de CDR1, la séquence d’acides aminés de la SEQ ID No: 31 à titre de CDR2, et la séquence d’acides aminés de la SEQ ID No: 32 à titre de CDR3 ;
(2) anticorps qui est en compétition avec l’anticorps selon (1) pour la liaison à l’épitope auquel l’anticorps selon (1) se lie.

**2.** Anticorps selon (2) dans la revendication 1, où l’anticorps est choisi parmi l’un quelconque de (a) à (g) :

(a) anticorps comprenant une chaîne H ayant la séquence d’acides aminés de la SEQ ID No: 5 à titre de CDR1, la séquence d’acides aminés de la SEQ ID No: 6 à titre de CDR2, et la séquence d’acides aminés de la SEQ ID No: 7 à titre de CDR3 et comprenant une chaîne L ayant la séquence d'acides aminés de la SEQ ID No: 10 à titre de CDR1, la séquence d'acides aminés de la SEQ ID No: 11 à titre de CDR2, et la séquence d'acides aminés de la SEQ ID No: 12 à titre de CDR3 ;

b) anticorps comprenant une chaîne H ayant la séquence d'acides aminés de la SEQ ID No: 15 à titre de CDR1, la séquence d'acides aminés de la SEQ ID No: 16 à titre de CDR2, et la séquence d'acides aminés de la SEQ ID No: 17 à titre de CDR3 et comprenant une chaîne L ayant la séquence d'acides aminés de la SEQ ID No: 20 à titre de CDR1, la séquence d'acides aminés de la SEQ ID No: 21 à titre de CDR2, et la séquence d'acides aminés de la SEQ ID No: 22 à titre de CDR3 ;

(c) anticorps comprenant une chaîne H ayant la séquence d'acides aminés de la SEQ ID No: 45 à titre de CDR1, la séquence d'acides aminés de la SEQ ID No: 46 à titre de CDR2, et la séquence d'acides aminés de la SEQ ID No: 47 à titre de CDR3 et comprenant une chaîne L ayant la séquence d'acides aminés de la SEQ ID No: 50 à titre de CDR1, la séquence d'acides aminés de la SEQ ID No: 51 à titre de CDR2, et la séquence d'acides aminés de la SEQ ID No: 52 à titre de CDR3 ;

(d) anticorps comprenant une chaîne H ayant la séquence d'acides aminés de la SEQ ID No: 55 à titre de CDR1, la séquence d'acides aminés de la SEQ ID No: 56 à titre de CDR2, et la séquence d'acides aminés de la SEQ ID No: 57 à titre de CDR3 et comprenant une chaîne L ayant la séquence d'acides aminés de la SEQ ID No: 60 à titre de CDR1, la séquence d'acides aminés de la SEQ ID No: 61 à titre de CDR2, et la séquence d'acides aminés de la SEQ ID No: 62 à titre de CDR3 ;

(e) anticorps comprenant une chaîne H ayant la séquence d'acides aminés de la SEQ ID No: 65 à titre de CDR1, la séquence d'acides aminés de la SEQ ID No: 66 à titre de CDR2, et la séquence d'acides aminés de la SEQ ID No: 67 à titre de CDR3 et comprenant une chaîne L ayant la séquence d'acides aminés de la SEQ ID No: 70 à titre de CDR1, la séquence d'acides aminés de la SEQ ID No: 71 à titre de CDR2, et la séquence d'acides aminés de la SEQ ID No: 72 à titre de CDR3 ;

(f) anticorps comprenant une chaîne H ayant la séquence d'acides aminés de la SEQ ID No: 75 à titre de CDR1, la séquence d'acides aminés de la SEQ ID No: 76 à titre de CDR2, et la séquence d'acides aminés de la SEQ ID No: 77 à titre de CDR3 et comprenant une chaîne L ayant la séquence d'acides aminés de la SEQ ID No: 80 à titre de CDR1, la séquence d'acides aminés de la SEQ ID No: 81 à titre de CDR2, et la séquence d'acides aminés de la SEQ ID No: 82 à titre de CDR3 ;

(g) anticorps comprenant une chaîne H ayant la séquence d'acides aminés de la SEQ ID No: 85 à titre de CDR1, la séquence d'acides aminés de SEQ ID No: 86 à titre de CDR2, et la séquence d'acides aminés de la SEQ ID No: 87 à titre de CDR3 et comprenant une chaîne L ayant la séquence d'acides aminés de la SEQ ID No: 90 à titre de CDR1, la séquence d'acides aminés de SEQ ID No: 91 à titre de CDR2, et la séquence d'acides aminés de la SEQ ID No: 92 à titre de CDR3.

**3.** Anticorps selon l'une quelconque des revendications 1 à 2, qui est un anticorps qui a une activité cytotoxique ; en particulier, dans lequel l'activité cytotoxique est une activité cytotoxique à médiation cellulaire dépendante de l'anticorps ou dans lequel l'activité cytotoxique est une activité cytotoxique dépendante du complément.

**4.** Anticorps selon l'une quelconque des revendications 1 à 3, qui est un anticorps auquel une substance cytotoxique est liée.

**5.** Anticorps selon l'une quelconque des revendications 1 à 4, qui est un anticorps qui supprime la croissance des cellules cancéreuses, en particulier, où les cellules cancéreuses sont des cellules cancéreuses ovariennes à cellules claires.

**6.** Anticorps selon l'une quelconque des revendications 1 à 5, où l'anticorps a une région constante humaine, en particulier, où l'anticorps est un anticorps chimérique, un anticorps humanisé, ou un anticorps humain.

**7.** Anticorps selon l'une quelconque des revendications 1 à 6, où l'anticorps est un anticorps ayant un déficit en fucose.

**8.** Anticorps selon l'une quelconque des revendications 1 à 7, pour une utilisation dans le traitement du cancer.

**9.** Anticorps pour une utilisation selon la revendication 8, où le cancer est le cancer de l'ovaire ; en particulier où le cancer de l'ovaire est un adénocarcinome à cellules claires.

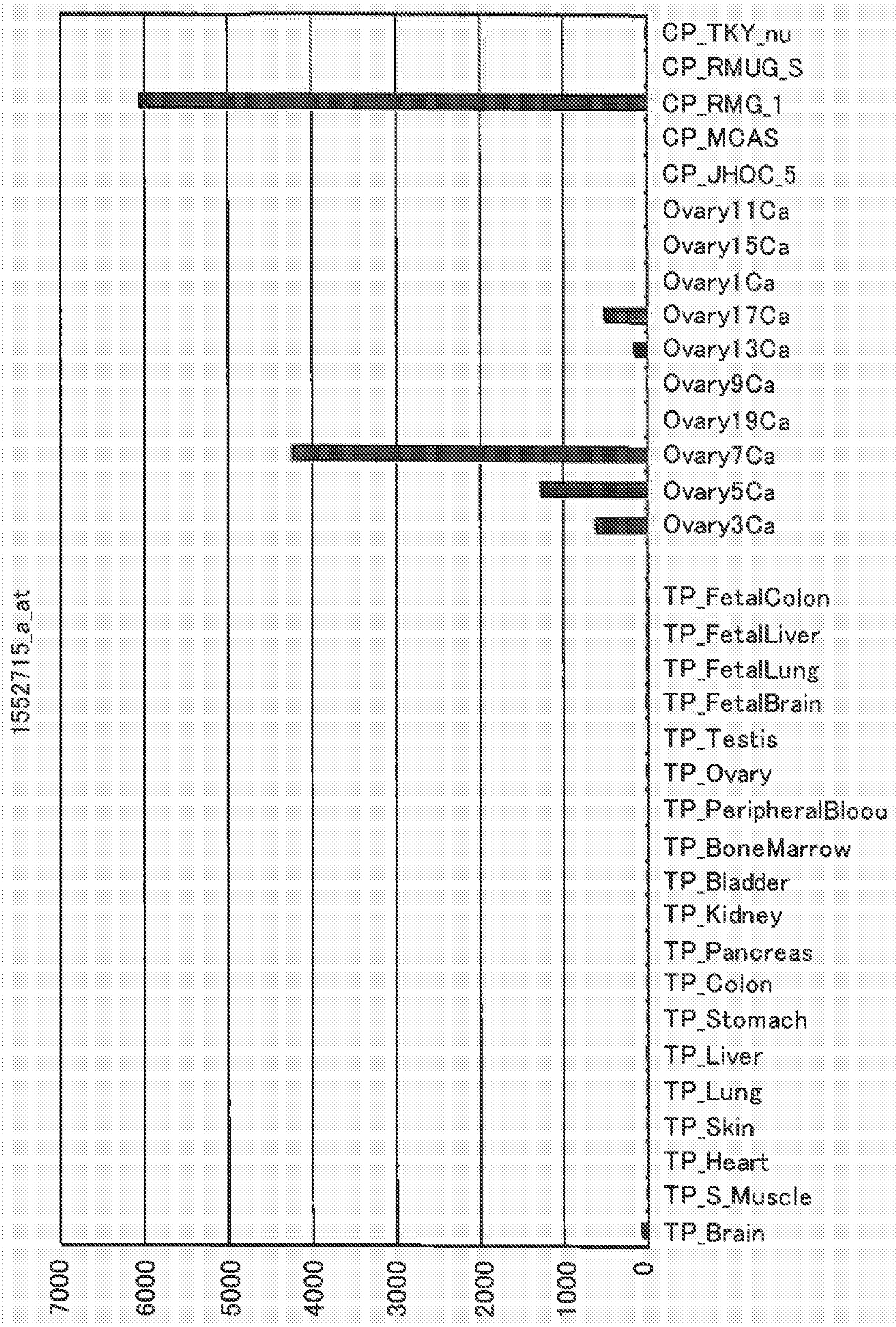
1552715_a_at
CP_TKY_nu
CP_RMUG_S
CP_RMG_1
CP_MCAS
CP_JHOC_5
Ovary11Ca
Ovary15Ca
Ovary1Ca
Ovary17Ca
Ovary13Ca
Ovary9Ca
Ovary19Ca
Ovary7Ca
Ovary5Ca
Ovary3Ca
TP_FetalColon
TP_FetalLiver
TP_FetalLung
TP_FetalBrain
TP_Testis
TP_Ovary
TP_PeripheralBlood
TP_BoneMarrow
TP_Bladder
TP_Kidney
TP_Pancreas
TP_Colon
TP_Stomach
TP_Liver
TP_Lung
TP_Skin
TP_Heart
TP_S_Muscle
TP_Brain
7000
6000
5000
4000
3000
2000
1000
0

FIG. 1-1

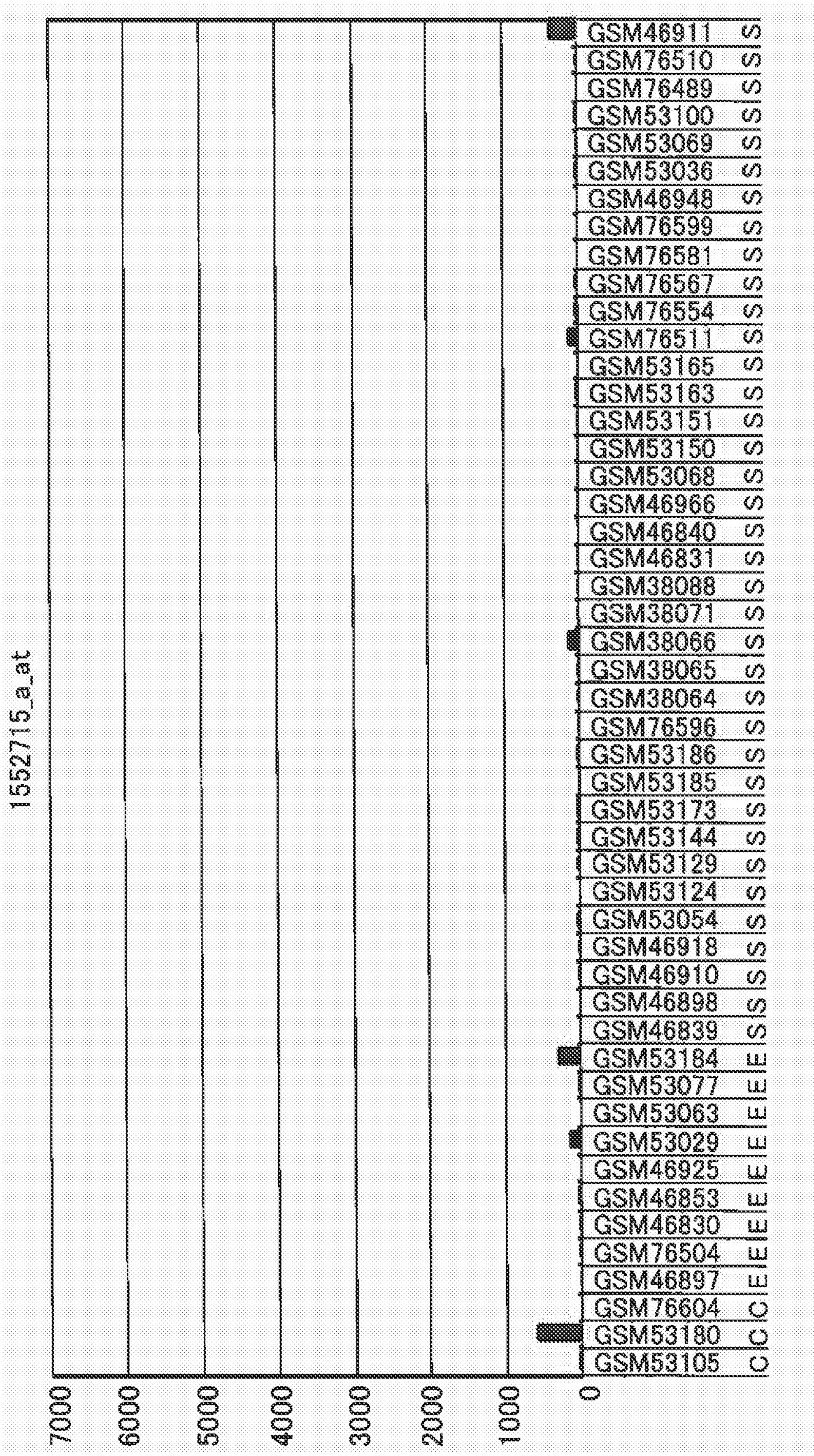
1552715_a_at
7000
6000
5000
4000
3000
2000
1000
0
GSM46911 S
GSM76510 S
GSM76489 S
GSM53100 S
GSM53069 S
GSM53036 S
GSM46948 S
GSM76599 S
GSM76581 S
GSM76567 S
GSM76554 S
GSM76511 S
GSM53165 S
GSM53163 S
GSM53151 S
GSM53150 S
GSM53068 S
GSM46966 S
GSM46840 S
GSM46831 S
GSM38088 S
GSM38071 S
GSM38066 S
GSM38065 S
GSM38064 S
GSM76596 S
GSM53186 S
GSM53185 S
GSM53173 S
GSM53144 S
GSM53129 S
GSM53124 S
GSM53054 S
GSM46918 S
GSM46910 S
GSM46898 S
GSM46839 S
GSM53184 E
GSM53077 E
GSM53063 E
GSM53029 E
GSM46925 E
GSM46853 E
GSM46830 E
GSM76504 E
GSM46897 E
GSM76604 C
GSM53180 C
GSM53105 C

FIG. 1-2

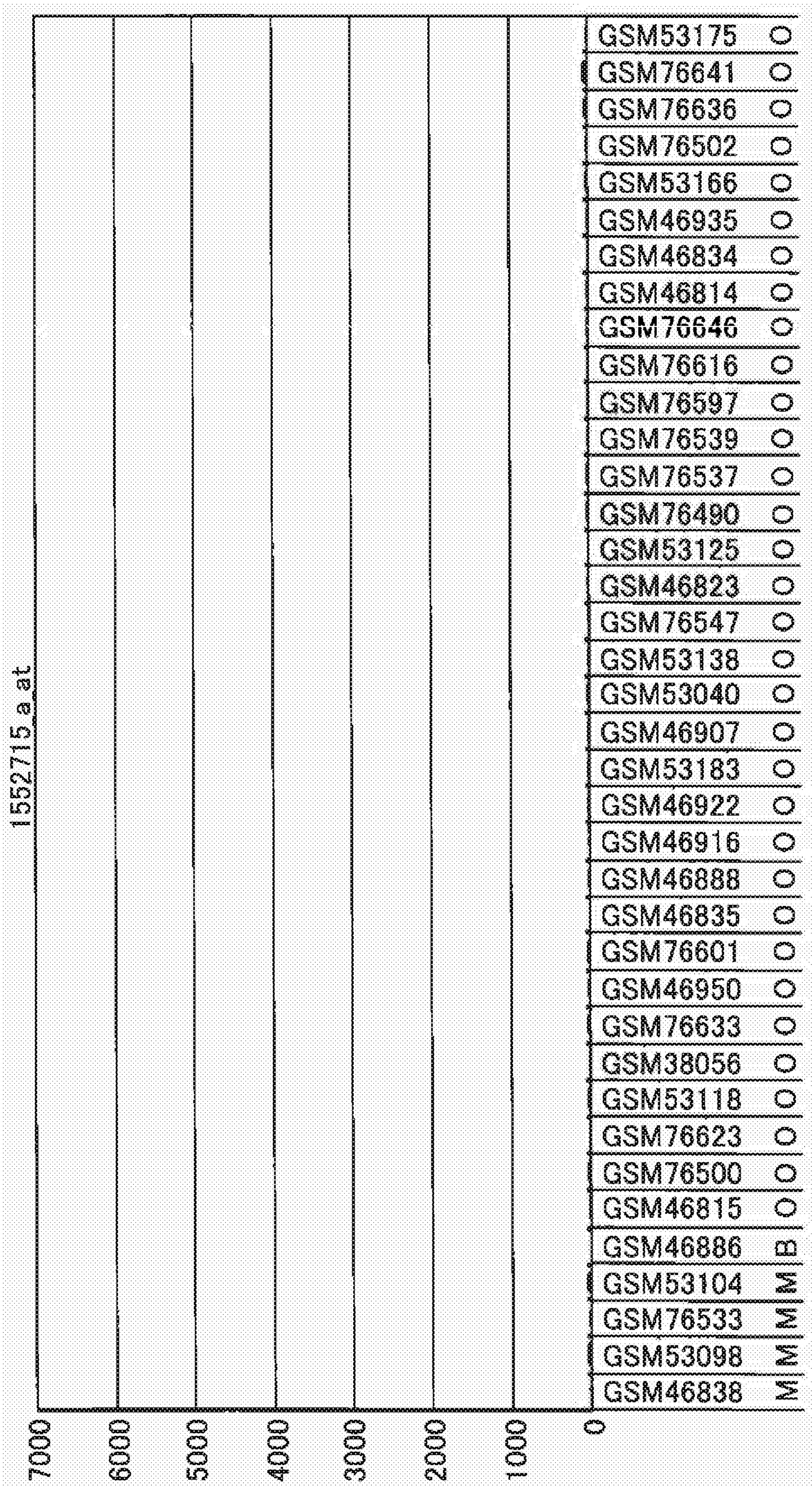
1552715_a_at
7000
6000
5000
4000
3000
2000
1000
0
GSM53175 O
GSM76641 O
GSM76636 O
GSM76502 O
GSM53166 O
GSM46935 O
GSM46834 O
GSM46814 O
GSM76646 O
GSM76616 O
GSM76597 O
GSM76539 O
GSM76537 O
GSM76490 O
GSM53125 O
GSM46823 O
GSM76547 O
GSM53138 O
GSM53040 O
GSM46907 O
GSM53183 O
GSM46922 O
GSM46916 O
GSM46888 O
GSM46835 O
GSM76601 O
GSM46950 O
GSM76633 O
GSM38056 O
GSM53118 O
GSM76623 O
GSM76500 O
GSM46815 O
GSM46886 B
GSM53104 M
GSM76533 M
GSM53098 M
GSM46838 M

FIG. 1-3

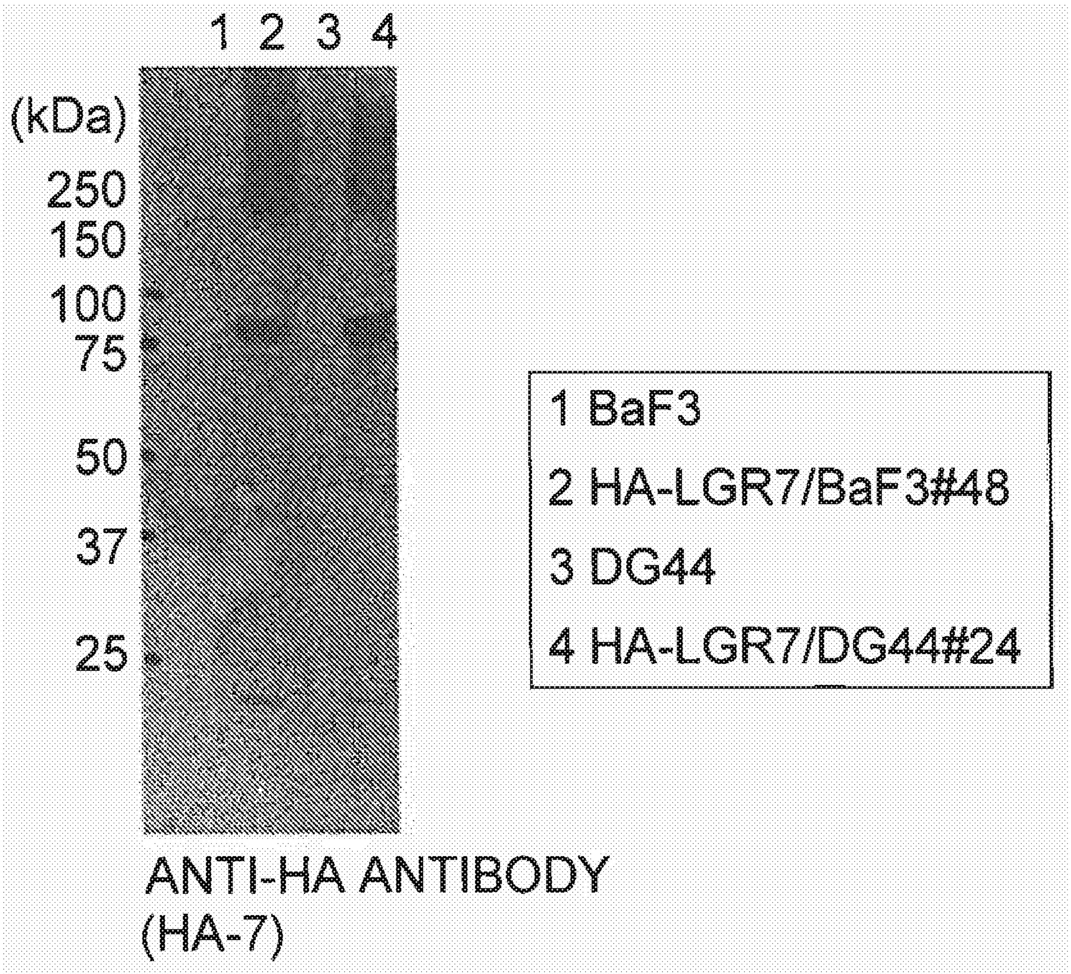
1 2 3 4
(kDa)
250
150
100
75
50
37
25
1 BaF3
2 HA-LGR7/BaF3#48
3 DG44
4 HA-LGR7/DG44#24
ANTI-HA ANTIBODY
(HA-7)

FIG. 2

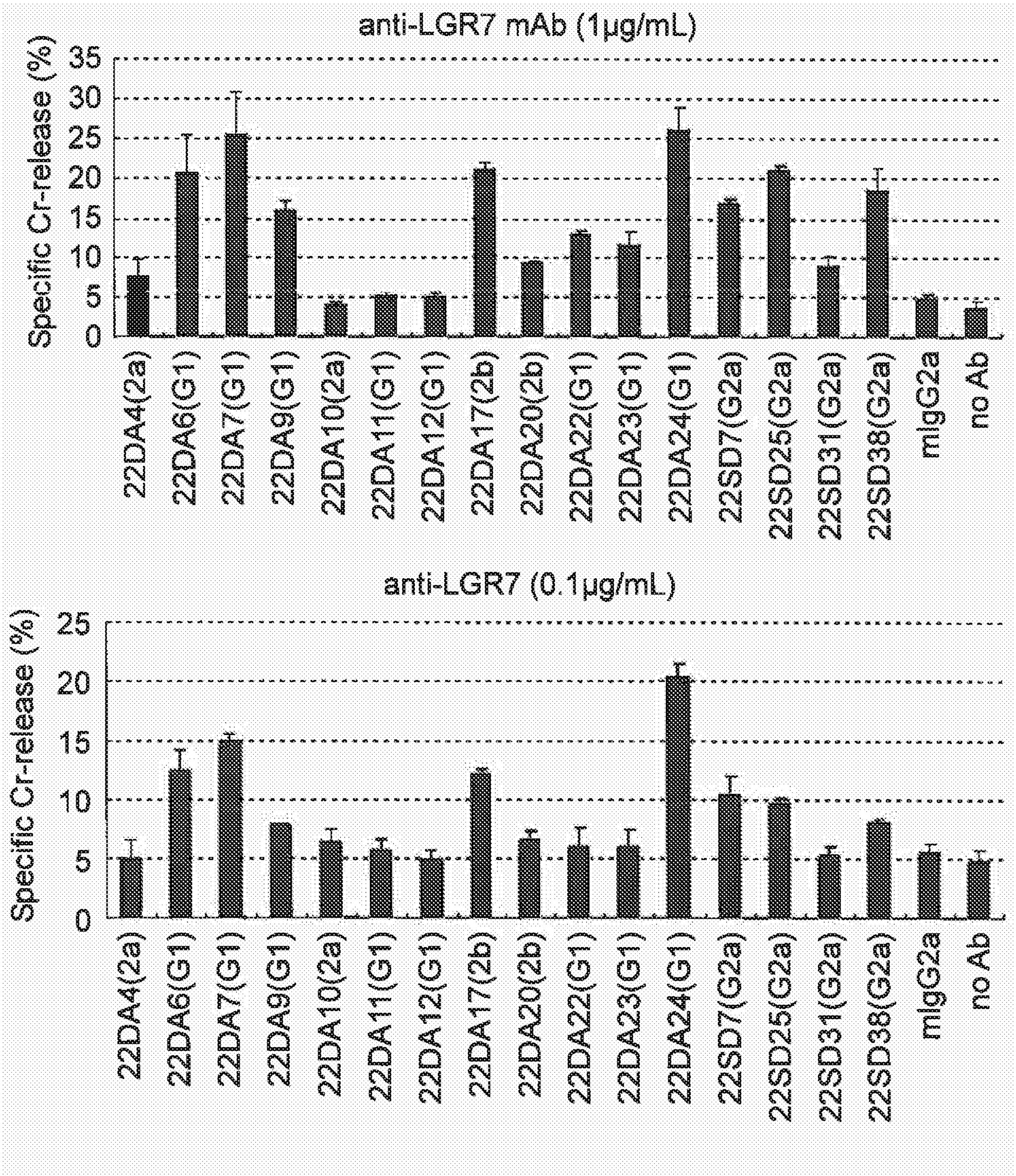
anti-LGR7 mAb (1μg/mL)
Specific Cr-release (%)
35
30
25
20
15
10
5
0
22DA4(2a)
22DA6(G1)
22DA7(G1)
22DA9(G1)
22DA10(2a)
22DA11(G1)
22DA12(G1)
22DA17(2b)
22DA20(2b)
22DA22(G1)
22DA23(G1)
22DA24(G1)
22SD7(G2a)
22SD25(G2a)
22SD31(G2a)
22SD38(G2a)
mlgG2a
no Ab
anti-LGR7 (0.1μg/mL)
Specific Cr-release (%)
25
20
15
10
5
0
22DA4(2a)
22DA6(G1)
22DA7(G1)
22DA9(G1)
22DA10(2a)
22DA11(G1)
22DA12(G1)
22DA17(2b)
22DA20(2b)
22DA22(G1)
22DA23(G1)
22DA24(G1)
22SD7(G2a)
22SD25(G2a)
22SD31(G2a)
22SD38(G2a)
mlgG2a
no Ab

FIG. 3

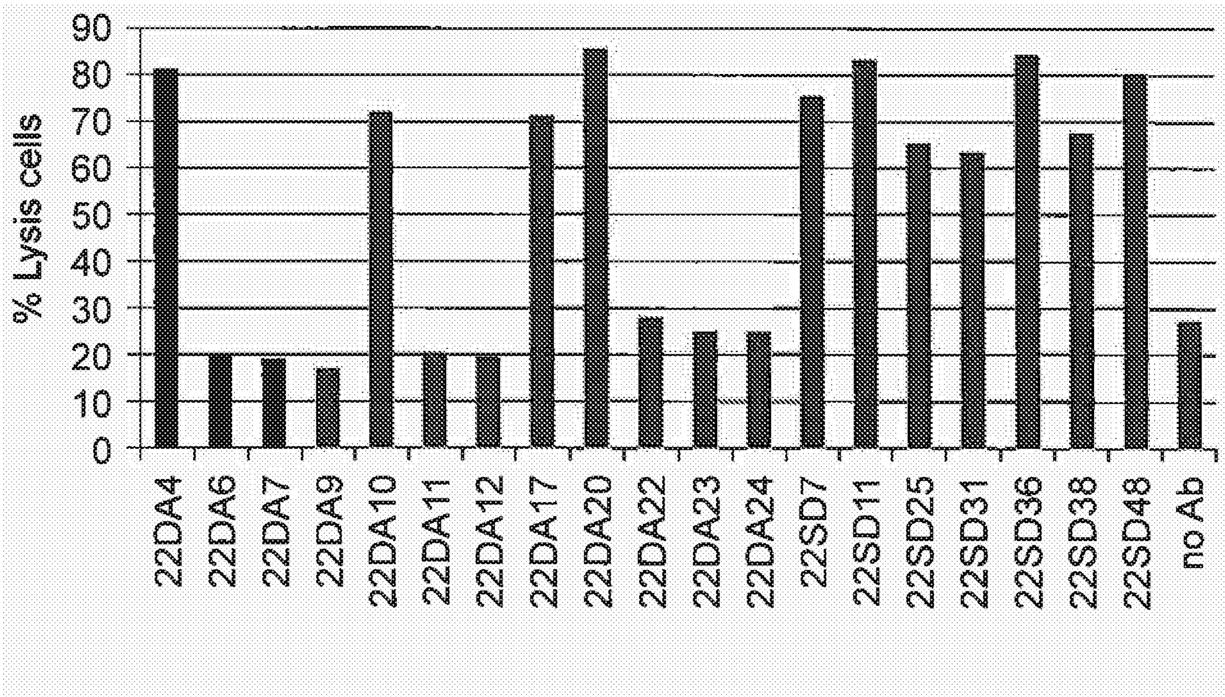
% Lysis cells
0
10
20
30
40
50
60
70
80
90
22DA4
22DA6
22DA7
22DA9
22DA10
22DA11
22DA12
22DA17
22DA20
22DA22
22DA23
22DA24
22SD7
22SD11
22SD25
22SD31
22SD36
22SD38
22SD48
no Ab

FIG. 4

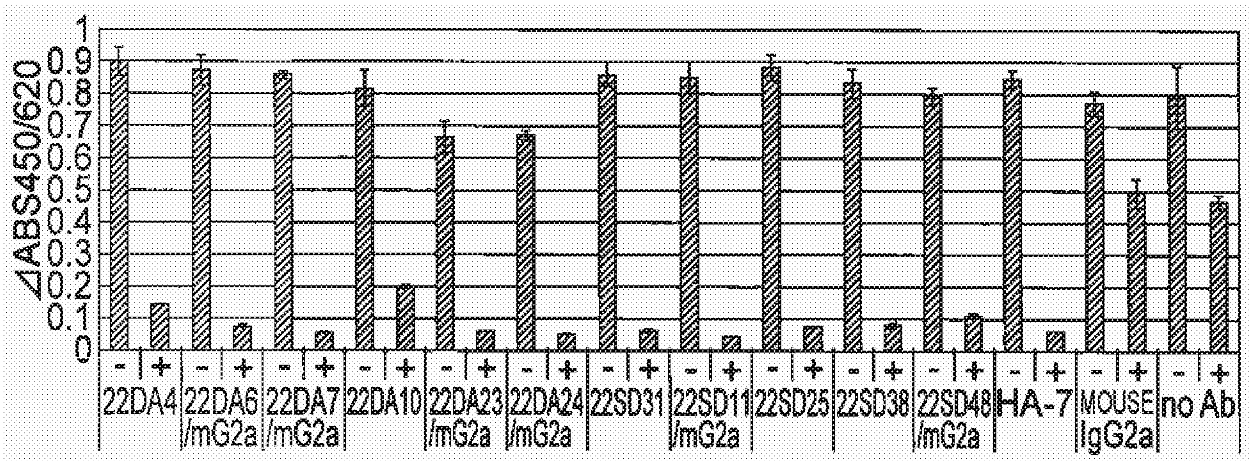
ΔABS450/620
1
0.9
0.8
0.7
0.6
0.5
0.4
0.3
0.2
0.1
0
- + - + - + - + - + - + - + - + - + - + - + - + - + -
22DA4
22DA6 /mG2a
22DA7 /mG2a
22DA10
22DA23 /mG2a
22DA24 /mG2a
22SD31
22SD11 /mG2a
22SD25
22SD38
22SD48 /mG2a
HA-7
MOUSE IgG2a
no Ab

FIG. 5

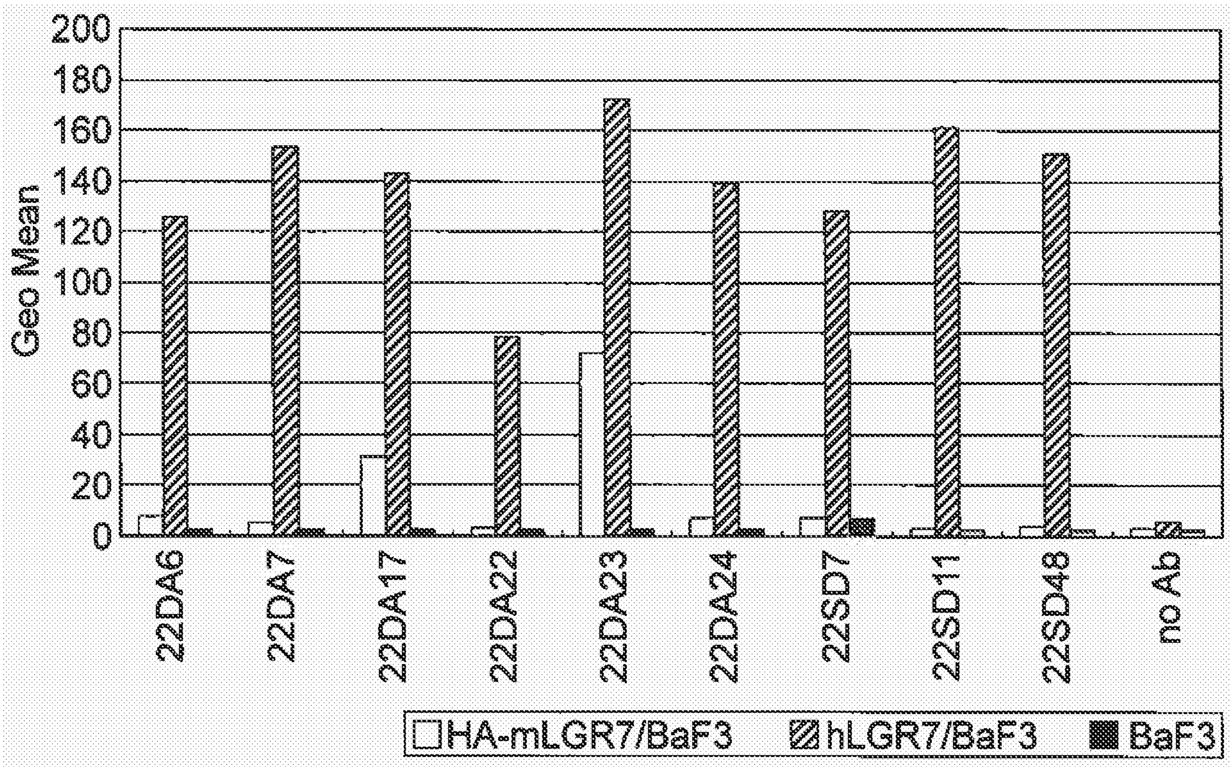
200
180
160
140
120
100
80
60
40
20
0
Geo Mean
22DA6
22DA7
22DA17
22DA22
22DA23
22DA24
22SD7
22SD11
22SD48
no Ab
HA-mLGR7/BaF3
hLGR7/BaF3
BaF3

FIG. 6

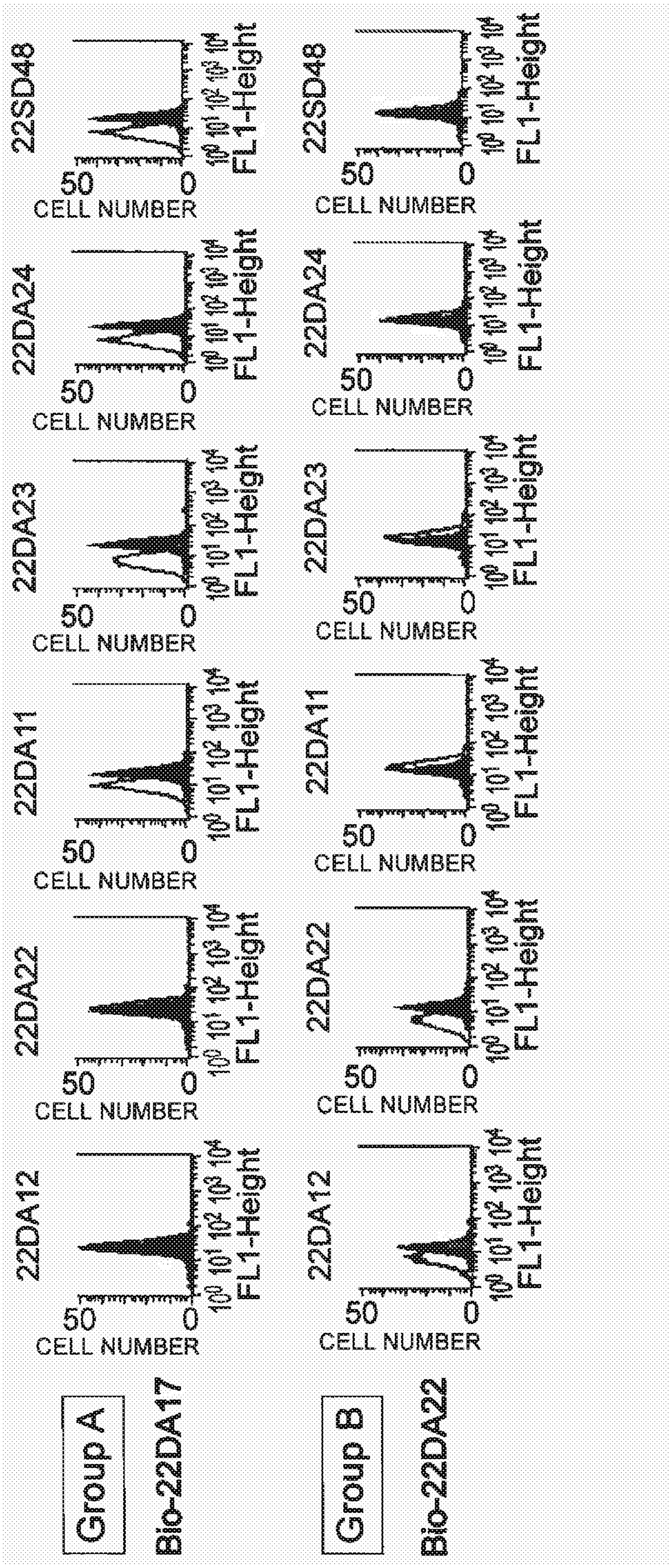
Group A
Bio-22DA17
22DA12
22DA22
22DA11
22DA23
22DA24
22SD48
Group B
Bio-22DA22
CELL NUMBER
50
0
FL1-Height

FIG. 7

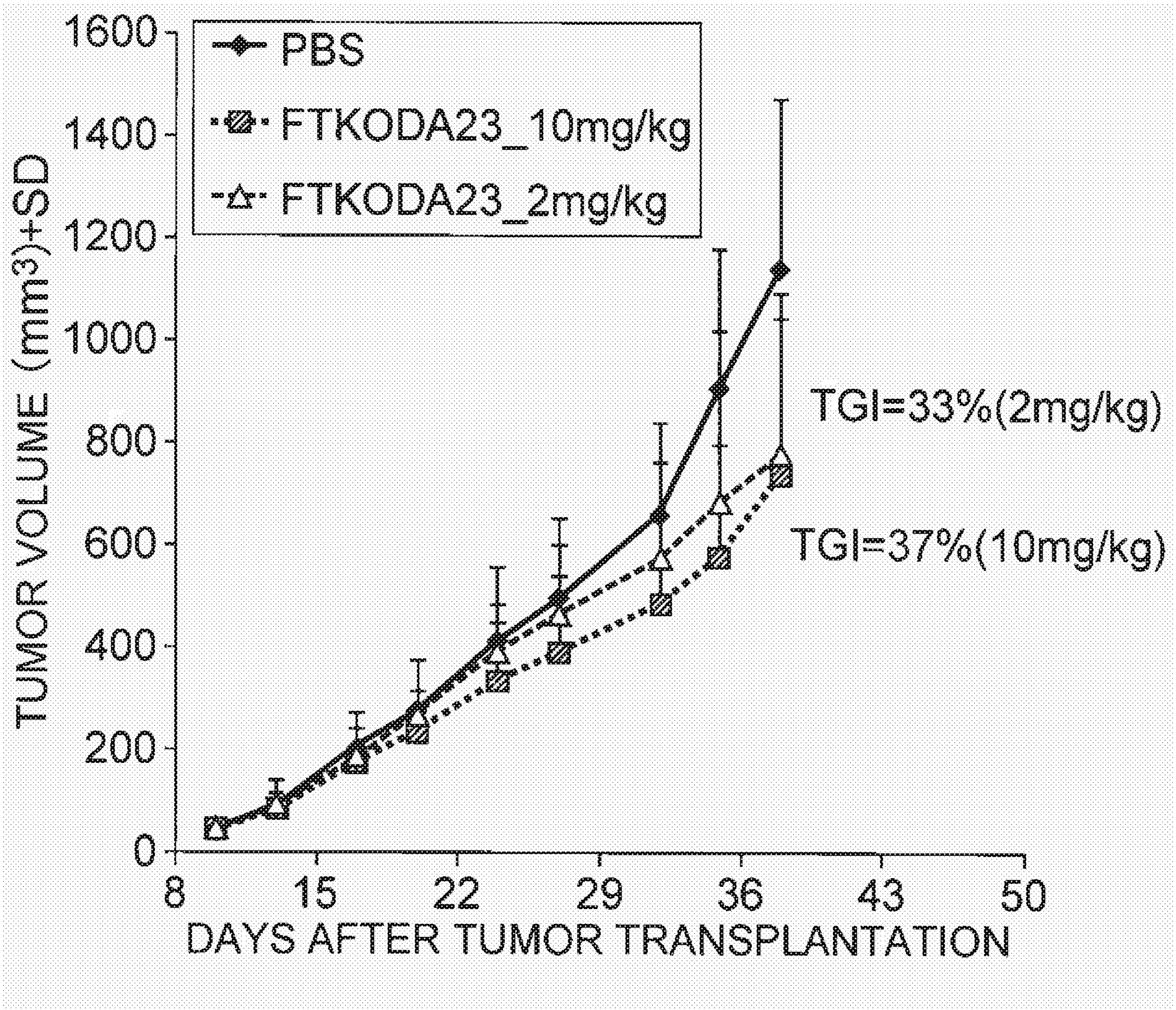
PBS
FTKODA23_10mg/kg
FTKODA23_2mg/kg
TUMOR VOLUME (mm3)+SD
1600
1400
1200
1000
800
600
400
200
0
8
15
22
29
36
43
50
DAYS AFTER TUMOR TRANSPLANTATION
TGI=33%(2mg/kg)
TGI=37%(10mg/kg)

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9948921 A
- US 2005107595 A
- WO 2003016487 A
- WO 2003093827 A
- WO 2005107396 A
- WO 0228418 A
- WO 03016487 A
- WO 03093827 A
- WO 03104453 A
- JP H0159878 B
- WO 9425585 A
- WO 9312227 A
- WO 9203918 A
- WO 9402602 A
- WO 9411523 A
- EP 239400 A
- WO 9602576 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- EP 404097 A
- WO 9311161 A
- WO 9954342 A
- WO 0061739 A
- WO 0231140 A
- WO 2006067913 A
- WO 0279255 A
- WO 2008093688 A
- JP 2008333149 A


### Non-patent literature cited in the description


- **NIHON SANFUJINKA.** *Gakkai-shi,* 2005, vol. 57 (11), 1711
- **HSU, S. Y. et al.** *Molec. Endocr.,* 2000, vol. 14, 1257-1271
- **HSUEH A. J. W. et al.** *Journal of Endocrinology,* 2005, vol. 187, 333-338
- **BATHGATE RA. et al.** *Pharmacol Rev,* 2006, vol. 58, 7-31
- **KERN A. et al.** *Endocrinology,* 2007, vol. 148, 1181-1194
- **KRISTIANSEN K.** *Pharmacology & Therapeutics,* 2004, vol. 103, 21-80
- **HALLS M. L. et al.** *British Journal of Pharmacology,* 2007, vol. 150, 677-691
- **VAN DER WESTHUIZEN, E. T. et al.** *Current Drug Targets,* 2007, vol. 8, 91-104
- **HOMBACH-KLONISCH S. et al.** *American Journal of Pathology,* 2006, vol. 169, 617-632
- **VINALL R. L. et al.** *Oncogene,* 2006, vol. 25, 2082-2093
- **SUGIYAMA T. et al.** *Cancer,* 2000, vol. 88, 2584
- **ENOMOTO T. et al.** *Proceedings of ASCO,* 2003, 1797
- **IVELL R. et al.** *Reprod. Biol. Endocrinol.,* 2003, vol. 114, 1, , 13-13, 13
- **KAMAT A. A. et al.** *Cancer Biology & Therapy,* 2006, vol. 5, 71-77
- **SAMBROOK J et al.** Molecular Cloning. Cold Spring Harbor Lab. Press, 1989, 9.47-9.58
- *J. Immunol.,* 1979, vol. 123, 1548-1550
- *Current Topics in Microbiology and Immunology,* 1978, vol. 81, 1-7
- **KOHLER. G. ; MILSTEIN, C.** *Eur. J. Immunol.,* 1976, vol. 6, 511-519
- **MARGULIES. D.H. et al.** *Cell,* 1976, vol. 8, 405-415
- **SHULMAN, M. et al.** *Nature,* 1978, vol. 276, 269-270
- **DE ST. GROTH, S. F. et al.** *J. Immunol. Methods,* 1980, vol. 35, 1-21
- **TROWBRIDGE, I. S.** *J. Exp. Med.,* 1978, vol. 148, 313-323
- **GALFRE, G. et al.** *Nature,* 1979, vol. 277, 131-133
- **KOHLER. G. ; MILSTEIN, C.** *Methods Enzymol.,* 1981, vol. 73, 3-46
- **VANDAMME, A. M. et al.** *Eur. J. Biochem.,* 1990, vol. 192, 767-775
- **CHIRGWIN, J. M. et al.** *Biochemistry,* 1979, vol. 18, 5294-5299
- **CHOMCZYNSKI, P. et al.** *Anal. Biochem.,* 1987, vol. 162, 156-159

- **FROHMAN, M. A. et al.** *Proc. Natl. Acad. Sci. USA,* 1988, vol. 85, 8998-9002
- **BELYAVSKY, A. et al.** *Nucleic Acids Res.,* 1989, vol. 17, 2919-2932
- *J. Mol. Biol.,* 1991, vol. 222, 581-597
- **MULLIGAN et al.** *Nature,* 1979, vol. 277, 108
- **MIZUSHIMA et al.** *Nucleic Acids Res.,* 1990, vol. 18, 5322
- **WARD et al.** *Nature,* 1989, vol. 341, 544-546
- *FASEB J.,* 1992, vol. 6, 2422-2427
- **BETTER et al.** *Science,* 1988, vol. 240, 1041-1043
- **LEI, S. P. et al.** *J. Bacteriol.,* 1987, vol. 169, 4379
- Antibodies: A Laboratory Manual. Cold Spring Harbor Laboratory, 1988
- **EBERT, K. M. et al.** *Bio/Technology,* 1994, vol. 12, 699-702
- **SATO, K. et al.** *Cancer Res.,* 1993, vol. 53, 851-856
- **CO, M. S. et al.** *J. Immunol.,* 1994, vol. 152, 2968-2976
- **BETTER, M. ; HORWITZ, A. H.** *Methods in Enzymology,* 1989, vol. 178, 476-496
- **PLUECKTHUN, A. ; SKERRA, A.** *Methods in Enzymology,* 1989, vol. 178, 476-496
- **LAMOYI, E.** *Methods in Enzymology,* 1989, vol. 121, 652-663
- **ROUSSEAUX, J. et al.** *Methods in Enzymology,* 1989, vol. 121, 663-669
- **BIRD, R. E. et al.** *TIBTECH,* 1991, vol. 9, 132-137
- **HOLLINGER P. et al.** *Proc. Natl. Acad. Sci. USA,* 1993, vol. 90, 6444-6448
- **HUSTON, J. S. et al.** *Proc. Natl. Acad. Sci. U.S.A.,* 1988, vol. 85, 5879-5883
- *Cellular & Molecular Immunology,* 2006, vol. 3, 439-443
- **HUDSON et al.** *J. Immunol. Methods,* 1999, vol. 231, 177-189
- *Protein Engineering,* 1996, vol. 9 (3), 299-305
- **BETTER, M. ; HORWITZ, A. H.** *Methods Enzymol.,* 1989, vol. 178, 476-496
- **PLUCKTHUN, A. ; SKERRA, A.** *Methods Enzymol.,* 1989, vol. 178, 497-515
- **LAMOYI, E.** *Methods Enzymol.,* 1986, vol. 121, 652-663
- **ROUSSEAUX, J. et al.** *Methods Enzymol.,* 1986, vol. 121, 663-669
- **BIRD, R. E. ; WALKER, B. W.** *Trends Biotechnol.,* 1991, vol. 9, 132-137
- **LANGONE J. J. et al.** *Methods in Enzymology,* 1983, vol. 93, 307-308
- *Nature Medicine,* 1996, vol. 2, 350-353
- **FULTON R. J. et al.** *J. Biol. Chem.,* 1986, vol. 261, 5314-5319
- **SIVAM G. et al.** *Cancer Res.,* 1987, vol. 47, 3169-3173
- **CUMBER A. J. et al.** *J. Immunol. Methods,* 1990, vol. 135, 15-24
- **WAWRZYNCZAK E. J. et al.** *Cancer Res.,* 1990, vol. 50, 7519-7562
- **GHEEITE V. et al.** *J. Immunol. Methods,* 1991, vol. 142, 223-230
- **THORPE P. E. et al.** *Cancer Res.,* 1987, vol. 47, 5924-5931
- **WAWRZYNCZAK E. J. et al.** *Br. J. Cancer,* 1992, vol. 66, 361-366
- **BOLOGNESI A. et al.** *Clin. exp. Immunol.,* 1992, vol. 89, 341-346
- **STIRPE F. ; BARBIERI L.** *FEBS letter,* 1986, vol. 195, 1-8
- **STIRPE F. ; BARBIERI L.** *FEBS letter,* 1986, vol. 195 (1-8
- **CASELLAS P. et al.** *Eur. J. Biochem.,* 1988, vol. 176, 581-588
- Antibodies A Laboratory Manual. Cold Spring Harbor Laboratory, 1988
- Immunologic studies in humans. Current Protocols in Immunology. John Wiley & Sons, Inc, 1993
- **HASHIMOTO-GOTOH, T. et al.** *Gene,* 1995, vol. 152, 271-275
- **ZOLLER, MJ ; SMITH, M.** *Methods Enzymol.,* 1983, vol. 100, 468-500
- **KRAMER, W. et al.** *Nucleic Acids Res.,* 1984, vol. 12, 9441-9456
- **KRAMER W ; FRITZ HJ.** *Methods. Enzymol.,* 1987, vol. 154, 350-367
- **KUNKEL, TA.** *Proc. Natl. Acad. Sci. USA.,* 1985, vol. 82, 488-492
- **KUNKEL.** *Methods Enzymol.,* 1988, vol. 85, 2763-2766
- **MARK, D. F. et al.** *Proc. Natl. Acad. Sci. USA,* 1984, vol. 81, 5662-5666
- **ZOLLER, M. J. ; SMITH, M.** *Nucleic Acids Research,* 1982, vol. 10, 6487-6500
- **WANG, A. et al.** *Science,* vol. 224, 1431-1433
- **DALBADIE-MCFARLAND, G. et al.** *Proc. Natl. Acad. Sci. USA,* 1982, vol. 79, 6409-6413
- Remington's Pharmaceutical Science. Mark Publishing Company
- Immunologic studies in humans. Current protocols in Immunology. John Wiley & Sons, Inc, 1993
- Acta Oncol. 1993, vol. 32, 825-830
- *Methods,* 2003, vol. 31, 232-242

SEQUENCE LISTING

<110> THE UNIVERSITY OF TOKYO FORERUNNER PHARMA RESEARCH CO., LTD.

<120> DIAGNOSIS AND TREATMENT OF CANCER USING ANTI-LGR7 ANTIBODY

<130> C1-A0808P

<150> JP 2008-333149
<151> 2008-12-26

<160> 108

<170> PatentIn version 3.4

<210> 1
<211> 757
<212> PRT
<213> Homo sapiens

<400> 1

```
Met Thr Ser Gly Ser Val Phe Phe Tyr Ile Leu Ile Phe Gly Lys Tyr
1               5                   10                  15

Phe Ser His Gly Gly Gly Gln Asp Val Lys Cys Ser Leu Gly Tyr Phe
            20                  25                  30

Pro Cys Gly Asn Ile Thr Lys Cys Leu Pro Gln Leu Leu His Cys Asn
        35                  40                  45

Gly Val Asp Asp Cys Gly Asn Gln Ala Asp Glu Asp Asn Cys Gly Asp
    50                  55                  60

Asn Asn Gly Trp Ser Leu Gln Phe Asp Lys Tyr Phe Ala Ser Tyr Tyr
65                  70                  75                  80

Lys Met Thr Ser Gln Tyr Pro Phe Glu Ala Glu Thr Pro Glu Cys Leu
                85                  90                  95

Val Gly Ser Val Pro Val Gln Cys Leu Cys Gln Gly Leu Glu Leu Asp
            100                 105                 110

Cys Asp Glu Thr Asn Leu Arg Ala Val Pro Ser Val Ser Ser Asn Val
        115                 120                 125

Thr Ala Met Ser Leu Gln Trp Asn Leu Ile Arg Lys Leu Pro Pro Asp
    130                 135                 140

Cys Phe Lys Asn Tyr His Asp Leu Gln Lys Leu Tyr Leu Gln Asn Asn
145                 150                 155                 160

Lys Ile Thr Ser Ile Ser Ile Tyr Ala Phe Arg Gly Leu Asn Ser Leu
                165                 170                 175
```

```
Thr Lys Leu Tyr Leu Ser His Asn Arg Ile Thr Phe Leu Lys Pro Gly
            180                 185                 190

Val Phe Glu Asp Leu His Arg Leu Glu Trp Leu Ile Ile Glu Asp Asn
        195                 200                 205

His Leu Ser Arg Ile Ser Pro Pro Thr Phe Tyr Gly Leu Asn Ser Leu
    210                 215                 220

Ile Leu Leu Val Leu Met Asn Asn Val Leu Thr Arg Leu Pro Asp Lys
225                 230                 235                 240

Pro Leu Cys Gln His Met Pro Arg Leu His Trp Leu Asp Leu Glu Gly
                245                 250                 255

Asn His Ile His Asn Leu Arg Asn Leu Thr Phe Ile Ser Cys Ser Asn
            260                 265                 270

Leu Thr Val Leu Val Met Arg Lys Asn Lys Ile Asn His Leu Asn Glu
        275                 280                 285

Asn Thr Phe Ala Pro Leu Gln Lys Leu Asp Glu Leu Asp Leu Gly Ser
    290                 295                 300

Asn Lys Ile Glu Asn Leu Pro Pro Leu Ile Phe Lys Asp Leu Lys Glu
305                 310                 315                 320

Leu Ser Gln Leu Asn Leu Ser Tyr Asn Pro Ile Gln Lys Ile Gln Ala
                325                 330                 335

Asn Gln Phe Asp Tyr Leu Val Lys Leu Lys Ser Leu Ser Leu Glu Gly
            340                 345                 350

Ile Glu Ile Ser Asn Ile Gln Gln Arg Met Phe Arg Pro Leu Met Asn
        355                 360                 365

Leu Ser His Ile Tyr Phe Lys Lys Phe Gln Tyr Cys Gly Tyr Ala Pro
    370                 375                 380

His Val Arg Ser Cys Lys Pro Asn Thr Asp Gly Ile Ser Ser Leu Glu
385                 390                 395                 400

Asn Leu Leu Ala Ser Ile Ile Gln Arg Val Phe Val Trp Val Val Ser
                405                 410                 415

Ala Val Thr Cys Phe Gly Asn Ile Phe Val Ile Cys Met Arg Pro Tyr
            420                 425                 430
```

```
Ile Arg Ser Glu Asn Lys Leu Tyr Ala Met Ser Ile Ile Ser Leu Cys
        435                 440                 445

Cys Ala Asp Cys Leu Met Gly Ile Tyr Leu Phe Val Ile Gly Gly Phe
    450                 455                 460

Asp Leu Lys Phe Arg Gly Glu Tyr Asn Lys His Ala Gln Leu Trp Met
465                 470                 475                 480

Glu Ser Thr His Cys Gln Leu Val Gly Ser Leu Ala Ile Leu Ser Thr
                485                 490                 495

Glu Val Ser Val Leu Leu Leu Thr Phe Leu Thr Leu Glu Lys Tyr Ile
            500                 505                 510

Cys Ile Val Tyr Pro Phe Arg Cys Val Arg Pro Gly Lys Cys Arg Thr
        515                 520                 525

Ile Thr Val Leu Ile Leu Ile Trp Ile Thr Gly Phe Ile Val Ala Phe
    530                 535                 540

Ile Pro Leu Ser Asn Lys Glu Phe Phe Lys Asn Tyr Tyr Gly Thr Asn
545                 550                 555                 560

Gly Val Cys Phe Pro Leu His Ser Glu Asp Thr Glu Ser Ile Gly Ala
                565                 570                 575

Gln Ile Tyr Ser Val Ala Ile Phe Leu Gly Ile Asn Leu Ala Ala Phe
            580                 585                 590

Ile Ile Ile Val Phe Ser Tyr Gly Ser Met Phe Tyr Ser Val His Gln
        595                 600                 605

Ser Ala Ile Thr Ala Thr Glu Ile Arg Asn Gln Val Lys Lys Glu Met
    610                 615                 620

Ile Leu Ala Lys Arg Phe Phe Phe Ile Val Phe Thr Asp Ala Leu Cys
625                 630                 635                 640

Trp Ile Pro Ile Phe Val Val Lys Phe Leu Ser Leu Leu Gln Val Glu
                645                 650                 655

Ile Pro Gly Thr Ile Thr Ser Trp Val Val Ile Phe Ile Leu Pro Ile
            660                 665                 670

Asn Ser Ala Leu Asn Pro Ile Leu Tyr Thr Leu Thr Thr Arg Pro Phe
        675                 680                 685
```

```
Lys Glu Met Ile His Arg Phe Trp Tyr Asn Tyr Arg Gln Arg Lys Ser
    690                 695                 700

Met Asp Ser Lys Gly Gln Lys Thr Tyr Ala Pro Ser Phe Ile Trp Val
705                 710                 715                 720

Glu Met Trp Pro Leu Gln Glu Met Pro Pro Glu Leu Met Lys Pro Asp
                725                 730                 735

Leu Phe Thr Tyr Pro Cys Glu Met Ser Leu Ile Ser Gln Ser Thr Arg
            740                 745                 750

Leu Asn Ser Tyr Ser
        755
```

<210> 2
<211> 2274
<212> DNA
<213> Homo sapiens

<400> 2

```
atgacatctg gttctgtctt cttctacatc ttaatttttg gaaaatattt ttctcatggg     60
ggtggacagg atgtcaagtg ctcccttggc tatttcccct gtgggaacat cacaaagtgc    120
ttgcctcagc tcctgcactg taacggtgtg gacgactgcg ggaatcaggc cgatgaggac    180
aactgtggag acaacaatgg atggtctctg caatttgaca aatattttgc cagttactac    240
aaaatgactt cccaatatcc ttttgaggca gaaacacctg aatgtttggt cggttctgtg    300
ccagtgcaat gtctttgcca aggtctggag cttgactgtg atgaaaccaa tttacgagct    360
gttccatcgg tttcttcaaa tgtgactgca atgtcacttc agtggaactt aataagaaag    420
cttcctcctg attgcttcaa gaattatcat gatcttcaga agctgtacct gcaaaacaat    480
aagattacat ccatctccat ctatgctttc agaggactga atagccttac taaactgtat    540
ctcagtcata acagaataac cttcctgaag ccgggtgttt ttgaagatct tcacagacta    600
gaatggctga taattgaaga taatcacctc agtcgaattt ccccaccaac attttatgga    660
ctaaattctc ttattctctt agtcctgatg aataacgtcc tcacccgttt acctgataaa    720
cctctctgtc aacacatgcc aagactacat tggctggacc ttgaaggcaa ccatatccat    780
aatttaagaa atttgacttt tatttcctgc agtaatttaa ctgttttagt gatgaggaaa    840
aacaaaatta atcacttaaa tgaaaatact tttgcacctc tccagaaact ggatgaattg    900
gatttaggaa gtaataagat tgaaaatctt ccaccgctta tattcaagga cctgaaggag    960
ctgtcacaat tgaatctttc ctataatcca atccagaaaa ttcaagcaaa ccaatttgat   1020
tatcttgtca aactcaagtc tctcagccta gaagggattg aaatttcaaa tatccaacaa   1080
aggatgttta gacctcttat gaatctctct cacatatatt ttaagaaatt ccagtactgt   1140
gggtatgcac cacatgttcg cagctgtaaa ccaaacactg atggaatttc atctctagag   1200
```

aatctcttgg caagcattat tcagagagta tttgtctggg ttgtatctgc agttacctgc 1260

tttggaaaca tttttgtcat ttgcatgcga ccttatatca ggtctgagaa caagctgtat 1320

gccatgtcaa tcatttctct ctgctgtgcc gactgcttaa tgggaatata tttattcgtg 1380

atcggaggct ttgacctaaa gtttcgtgga gaatacaata agcatgcgca gctgtggatg 1440

gagagtactc attgtcagct tgtaggatct ttggccattc tgtccacaga agtatcagtt 1500

ttactgttaa catttctgac attggaaaaa tacatctgca ttgtctatcc ttttagatgt 1560

gtgagacctg gaaaatgcag aacaattaca gttctgattc tcatttggat tactggtttt 1620

atagtggctt tcattccatt gagcaataag gaatttttca aaaactacta tggcaccaat 1680

ggagtatgct tccctcttca ttcagaagat acagaaagta ttggagccca gatttattca 1740

gtggcaattt ttcttggtat taatttggcc gcatttatca tcatagtttt ttcctatgga 1800

agcatgtttt atagtgttca tcaaagtgcc ataacagcaa ctgaaatacg gaatcaagtt 1860

aaaaaagaga tgatccttgc caaacgtttt ttctttatag tatttactga tgcattatgc 1920

tggataccca tttttgtagt gaaatttctt tcactgcttc aggtagaaat accaggtacc 1980

ataacctctt gggtagtgat tttattctg cccattaaca gtgctttgaa cccaattctc 2040

tatactctga ccacaagacc atttaaagaa atgattcatc ggttttggta taactacaga 2100

caaagaaaat ctatggacag caaaggtcag aaaacatatg ctccatcatt catctgggtg 2160

gaaatgtggc cactgcagga gatgccacct gagttaatga agccggacct tttcacatac 2220

ccctgtgaaa tgtcactgat ttctcaatca acgagactca attcctattc atga 2274

<210> 3
<211> 366
<212> DNA
<213> Artificial

<220>
<223> An artificially synthesized nucleotide sequence

<400> 3

caggtccaac tgcagcagcc tggggctgag ctggtgaggc ctggagcttc agtgaaactg 60

tcctgcaagg cttctggcta ctccttcaac agctactgga tgaactgggt gaaacagagg 120

cctggacaag gccttgagtg gattggcatg tttcatcctt ccgatagtga aagtagatta 180

aatcagaagt tcaaggacaa ggccacattg actgcagaca aatcctccag cacagcctac 240

atgcaactca gcagcccgac atctgaagac tctgcggtct attactgtgc aagattggat 300

gatgataact accccttaca aagtgtggac tactggggtc aaggaacctc agtcaccgtc 360

tcctca 366

<210> 4
<211> 122
<212> PRT
<213> Artificial

<220>
<223> An artificially synthesized peptide sequence

<400> 4

```
Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Ser Phe Asn Ser Tyr
            20                  25                  30

Trp Met Asn Trp Val Lys Gln Arg Pro Gly Gln Gly Leu Glu Trp Ile
        35                  40                  45

Gly Met Phe His Pro Ser Asp Ser Glu Ser Arg Leu Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Ala Arg Leu Asp Asp Asp Asn Tyr Pro Leu Gln Ser Val Asp Tyr Trp
            100                 105                 110

Gly Gln Gly Thr Ser Val Thr Val Ser Ser
        115                 120
```

<210> 5
<211> 5
<212> PRT
<213> Artificial

<220>
<223> An artificially synthesized peptide sequence

<400> 5

```
Ser Tyr Trp Met Asn
1               5
```

<210> 6
<211> 17
<212> PRT
<213> Artificial

<220>
<223> An artificially synthesized peptide sequence

<400> 6

Met Phe His Pro Ser Asp Ser Glu Ser Arg Leu Asn Gln Lys Phe Lys
1               5                   10                  15

Asp

<210> 7
<211> 13
<212> PRT
<213> Artificial

<220>
<223> An artificially synthesized peptide sequence

<400> 7

Leu Asp Asp Asp Asn Tyr Pro Leu Gln Ser Val Asp Tyr
1               5                   10

<210> 8
<211> 321
<212> DNA
<213> Artificial

<220>
<223> An artificially synthesized nucleotide sequence

<400> 8

gatatccaga tgacacagac tacatcctcc ctgtctgcct ctctgggaga cagagtcacc 60

atcagttgca gggcaagtca ggacattagc aatcatttaa actggtatca gcagaaacca 120

gatggaactg ttaaactcct gatctactac acatcaagat tacactcagg agtcccatca 180

aggttcagtg gcagtgggtc tggaacagat tattcgctca ccattagcaa cctggagcaa 240

gaagatgttg ccacttactt ttgccaacag ggtaatacgc ttccgctcac gttcggtgct 300

gggaccaagc tggagctgaa a 321

<210> 9
<211> 107
<212> PRT
<213> Artificial

<220>
<223> An artificially synthesized peptide sequence

<400> 9

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Ser Asn His
20 25 30

Leu Asn Trp Tyr Gln Gln Lys Pro Asp Gly Thr Val Lys Leu Leu Ile
35 40 45

Tyr Tyr Thr Ser Arg Leu His Ser Gly Val Pro Ser Arg Phe Ser Gly

50 55 60

Ser Gly Ser Gly Thr Asp Tyr Ser Leu Thr Ile Ser Asn Leu Glu Gln
65 70 75 80

Glu Asp Val Ala Thr Tyr Phe Cys Gln Gln Gly Asn Thr Leu Pro Leu
85 90 95

Thr Phe Gly Ala Gly Thr Lys Leu Glu Leu Lys
100 105

<210> 10
<211> 11
<212> PRT
<213> Artificial

<220>
<223> An artificially synthesized peptide sequence

<400> 10

Arg Ala Ser Gln Asp Ile Ser Asn His Leu Asn
1 5 10

<210> 11
<211> 7
<212> PRT
<213> Artificial

<220>
<223> An artificially synthesized peptide sequence

<400> 11

Tyr Thr Ser Arg Leu His Ser
1 5

<210> 12
<211> 9
<212> PRT
<213> Artificial

<220>
<223> An artificially synthesized peptide sequence

<400> 12

```
Gln Gln Gly Asn Thr Leu Pro Leu Thr
1               5
```

<210> 13
<211> 363
<212> DNA
<213> Artificial

<220>
<223> An artificially synthesized nucleotide sequence

<400> 13

```
caggtccaac tgcagcagcc tggggctgag gttgtgaagc ctggggctcc agtgaagttg     60
tcctgcaagg cttctggcta caccttcacc acctcctgga tgaactgggt gaaacagagg    120
cctggacgag gcctcgagtg gattggaagg attgatcctt ccgatagtga aactcactac    180
aatcaaaagt tcaaggacaa ggccaaactg actgtagaca aatcctccag tacagcctac    240
atccaactca gcagcctgac atctgaggac tctgcggtct attactgtgc aagagggaac    300
tatgattacg gcggctatgc tgtcgactac tggggtcagg gaacctcagt caccgtctcc    360
tca                                                                  363
```

<210> 14
<211> 121
<212> PRT
<213> Artificial

<220>
<223> An artificially synthesized peptide sequence

<400> 14

```
Gln Val Gln Leu Gln Gln Pro Gly Ala Glu Val Val Lys Pro Gly Ala
1               5                   10                  15

Pro Val Lys Leu Ser Cys Lys Ala Ser Gly Tyr Thr Phe Thr Thr Ser
            20                  25                  30

Trp Met Asn Trp Val Lys Gln Arg Pro Gly Arg Gly Leu Glu Trp Ile
        35                  40                  45

Gly Arg Ile Asp Pro Ser Asp Ser Glu Thr His Tyr Asn Gln Lys Phe
    50                  55                  60

Lys Asp Lys Ala Lys Leu Thr Val Asp Lys Ser Ser Ser Thr Ala Tyr
65                  70                  75                  80

Ile Gln Leu Ser Ser Leu Thr Ser Glu Asp Ser Ala Val Tyr Tyr Cys
                85                  90                  95

Ala Arg Gly Asn Tyr Asp Tyr Gly Gly Tyr Ala Val Asp Tyr Trp Gly
            100                 105                 110

Gln Gly Thr Ser Val Thr Val Ser Ser
        115                 120
```

<210> 15
<211> 5
<212> PRT
<213> Artificial

<220>
<223> An artificially synthesized peptide sequence

<400> 15

```
Thr Ser Trp Met Asn
1               5
```

<210> 16
<211> 17
<212> PRT
<213> Artificial

<220>
<223> An artificially synthesized peptide sequence

<400> 16

```
Arg Ile Asp Pro Ser Asp Ser Glu Thr His Tyr Asn Gln Lys Phe Lys
1               5                   10                  15

Asp
```

<210> 17
<211> 12
<212> PRT
<213> Artificial

<220>
<223> An artificially synthesized peptide sequence

<400> 17

```
Gly Asn Tyr Asp Tyr Gly Gly Tyr Ala Val Asp Tyr
1               5                   10
```

<210> 18
<211> 336
<212> DNA
<213> Artificial

<220>
<223> An artificially synthesized nucleotide sequence

<400> 18

```
gatgttgtga tgacccaaag tccactctcc ctgcctgtca gtcttggaga tcaagcctcc     60
atctcttgca aatccagtca gagccttgta cacagtaatg gaaacaccca tttacattgg    120
tacctgaaga ggccaggcca gtctccaaag ctcctgatct acaaagtttc caaccgattt    180
tctggggtcc cagacaggtt cagtggcagt ggatcaggga cagatttcac actcaagatc    240
agcagagtgg aggctgagga tctgggaatt tatttctgct ctcaaagtac acatgttccg    300
tacacgttcg gaggggggac caagctggaa ataaaa                              336
```

<210> 19
<211> 112
<212> PRT
<213> Artificial

<220>
<223> An artificially synthesized peptide sequence

<400> 19

```
Asp Val Val Met Thr Gln Ser Pro Leu Ser Leu Pro Val Ser Leu Gly
1               5                   10                  15

Asp Gln Ala Ser Ile Ser Cys Lys Ser Ser Gln Ser Leu Val His Ser
            20                  25                  30

Asn Gly Asn Thr His Leu His Trp Tyr Leu Lys Arg Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Ile Tyr Phe Cys Ser Gln Ser
                85                  90                  95

Thr His Val Pro Tyr Thr Phe Gly Gly Gly Thr Lys Leu Glu Ile Lys
            100                 105                 110
```

<210> 20
<211> 16
<212> PRT
<213> Artificial

<220>
<223> An artificially synthesized peptide sequence

<400> 20

```
Lys Ser Ser Gln Ser Leu Val His Ser Asn Gly Asn Thr His Leu His
1               5                   10                  15
```

<210> 21
<211> 7
<212> PRT
<213> Artificial

<220>
<223> An artificially synthesized peptide sequence

<400> 21

```
Lys Val Ser Asn Arg Phe Ser
1               5
```

<210> 22
<211> 9
<212> PRT
<213> Artificial

<220>

<223> An artificially synthesized peptide sequence

<400> 22

Ser Gln Ser Thr His Val Pro Tyr Thr
1 5

<210> 23
<211> 354
<212> DNA
<213> Artificial

<220>
<223> An artificially synthesized nucleotide sequence

<400> 23

```
caggtccaac tgcagcagtc tggggctgag ttggtgaggc ctggagtttc agtgaagctg     60
tcctgcaagg cttctggcta ctccctcacc acctactgga tgaactgggt gaagcagagg    120
cctggacaag gccttgagtg gattggcatg attcatcctt ccgatagtga aactgtaata    180
aatcagaagt tcaaagacaa ggccacattg actgtagaca aatcctccag cacagcctac    240
atgcaactca gcagcccgac atctgaggac tctgcggtct attactgtgc aagatgggat    300
tacgactact actttgacta ctggggccaa ggcaccactc tcacagtctc ctca          354
```

<210> 24
<211> 118
<212> PRT
<213> Artificial

<220>
<223> An artificially synthesized peptide sequence

<400> 24

Gln Val Gln Leu Gln Gln Ser Gly Ala Glu Leu Val Arg Pro Gly Val
1 5 10 15

Ser Val Lys Leu Ser Cys Lys Ala Ser Gly Tyr Ser Leu Thr Thr Tyr
20 25 30

Trp Met Asn Trp Val Lys Gln Arg Pro Gly Gln Gly Leu Glu Trp Ile
35 40 45

Gly Met Ile His Pro Ser Asp Ser Glu Thr Val Ile Asn Gln Lys Phe
50 55 60

Lys Asp Lys Ala Thr Leu Thr Val Asp Lys Ser Ser Ser Thr Ala Tyr
65 70 75 80

Met Gln Leu Ser Ser Pro Thr Ser Glu Asp Ser Ala Val Tyr Tyr Cys
85 90 95

Ala Arg Trp Asp Tyr Asp Tyr Tyr Phe Asp Tyr Trp Gly Gln Gly Thr
100 105 110

Thr Leu Thr Val Ser Ser
115

<210> 25
<211> 5
<212> PRT
<213> Artificial

<220>
<223> An artificially synthesized peptide sequence

<400> 25

Thr Tyr Trp Met Asn
1 5

<210> 26
<211> 17
<212> PRT
<213> Artificial

<220>
<223> An artificially synthesized peptide sequence

<400> 26

Met Ile His Pro Ser Asp Ser Glu Thr Val Ile Asn Gln Lys Phe Lys
1 5 10 15

Asp

<210> 27
<211> 9
<212> PRT
<213> Artificial

<220>
<223> An artificially synthesized peptide sequence

<400> 27

```
Trp Asp Tyr Asp Tyr Tyr Phe Asp Tyr
1               5
```

<210> 28
<211> 336
<212> DNA
<213> Artificial

<220>
<223> An artificially synthesized nucleotide sequence

<400> 28

```
gatgttttga tgacccaaac tccactctcc ctgcctgtca gtcttggaga tcaagcctcc     60
atctcttgca gatctagtca gagcatagta catagtaatg gaaacaccta tttagaatgg    120
tacctgcaga aaccgggcca gtctccgaac ctcctgatct acaaagtttc caaccgattt    180
tctggggtcc cagacaggtt cagtggcagt ggatcaggga cagatttcac actcaagatc    240
agcagagtgg aggctgagga tctgggagtt tattactgct ttcaaggttc tcatgttcca    300
ttcacgttcg gctcggggac aaagttggaa ataaaa                               336
```

<210> 29
<211> 112
<212> PRT
<213> Artificial

<220>
<223> An artificially synthesized peptide sequence

<400> 29

Asp Val Leu Met Thr Gln Thr Pro Leu Ser Leu Pro Val Ser Leu Gly
1 5 10 15

Asp Gln Ala Ser Ile Ser Cys Arg Ser Ser Gln Ser Ile Val His Ser
20 25 30

Asn Gly Asn Thr Tyr Leu Glu Trp Tyr Leu Gln Lys Pro Gly Gln Ser
35 40 45

Pro Asn Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Leu Gly Val Tyr Tyr Cys Phe Gln Gly
85 90 95

Ser His Val Pro Phe Thr Phe Gly Ser Gly Thr Lys Leu Glu Ile Lys
100 105 110

<210> 30
<211> 16
<212> PRT
<213> Artificial

<220>
<223> An artificially synthesized peptide sequence

<400> 30

Arg Ser Ser Gln Ser Ile Val His Ser Asn Gly Asn Thr Tyr Leu Glu
1 5 10 15

<210> 31
<211> 7
<212> PRT
<213> Artificial

<220>
<223> An artificially synthesized peptide sequence

<400> 31

Lys Val Ser Asn Arg Phe Ser
1 5

<210> 32
<211> 9
<212> PRT
<213> Artificial

<220>
<223> An artificially synthesized peptide sequence

<400> 32

Phe Gln Gly Ser His Val Pro Phe Thr
1 5

<210> 33
<211> 363
<212> DNA
<213> Artificial

<220>
<223> An artificially synthesized nucleotide sequence

<400> 33

cagattcagc ttaaggagtc tggacctgtt gtcatcaagc catcacagtc actgtctctc 60

acctgcatag tttctggatt ctccatcacg agtagtcgtt tttgctggaa ttggatccgc 120

cagccccag gaaaggggtt agagtggatg gggcgcatat gttatgaagg ttcaagatac 180

tatagtccat ccatcaaaag ccgcagcacc atctccagag acacatctct gaacaaaatc 240

ttttttcagc tgaattctgt gacaagcgag gacacagcca tgtactactg ttccagggaa 300

aaacagattg ctgggacggg gtttgactac tggggccaag gcaccactct cacagtctcc 360

tca 363

<210> 34
<211> 121
<212> PRT
<213> Artificial

<220>
<223> An artificially synthesized peptide sequence

<400> 34

Gln Ile Gln Leu Lys Glu Ser Gly Pro Val Val Ile Lys Pro Ser Gln
1 5 10 15

Ser Leu Ser Leu Thr Cys Ile Val Ser Gly Phe Ser Ile Thr Ser Ser
20 25 30

Arg Phe Cys Trp Asn Trp Ile Arg Gln Pro Pro Gly Lys Gly Leu Glu
35 40 45

Trp Met Gly Arg Ile Cys Tyr Glu Gly Ser Arg Tyr Tyr Ser Pro Ser
50 55 60

Ile Lys Ser Arg Ser Thr Ile Ser Arg Asp Thr Ser Leu Asn Lys Ile
65 70 75 80

Phe Phe Gln Leu Asn Ser Val Thr Ser Glu Asp Thr Ala Met Tyr Tyr
85 90 95

Cys Ser Arg Glu Lys Gln Ile Ala Gly Thr Gly Phe Asp Tyr Trp Gly
100 105 110

Gln Gly Thr Thr Leu Thr Val Ser Ser
115 120

<210> 35
<211> 7
<212> PRT
<213> Artificial

<220>
<223> An artificially synthesized peptide sequence

<400> 35

Ser Ser Arg Phe Cys Trp Asn
1 5

<210> 36
<211> 16
<212> PRT
<213> Artificial

<220>
<223> An artificially synthesized peptide sequence

<400> 36

Arg Ile Cys Tyr Glu Gly Ser Arg Tyr Tyr Ser Pro Ser Ile Lys Ser
1 5 10 15

<210> 37
<211> 11
<212> PRT
<213> Artificial

<220>

<223> An artificially synthesized peptide sequence

<400> 37

```
Glu Lys Gln Ile Ala Gly Thr Gly Phe Asp Tyr
1               5                   10
```

<210> 38
<211> 321
<212> DNA
<213> Artificial

<220>
<223> An artificially synthesized nucleotide sequence

<400> 38

```
gacattgtga tgacccagtc tcacaaattc atgtccacat cagtaggaga cagggtcagt     60
atcacctgca aggccagtca ggatgtgagt actgctgtag cctggtatca acagaaacca    120
ggacaatctc ctaaactact gatttactcg tcatcctacc gttacactgg agtccctgat    180
cgcttcactg gcagtggatc tgggacggat ttcactttca ccatcagcag tgtgcagtct    240
gaagacctgg cagtttatta ctgtcagcaa cattatagta ctccgctcac gttcggtgct    300
gggaccaagc tggagctgaa a                                              321
```

<210> 39
<211> 107
<212> PRT
<213> Artificial

<220>
<223> An artificially synthesized peptide sequence

<400> 39

Asp Ile Val Met Thr Gln Ser His Lys Phe Met Ser Thr Ser Val Gly
1 5 10 15

Asp Arg Val Ser Ile Thr Cys Lys Ala Ser Gln Asp Val Ser Thr Ala
20 25 30

Val Ala Trp Tyr Gln Gln Lys Pro Gly Gln Ser Pro Lys Leu Leu Ile
35 40 45

Tyr Ser Ser Ser Tyr Arg Tyr Thr Gly Val Pro Asp Arg Phe Thr Gly
50 55 60

Ser Gly Ser Gly Thr Asp Phe Thr Phe Thr Ile Ser Ser Val Gln Ser
65 70 75 80

Glu Asp Leu Ala Val Tyr Tyr Cys Gln Gln His Tyr Ser Thr Pro Leu
85 90 95

Thr Phe Gly Ala Gly Thr Lys Leu Glu Leu Lys
100 105

<210> 40
<211> 11
<212> PRT
<213> Artificial

<220>
<223> An artificially synthesized peptide sequence

<400> 40

Lys Ala Ser Gln Asp Val Ser Thr Ala Val Ala
1 5 10

<210> 41
<211> 7
<212> PRT
<213> Artificial

<220>
<223> An artificially synthesized peptide sequence

<400> 41

Ser Ser Ser Tyr Arg Tyr Thr
1 5

<210> 42
<211> 9
<212> PRT
<213> Artificial

<220>

<223> An artificially synthesized peptide sequence

<400> 42

Gln Gln His Tyr Ser Thr Pro Leu Thr
1 5

<210> 43
<211> 357
<212> DNA
<213> Artificial

<220>
<223> An artificially synthesized nucleotide sequence

<400> 43

gagatccagc tgcagcagtc tagacctgag ctggtgaggc ctggggcttc agtgaaggta 60

tcctgccagg cttctggtta tgcattcact aactacaaca tgtactgggt gaagcagagc 120

catggaaaga gccttgagtg gattggatat attgatcctt acaatgatga tactacctac 180

aaccagaagt tcaagggcaa ggccacattg actgttgaca agtcctccag cacagcctac 240

atgcatctca acagcctgac atctgaggac tctgcagtct tttactgtgc aagatttcga 300

ctgggatact atgctatgga ctactggggt caaggagcct cagtcaccgt ctcctca 357

<210> 44
<211> 119
<212> PRT
<213> Artificial

<220>
<223> An artificially synthesized peptide sequence

<400> 44

Glu Ile Gln Leu Gln Gln Ser Arg Pro Glu Leu Val Arg Pro Gly Ala
1 5 10 15

Ser Val Lys Val Ser Cys Gln Ala Ser Gly Tyr Ala Phe Thr Asn Tyr
20 25 30

Asn Met Tyr Trp Val Lys Gln Ser His Gly Lys Ser Leu Glu Trp Ile
35 40 45

Gly Tyr Ile Asp Pro Tyr Asn Asp Asp Thr Thr Tyr Asn Gln Lys Phe
50 55 60

Lys Gly Lys Ala Thr Leu Thr Val Asp Lys Ser Ser Ser Thr Ala Tyr
65 70 75 80

Met His Leu Asn Ser Leu Thr Ser Glu Asp Ser Ala Val Phe Tyr Cys
85 90 95

Ala Arg Phe Arg Leu Gly Tyr Tyr Ala Met Asp Tyr Trp Gly Gln Gly
100 105 110

Ala Ser Val Thr Val Ser Ser
115

<210> 45
<211> 5
<212> PRT
<213> Artificial

<220>
<223> An artificially synthesized peptide sequence

<400> 45

Asn Tyr Asn Met Tyr
1 5

<210> 46
<211> 17
<212> PRT
<213> Artificial

<220>
<223> An artificially synthesized peptide sequence

<400> 46

Tyr Ile Asp Pro Tyr Asn Asp Asp Thr Thr Tyr Asn Gln Lys Phe Lys
1 5 10 15

Gly

<210> 47
<211> 10
<212> PRT
<213> Artificial

<220>
<223> An artificially synthesized peptide sequence

<400> 47

```
Phe Arg Leu Gly Tyr Tyr Ala Met Asp Tyr
1               5                   10
```

<210> 48
<211> 333
<212> DNA
<213> Artificial

<220>
<223> An artificially synthesized nucleotide sequence

<400> 48

```
gacattgtgc tgacccaatc tccagcttct ttggctgtgt ctctagggca gagggccacc     60

atctcctgca aggccagcca aagtgttgat tatgatcttg atagttatat gaactggtac    120

caacagaaac caggacagcc acccaaactc ctcatctatg ttgcatccaa tctagaatct    180

gggatcccag ccaggtttag tggcagtggg tctgggacag acttcaccct caacatccat    240

cctgtggagg aggaggatgc tgcaacctat tactgtcaac aaagtaatga ggatccattc    300

acgttcggct cggggacaaa gttggaaata aaa                                 333
```

<210> 49
<211> 111
<212> PRT
<213> Artificial

<220>
<223> An artificially synthesized peptide sequence

<400> 49

```
Asp Ile Val Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Leu Asp Ser Tyr Met Asn Trp Tyr Gln Gln Lys Pro Gly Gln Pro Pro
        35                  40                  45
```

```
Lys Leu Leu Ile Tyr Val Ala Ser Asn Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser Asn
                85                  90                  95

Glu Asp Pro Phe Thr Phe Gly Ser Gly Thr Lys Leu Glu Ile Lys
            100                 105                 110
```

<210> 50
<211> 15
<212> PRT
<213> Artificial

<220>
<223> An artificially synthesized peptide sequence

<400> 50

```
Lys Ala Ser Gln Ser Val Asp Tyr Asp Leu Asp Ser Tyr Met Asn
1               5                   10                  15
```

<210> 51
<211> 7
<212> PRT
<213> Artificial

<220>
<223> An artificially synthesized peptide sequence

<400> 51

```
Val Ala Ser Asn Leu Glu Ser
1               5
```

<210> 52
<211> 9
<212> PRT
<213> Artificial

<220>
<223> An artificially synthesized peptide sequence

<400> 52

```
Gln Gln Ser Asn Glu Asp Pro Phe Thr
1               5
```

<210> 53
<211> 354
<212> DNA
<213> Artificial

<220>
<223> An artificially synthesized nucleotide sequence

<400> 53

```
caggtccaac tgcagcagcc tgggcctgag ctggtgaggc ctggagcttc agtgacgctg     60
tcctgcaagg cttctggcta ctccttcacc agctactgga tgaactgggt gaagcagagg    120
cctggacaag gccttgagtg gattggcatg attcatcctt ccgatagtga aactatatta    180
aatcagaagt tcaaggacaa ggccacattg actgtagaca aatcctccag cacagcctcc    240
atgcaactca gcagcccgac atctgaggac tctgcggtct attactgtgc aagatgctta    300
ctaccccact actttgacta ctggggccaa ggcaccactc tcacagtctc ctca          354
```

<210> 54
<211> 118
<212> PRT
<213> Artificial

<220>
<223> An artificially synthesized peptide sequence

<400> 54

```
Gln Val Gln Leu Gln Gln Pro Gly Pro Glu Leu Val Arg Pro Gly Ala
1               5                   10                  15

Ser Val Thr Leu Ser Cys Lys Ala Ser Gly Tyr Ser Phe Thr Ser Tyr
            20                  25                  30

Trp Met Asn Trp Val Lys Gln Arg Pro Gly Gln Gly Leu Glu Trp Ile
        35                  40                  45

Gly Met Ile His Pro Ser Asp Ser Glu Thr Ile Leu Asn Gln Lys Phe
    50                  55                  60

Lys Asp Lys Ala Thr Leu Thr Val Asp Lys Ser Ser Ser Thr Ala Ser
65                  70                  75                  80

Met Gln Leu Ser Ser Pro Thr Ser Glu Asp Ser Ala Val Tyr Tyr Cys
                85                  90                  95

Ala Arg Cys Leu Leu Pro His Tyr Phe Asp Tyr Trp Gly Gln Gly Thr
            100                 105                 110

Thr Leu Thr Val Ser Ser
        115
```

<210> 55

<211> 5
<212> PRT
<213> Artificial

<220>
<223> An artificially synthesized peptide sequence

<400> 55

```
Ser Tyr Trp Met Asn
1               5
```

<210> 56
<211> 17
<212> PRT
<213> Artificial

<220>
<223> An artificially synthesized peptide sequence

<400> 56

```
Met Ile His Pro Ser Asp Ser Glu Thr Ile Leu Asn Gln Lys Phe Lys
1               5                   10                  15

Asp
```

<210> 57
<211> 9
<212> PRT
<213> Artificial

<220>
<223> An artificially synthesized peptide sequence

<400> 57

```
Cys Leu Leu Pro His Tyr Phe Asp Tyr
1               5
```

<210> 58
<211> 339
<212> DNA
<213> Artificial

<220>
<223> An artificially synthesized nucleotide sequence

<400> 58

```
gatgttgtga tgacccaagc tccactctcc ctgcctgtca gtcttggaga tcaagcctcc     60
atctcttgca gatctagtca gagccttgta cacagtaatg gaaacaccta tttacattgg    120
tacctgcaga agccaggcca gtctccaaag ctcctgatct acaaagtttc caaccgattt    180
tctggggtcc cagacaggtt cagtggcagt ggatcaggga cagatttcac actcaagatc    240
agtagagtgg aggctgagga tctgggagtt tatttctgct ctcaaagtac acatgttcct    300
ccattcacgt tcggctcggg gacaaagttg gaaataaaa                           339
```

<210> 59
<211> 113
<212> PRT
<213> Artificial

<220>
<223> An artificially synthesized peptide sequence

<400> 59

```
Asp Val Val Met Thr Gln Ala Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Pro Phe Thr Phe Gly Ser Gly Thr Lys Leu Glu Ile
            100                 105                 110

Lys
```

<210> 60
<211> 16
<212> PRT
<213> Artificial

<220>
<223> An artificially synthesized peptide sequence

<400> 60

Arg Ser Ser Gln Ser Leu Val His Ser Asn Gly Asn Thr Tyr Leu His
1               5                   10                  15

<210> 61
<211> 7
<212> PRT
<213> Artificial

<220>
<223> An artificially synthesized peptide sequence

<400> 61

Lys Val Ser Asn Arg Phe Ser
1               5

<210> 62
<211> 10
<212> PRT
<213> Artificial

<220>
<223> An artificially synthesized peptide sequence

<400> 62

Ser Gln Ser Thr His Val Pro Pro Phe Thr
1               5                   10

<210> 63
<211> 354
<212> DNA
<213> Artificial

<220>
<223> An artificially synthesized nucleotide sequence

<400> 63

caggtccaac tgcagcagcc tggggctgag ctggtgaggc ctggggcttc agtgaagctg 60

tcctgcaagg cttctggcta cacgttcacc agctactgga tgaactgggt taagcagagg 120

cctgagcaag gccttgagtg gattggaagg attgatcctt acgatagtga aactcactac 180

aatcaaaagt tcaaggacaa ggccatattg actgtagaca aatcctccag cacagcctac 240

atgcaactca gcagcctgac atctgaggac tctgcggtct attactgtgc aagatgggag 300

gtagtagccc cctgggacta ctggggccaa ggcaccactc tcacagtctc ctca 354

<210> 64
<211> 118
<212> PRT
<213> Artificial

<220>
<223> An artificially synthesized peptide sequence

<400> 64

```
Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Thr Phe Thr Ser Tyr
            20                  25                  30

Trp Met Asn Trp Val Lys Gln Arg Pro Glu Gln Gly Leu Glu Trp Ile
        35                  40                  45

Gly Arg Ile Asp Pro Tyr Asp Ser Glu Thr His Tyr Asn Gln Lys Phe
    50                  55                  60

Lys Asp Lys Ala Ile Leu Thr Val Asp Lys Ser Ser Ser Thr Ala Tyr

65                  70                  75                  80

Met Gln Leu Ser Ser Leu Thr Ser Glu Asp Ser Ala Val Tyr Tyr Cys
                85                  90                  95

Ala Arg Trp Glu Val Val Ala Pro Trp Asp Tyr Trp Gly Gln Gly Thr
            100                 105                 110

Thr Leu Thr Val Ser Ser
        115
```

<210> 65
<211> 5
<212> PRT
<213> Artificial

<220>
<223> An artificially synthesized peptide sequence

<400> 65

```
Ser Tyr Trp Met Asn
1               5
```

<210> 66
<211> 17
<212> PRT
<213> Artificial

<220>
<223> An artificially synthesized peptide sequence

<400> 66

Arg Ile Asp Pro Tyr Asp Ser Glu Thr His Tyr Asn Gln Lys Phe Lys
1 5 10 15

Asp

<210> 67
<211> 9
<212> PRT
<213> Artificial

<220>
<223> An artificially synthesized peptide sequence

<400> 67

Trp Glu Val Val Ala Pro Trp Asp Tyr
1 5

<210> 68
<211> 336
<212> DNA
<213> Artificial

<220>
<223> An artificially synthesized nucleotide sequence

<400> 68

gatattgtga tgactcaggc tgcaccctct gtacctgtca ctcctggaga gtcagtatcc 60

atctcctgca ggtctagtaa gagtctcctg catagtaatg gcaacactta cttgtattgg 120

ttcctgcaga ggccaggcca gtctcctcag ctcctgatat atcggatgtc caaccttgcc 180

tcaggagtcc cagacaggtt cagtggcagt gggtcaggaa ctgctttcac actgagaatc 240

agtagagtgg aggctgagga tgtgggtgtt tattactgta tgcaacatct agaatatccg 300

tggacgttcg gtggaggcac caagctggaa atcaaa 336

<210> 69
<211> 112
<212> PRT
<213> Artificial

<220>
<223> An artificially synthesized peptide sequence

<400> 69

Asp Ile Val Met Thr Gln Ala Ala Pro Ser Val Pro Val Thr Pro Gly
1 5 10 15

Glu Ser Val Ser Ile Ser Cys Arg Ser Ser Lys Ser Leu Leu His Ser
20 25 30

Asn Gly Asn Thr Tyr Leu Tyr Trp Phe Leu Gln Arg Pro Gly Gln Ser
35 40 45

Pro Gln Leu Leu Ile Tyr Arg Met Ser Asn Leu Ala Ser Gly Val Pro
50 55 60

Asp Arg Phe Ser Gly Ser Gly Ser Gly Thr Ala Phe Thr Leu Arg Ile
65 70 75 80

Ser Arg Val Glu Ala Glu Asp Val Gly Val Tyr Tyr Cys Met Gln His
85 90 95

Leu Glu Tyr Pro Trp Thr Phe Gly Gly Gly Thr Lys Leu Glu Ile Lys
100 105 110

<210> 70
<211> 16
<212> PRT
<213> Artificial

<220>
<223> An artificially synthesized peptide sequence

<400> 70

Arg Ser Ser Lys Ser Leu Leu His Ser Asn Gly Asn Thr Tyr Leu Tyr
1 5 10 15

<210> 71
<211> 7
<212> PRT
<213> Artificial

<220>
<223> An artificially synthesized peptide sequence

<400> 71

Arg Met Ser Asn Leu Ala Ser
1 5

<210> 72
<211> 9
<212> PRT
<213> Artificial

<220>

<223> An artificially synthesized peptide sequence

<400> 72

```
Met Gln His Leu Glu Tyr Pro Trp Thr
1               5
```

<210> 73
<211> 357
<212> DNA
<213> Artificial

<220>
<223> An artificially synthesized nucleotide sequence

<400> 73

```
caggtccaac tgcagcagcc tggggctgag ctggtgaggc ctggagcttc agtgaagctg     60
tcctgcaagg cttctggcta ctccttcacc agctactgga tgaactgggt gaagcagagg    120
cctggacaag gccttgagtg gattggcatg attcatcctt ccgatagtga aactaggtta    180
aatcagaagt tcaaggacaa ggccacattg actgtagaca aatcctccag cacagcctcc    240
atgcaactca gcagcccgac atctgaggac tctgcggtct attactgtgc aagagggggg    300
gattatccct actactttga ctactggggc caaggcacca ctctcacagt ctcctca       357
```

<210> 74
<211> 119
<212> PRT
<213> Artificial

<220>
<223> An artificially synthesized peptide sequence

<400> 74

```
Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Ser Phe Thr Ser Tyr
            20                  25                  30

Trp Met Asn Trp Val Lys Gln Arg Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Lys Asp Lys Ala Thr Leu Thr Val Asp Lys Ser Ser Ser Thr Ala Ser
65                  70                  75                  80

Met Gln Leu Ser Ser Pro Thr Ser Glu Asp Ser Ala Val Tyr Tyr Cys
                85                  90                  95

Ala Arg Gly Gly Asp Tyr Pro Tyr Tyr Phe Asp Tyr Trp Gly Gln Gly
            100                 105                 110

Thr Thr Leu Thr Val Ser Ser
        115
```

<210> 75
<211> 5
<212> PRT
<213> Artificial

<220>
<223> An artificially synthesized peptide sequence

<400> 75

```
Ser Tyr Trp Met Asn
1               5
```

<210> 76
<211> 17
<212> PRT
<213> Artificial

<220>
<223> An artificially synthesized peptide sequence

<400> 76

```
Met Ile His Pro Ser Asp Ser Glu Thr Arg Leu Asn Gln Lys Phe Lys
1               5                   10                  15

Asp
```

<210> 77
<211> 10
<212> PRT
<213> Artificial

<220>
<223> An artificially synthesized peptide sequence

<400> 77

```
Gly Gly Asp Tyr Pro Tyr Tyr Phe Asp Tyr
1               5                   10
```

<210> 78
<211> 339
<212> DNA
<213> Artificial

<220>
<223> An artificially synthesized nucleotide sequence

<400> 78

```
gacattgtga tgtcacagtc tccatcctcc ctggctgtgt cagcaggaga gaaggtcact     60
atgagctgca aatccagtca gagtctgctc aacagtagaa cccgaaagaa ctacttggct    120
tggtaccagc agaaaccagg gcagtctcct aaactgctga tctactgggc atccactagg    180
gaatctgggg tccctgatcg cttcacaggc agtggatctg ggacagattt cactctcacc    240
atcagcagtg tgcaggctga agacctggca gtttattact gcaagcactc ttataatctt    300
atattcacgt tcggctcggg gacaaagttg gaaataaaa                           339
```

<210> 79
<211> 113
<212> PRT
<213> Artificial

<220>
<223> An artificially synthesized peptide sequence

<400> 79

Asp Ile Val Met Ser Gln Ser Pro Ser Ser Leu Ala Val Ser Ala Gly
1 5 10 15

Glu Lys Val Thr Met Ser Cys Lys Ser Ser Gln Ser Leu Leu Asn Ser
20 25 30

Arg Thr Arg Lys Asn Tyr Leu Ala Trp Tyr Gln Gln Lys Pro Gly Gln
35 40 45

Ser Pro Lys Leu Leu Ile Tyr Trp Ala Ser Thr Arg Glu Ser Gly Val
50 55 60

Pro Asp Arg Phe Thr Gly Ser Gly Ser Gly Thr Asp Phe Thr Leu Thr
65 70 75 80

Ile Ser Ser Val Gln Ala Glu Asp Leu Ala Val Tyr Tyr Cys Lys His
85 90 95

Ser Tyr Asn Leu Ile Phe Thr Phe Gly Ser Gly Thr Lys Leu Glu Ile
100 105 110

Lys

<210> 80
<211> 17
<212> PRT
<213> Artificial

<220>
<223> An artificially synthesized peptide sequence

<400> 80

Lys Ser Ser Gln Ser Leu Leu Asn Ser Arg Thr Arg Lys Asn Tyr Leu
1 5 10 15

Ala

<210> 81
<211> 7
<212> PRT
<213> Artificial

<220>
<223> An artificially synthesized peptide sequence

<400> 81

Trp Ala Ser Thr Arg Glu Ser
1 5

<210> 82

<211> 9
<212> PRT
<213> Artificial

<220>
<223> An artificially synthesized peptide sequence

<400> 82

```
Lys His Ser Tyr Asn Leu Ile Phe Thr
1               5
```

<210> 83
<211> 357
<212> DNA
<213> Artificial

<220>
<223> An artificially synthesized nucleotide sequence

<400> 83

```
caggtccaac tgcagcagcc tggggctgag ctggtgaggc ctggggcttc agtgaagctg     60
tcctgcaagg cttctggcta cacgttcacc agctactggg tgaactgggt taagcagagg    120
cctgaggaag gccttgagtg gattggaagg attgatcctt acgatagtga aattcactac    180
aatcaaaagt tcaaggacaa ggccatattg actgtagaca aatcctccag cacagcctac    240
atgcaactca gcagcctgac atctggggac tctgcggtct attactgtgc aaaagggggg    300
gaattacctt actactttga ctattggggc cacggcacca ctctcacagt ctcctca       357
```

<210> 84
<211> 119
<212> PRT
<213> Artificial

<220>
<223> An artificially synthesized peptide sequence

<400> 84

Gln Val Gln Leu Gln Gln Pro Gly Ala Glu Leu Val Arg Pro Gly Ala
1 5 10 15

Ser Val Lys Leu Ser Cys Lys Ala Ser Gly Tyr Thr Phe Thr Ser Tyr
20 25 30

Trp Val Asn Trp Val Lys Gln Arg Pro Glu Glu Gly Leu Glu Trp Ile
35 40 45

Gly Arg Ile Asp Pro Tyr Asp Ser Glu Ile His Tyr Asn Gln Lys Phe
50 55 60

Lys Asp Lys Ala Ile Leu Thr Val Asp Lys Ser Ser Ser Thr Ala Tyr
65 70 75 80

Met Gln Leu Ser Ser Leu Thr Ser Gly Asp Ser Ala Val Tyr Tyr Cys
85 90 95

Ala Lys Gly Gly Glu Leu Pro Tyr Tyr Phe Asp Tyr Trp Gly His Gly
100 105 110

Thr Thr Leu Thr Val Ser Ser
115

<210> 85
<211> 5
<212> PRT
<213> Artificial

<220>
<223> An artificially synthesized peptide sequence

<400> 85

Ser Tyr Trp Val Asn
1 5

<210> 86
<211> 17
<212> PRT
<213> Artificial

<220>
<223> An artificially synthesized peptide sequence

<400> 86

Arg Ile Asp Pro Tyr Asp Ser Glu Ile His Tyr Asn Gln Lys Phe Lys
1 5 10 15

Asp

<210> 87
<211> 10
<212> PRT
<213> Artificial

<220>
<223> An artificially synthesized peptide sequence

<400> 87

```
Gly Gly Glu Leu Pro Tyr Tyr Phe Asp Tyr
1               5                   10
```

<210> 88
<211> 336
<212> DNA
<213> Artificial

<220>
<223> An artificially synthesized nucleotide sequence

<400> 88

```
gacattgtga tgtcacagtc tccatcctcc ctggctgtgt cagcaggaga gaaggtcact    60
atgagctgca aatccagtca gaatctgctc aacagtggaa cccgaaagaa ctacttggct   120
tggtaccagc agaaaccagg gcagtctcct aaactgctga tctactgggc atccactagg   180
gaatctgggg tccctgatcg cttcacaggc agtggatctg ggacagattt cactctcacc   240
atcagcagtg tgcaggctga agacctggca gtttattact gcaagcaatc ttataatctg   300
tggacgttcg gtggaggcac caagctggaa atcaaa                             336
```

<210> 89
<211> 112
<212> PRT
<213> Artificial

<220>
<223> An artificially synthesized peptide sequence

<400> 89

Asp Ile Val Met Ser Gln Ser Pro Ser Ser Leu Ala Val Ser Ala Gly
1               5                   10                  15

Glu Lys Val Thr Met Ser Cys Lys Ser Ser Gln Asn Leu Leu Asn Ser
            20                  25                  30

Gly Thr Arg Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Lys Gln
                85                  90                  95

Ser Tyr Asn Leu Trp Thr Phe Gly Gly Gly Thr Lys Leu Glu Ile Lys
            100                 105                 110

<210> 90
<211> 17
<212> PRT
<213> Artificial

<220>
<223> An artificially synthesized peptide sequence

<400> 90

Lys Ser Ser Gln Asn Leu Leu Asn Ser Gly Thr Arg Lys Asn Tyr Leu
1               5                   10                  15

Ala

<210> 91
<211> 7
<212> PRT
<213> Artificial

<220>
<223> An artificially synthesized peptide sequence

<400> 91

Trp Ala Ser Thr Arg Glu Ser
1               5

<210> 92
<211> 8
<212> PRT

<213> Artificial

<220>
<223> An artificially synthesized peptide sequence

<400> 92

```
Lys Gln Ser Tyr Asn Leu Trp Thr
1               5
```

<210> 93
<211> 758
<212> PRT
<213> Mus musculus

<400> 93

```
Met Thr Ser Gly Pro Phe Phe Phe Cys Ile Phe Ile Ile Gly Lys Tyr
1               5                   10                  15

Phe Thr Leu Gly Ser Ala Gln Asp Val Ser Cys Pro Leu Gly Ser Phe
            20                  25                  30

Pro Cys Gly Asn Met Ser Arg Cys Leu Pro Gln Leu Leu His Cys Asn
        35                  40                  45

Gly Val Asp Asp Cys Gly Asn Arg Ala Asp Glu Asp His Cys Gly Asp
    50                  55                  60

Asn Asn Gly Trp Ser Leu Gln Leu Asp Lys Tyr Phe Ala Asn Tyr Tyr
65                  70                  75                  80

Lys Leu Ala Ser Thr Asn Ser Phe Glu Ala Glu Thr Ser Glu Cys Leu
                85                  90                  95

Val Gly Ser Val Pro Met His Cys Leu Cys Arg Asp Leu Glu Leu Asp
            100                 105                 110

Cys Asp Glu Ala Asn Leu Arg Ala Val Pro Ser Val Ser Ser Asn Val
        115                 120                 125

Thr Val Met Ser Leu Gln Arg Asn Phe Ile Arg Thr Leu Pro Pro Asn
    130                 135                 140

Gly Phe Arg Lys Tyr His Glu Leu Gln Lys Leu Cys Leu Gln Asn Asn
145                 150                 155                 160

Arg Ile His Ser Val Ser Val Ser Ala Phe Arg Gly Leu Arg Ser Leu
                165                 170                 175
```

```
Thr Lys Leu Tyr Leu Ser His Asn Arg Ile Thr Phe Leu Lys Pro Gly
            180                 185                 190

Val Phe Glu Asp Leu His Arg Leu Glu Trp Leu Ile Ile Glu Asp Asn
        195                 200                 205

His Leu Ser Arg Ile Ser Pro Leu Thr Phe Tyr Gly Leu Asn Ser Leu
    210                 215                 220

Ile Leu Leu Val Leu Met Asn Asn Ala Leu Thr Arg Leu Pro Asp Lys
225                 230                 235                 240

Pro Leu Cys Gln His Met Pro Arg Leu His Trp Leu Asp Phe Glu Gly
                245                 250                 255

Asn Arg Ile His Asn Leu Arg Asn Leu Thr Phe Ile Ser Cys Asn Asn
            260                 265                 270

Leu Thr Val Leu Val Met Arg Lys Asn Lys Ile Asn Tyr Leu Asn Glu
        275                 280                 285

His Ala Phe Thr His Leu Gln Lys Leu Asp Glu Leu Asp Leu Gly Ser
    290                 295                 300

Asn Lys Ile Glu Asn Leu Pro Pro Asn Ile Phe Lys Asp Leu Lys Glu
305                 310                 315                 320

Leu Ser Gln Leu Asn Ile Ser Tyr Asn Pro Ile Gln Lys Ile Glu Val
                325                 330                 335

Asn Gln Phe Asp Cys Leu Ala Lys Leu Lys Ser Leu Ser Leu Glu Gly
            340                 345                 350

Ile Glu Ile Ser Asn Ile Gln Gln Arg Met Phe Arg Pro Leu Ile Asn
        355                 360                 365

Leu Ser His Ile Tyr Phe Lys Lys Phe Gln Tyr Cys Gly Tyr Ala Pro
    370                 375                 380

His Val Arg Ser Cys Lys Pro Asn Thr Asp Gly Ile Ser Ser Leu Glu
385                 390                 395                 400

Asn Leu Leu Ala Ser Ile Ile Gln Arg Val Phe Val Trp Val Val Ser
                405                 410                 415

Ala Ile Thr Cys Phe Gly Asn Ile Phe Val Ile Cys Met Arg Pro Tyr
            420                 425                 430

Ile Arg Ser Glu Asn Lys Leu His Ala Met Ser Ile Ile Ser Leu Cys
```

```
        435                 440                 445

Cys Ala Asp Cys Leu Met Gly Val Tyr Leu Phe Val Ile Gly Ala Phe
    450                 455                 460

Asp Leu Lys Phe Arg Gly Glu Tyr Asn Lys His Ala Gln Pro Trp Met
465                 470                 475                 480

Glu Ser Val His Cys Gln Phe Met Gly Ser Leu Ala Ile Leu Ser Thr
                485                 490                 495

Glu Val Ser Val Leu Leu Leu Thr Phe Leu Thr Leu Glu Lys Tyr Ile
            500                 505                 510

Cys Ile Val Tyr Pro Phe Arg Cys Leu Arg Pro Arg Lys Cys Arg Thr
        515                 520                 525

Ile Thr Val Leu Ile Phe Ile Trp Ile Ile Gly Phe Ile Val Ala Phe
    530                 535                 540

Ala Pro Leu Gly Asn Lys Glu Phe Phe Lys Asn Tyr Tyr Gly Thr Asn
545                 550                 555                 560

Gly Val Cys Phe Pro Leu His Ser Glu Asp Thr Gly Ser Thr Gly Ala
                565                 570                 575

Gln Ile Tyr Ser Val Val Ile Phe Leu Gly Ile Asn Leu Val Ala Phe
            580                 585                 590

Ile Ile Ile Val Phe Ser Tyr Gly Ser Met Phe Tyr Ser Val His Gln
        595                 600                 605

Ser Ser Val Thr Val Thr Glu Ile Gln Lys Gln Val Lys Lys Glu Val
    610                 615                 620

Val Leu Ala Lys Arg Phe Phe Phe Ile Val Phe Thr Asp Ala Leu Cys
625                 630                 635                 640

Trp Ile Pro Ile Phe Ile Leu Lys Phe Leu Ser Leu Leu Gln Val Glu
                645                 650                 655

Ile Pro Asp Ser Ile Thr Ser Trp Val Val Ile Phe Ile Leu Pro Ile
            660                 665                 670

Asn Ser Ala Leu Asn Pro Ile Ile Tyr Thr Leu Thr Thr Arg Pro Phe
        675                 680                 685

Lys Glu Met Ile His Gln Leu Trp His Asn Tyr Arg Gln Arg Arg Ser
    690                 695                 700
```

Val Asp Arg Lys Glu Thr Gln Lys Ala Tyr Ala Pro Ser Phe Ile Trp
705 710 715 720

Val Glu Met Trp Pro Leu Gln Glu Met Ser Ser Gly Phe Met Lys Pro
725 730 735

Gly Ala Phe Thr Asp Pro Cys Asp Leu Ser Leu Val Ser Gln Ser Ser
740 745 750

Arg Leu Asn Ser Tyr Ser
755

<210> 94
<211> 2277
<212> DNA
<213> Mus musculus

<400> 94

```
atgacatctg gtccattctt cttctgcatc ttcattattg gaaaatattt cactctcgga     60
agtgcacagg atgtcagttg tcccctcggc tccttcccct gtgggaacat gagcaggtgc    120
ctgcctcagc tgcttcactg caatggtgtg gacgactgcg ggaaccgagc tgatgaggac    180
cactgcggag acaacaatgg gtggtctctg caactggaca aatattttgc taattactat    240
aaactggctt ccactaactc ctttgaggca gaaacttccg aatgcttggt tggctctgtg    300
cccatgcatt gtttgtgccg agatctagaa cttgactgtg atgaagccaa tttacgagct    360
gtcccatcag tttcttccaa tgtgactgta atgtccctgc agaggaactt cataagaaca    420
ctccctccca acggcttcag gaagtaccat gagctgcaga agctgtgcct gcaaaacaat    480
aggattcact ccgtgagcgt ctctgccttc agaggactgc gcagcctcac taaactgtac    540
ctcagtcata acagaataac cttcctaaag ccaggagttt ttgaagatct ccacagactg    600
gaatggctga taattgaaga caatcacctc agtcgaattt ccccactcac attttacgga    660
ctaaattctc ttattctctt agtgctgatg aataatgccc tcacccgttt gcctgacaaa    720
cccctctgtc agcacatgcc ccgcctgcac tggctggact ttgaaggcaa ccgtatccat    780
aatttaagaa atttgacttt tatttcctgc aataatttaa ctgttttggt aatgaggaag    840
aacaaaatca attacttaaa tgaacatgca tttacacacc tccagaaact agatgaattg    900
gatttaggaa gtaataagat tgaaaatctt ccaccgaaca tatttaagga tctgaaggag    960
ctgtcgcaat tgaatatttc ttataaccca atccagaaaa ttgaagtaaa tcaattcgat   1020
tgtcttgcca aactcaagtc tctcagcctt gaaggaatag aaatttcaaa tatccaacaa   1080
aggatgttta gaccgctcat aaatctctct cacatctatt ttaagaaatt tcagtactgt   1140
ggatacgcac cacatgtgcg cagctgtaag cccaacacgg atgggatttc atctctagag   1200
aacctcttgg caagcatcat ccagagagtc tttgtctggg ttgtatctgc cattacctgc   1260
```

```
tttggaaaca tttttgttat ttgtatgcga ccatacatca gatctgagaa caagctgcat   1320
gccatgtcga tcatttctct ctgctgtgct gactgcctaa tgggggtgta tctatttgtg   1380
attggtgcct ttgacctcaa gtttcgggga gagtacaata agcacgcaca gccgtggatg   1440
gagagtgttc attgtcagtt catggggtcg ttggccattc tgtccacaga agtatccgtt   1500
ttacttttaa catttctgac attggaaaag tacatctgca ttgtgtatcc ttttcggtgt   1560
ttaaggcctc gaaaatgcag aacaatcacg gttctgattt tcatttggat tattgggttt   1620
atagtggctt ttgctccact tggcaataag gaatttttca aaaactacta tggcaccaat   1680
ggagtatgct ttcctcttca ttcagaagac acgggaagta ctggagccca gatttattca   1740
gtggtaattt ttcttggtat taacctggtg gcatttatca tcattgtgtt ctcctatgga   1800
agcatgtttt acagtgttca tcaaagctcc gtaacagtaa ccgaaataca gaagcaagtg   1860
aagaaggagg tggttctcgc caaacgcttt ttctttatcg ttttcaccga cgcgctgtgc   1920
tggattccca tcttcatact gaaatttctt tcactgcttc aggtggaaat accagattct   1980
attacctctt gggtggtgat ttttattctg cctatcaaca gtgctttgaa cccaattatc   2040
tacacgttga ccactagacc tttcaaggaa atgatccatc aactctggca caactacaga   2100
caaagaaggt ctgttgacag gaaagagact cagaaggcat atgctccatc attcatctgg   2160
gtggaaatgt ggcccttgca ggagatgtcc tctgggttca tgaagccagg cgctttcaca   2220
gacccctgtg atctgtcgct agtttctcag tcatctagac tcaattctta ttcgtaa      2277
```

<210> 95
<211> 691
<212> DNA
<213> Homo sapiens

<400> 95

```
ggtcaccgac aaaactcaca catgcccacc gtgcccagca cctgaactcc tgggggggacc     60
gtcagtcttc ctcttccccc caaaacccaa ggacaccctc atgatctccc ggacccctga    120
ggtcacatgc gtggtggtgg acgtgagcca cgaagaccct gaggtcaagt tcaactggta    180
cgtggacggc gtggaggtgc ataatgccaa gacaaagccg cgggaggagc agtacaacag    240
cacgtaccgt gtggtcagcg tcctcaccgt cctgcaccag gactggctga atggcaagga    300
gtacaagtgc aaggtctcca acaaagccct cccagccccc atcgagaaaa ccatctccaa    360
agccaaaggg cagccccgag aaccacaggt gtacaccctg cccccatccc gggatgagct    420
gaccaagaac caggtcagcc tgacctgcct ggtcaaaggc ttctatccca gcgacatcgc    480
cgtggagtgg gagagcaatg ggcagccgga gaacaactac aagaccacgc ctcccgtgct    540
ggactccgac ggctccttct tcctctacag caagctcacc gtggacaaga gcaggtggca    600
gcaggggaac gtcttctcat gctccgtgat gcatgaggct ctgcacaacc actacacgca    660
gaagagcctc tccctgtctc cgggtaaatg a                                    691
```

<210> 96
<211> 229
<212> PRT
<213> Homo sapiens

<400> 96

```
Val Thr Asp Lys Thr His Thr Cys Pro Pro Cys Pro Ala Pro Glu Leu
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser His Glu Asp Pro Glu Val Lys Phe Asn Trp Tyr Val Asp Gly Val
    50                  55                  60

Glu Val His Asn Ala Lys Thr Lys Pro Arg Glu Glu Gln Tyr Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Ala Leu Pro Ala
            100                 105                 110

Pro Ile Glu Lys Thr Ile Ser Lys Ala Lys Gly Gln Pro Arg Glu Pro
        115                 120                 125

Gln Val Tyr Thr Leu Pro Pro Ser Arg Asp Glu Leu Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Lys Leu
            180                 185                 190

Thr Val Asp Lys Ser Arg Trp Gln Gln Gly Asn Val Phe Ser Cys Ser
        195                 200                 205

Val Met His Glu Ala Leu His Asn His Tyr Thr Gln Lys Ser Leu Ser
    210                 215                 220

                      Leu Ser Pro Gly Lys
                      225
```

<210> 97
<211> 21

<212> DNA
<213> Artificial

<220>
<223> An artificially synthesized nucleotide sequence

<400> 97
gggccagtgg atagacagat g 21

<210> 98
<211> 24
<212> DNA
<213> Artificial

<220>
<223> An artificially synthesized nucleotide sequence

<400> 98
caggggccag tggatagacc gatg 24

<210> 99
<211> 24
<212> DNA
<213> Artificial

<220>
<223> An artificially synthesized nucleotide sequence

<400> 99
caggggccag tggatagact gatg 24

<210> 100
<211> 23
<212> DNA
<213> Artificial

<220>
<223> An artificially synthesized nucleotide sequence

<400> 100
gctcactgga tggtgggaag atg 23

<210> 101
<211> 4
<212> PRT
<213> Artificial

<220>
<223> An artificially synthesized peptide sequence

<400> 101

```
Gly Gly Gly Ser
1
```

<210> 102
<211> 4
<212> PRT

<213> Artificial

<220>
<223> An artificially synthesized peptide sequence

<400> 102

Ser Gly Gly Gly
1

<210> 103
<211> 5
<212> PRT
<213> Artificial

<220>
<223> An artificially synthesized peptide sequence

<400> 103

Gly Gly Gly Gly Ser
1 5

<210> 104
<211> 5
<212> PRT
<213> Artificial

<220>
<223> An artificially synthesized peptide sequence

<400> 104

Ser Gly Gly Gly Gly
1 5

<210> 105
<211> 6
<212> PRT
<213> Artificial

<220>
<223> An artificially synthesized peptide sequence

<400> 105

Gly Gly Gly Gly Gly Ser
1 5

<210> 106
<211> 6
<212> PRT
<213> Artificial

<220>
<223> An artificially synthesized peptide sequence

<400> 106

Ser Gly Gly Gly Gly Gly
1 5

<210> 107
<211> 7
<212> PRT
<213> Artificial

<220>
<223> An artificially synthesized peptide sequence

<400> 107

Gly Gly Gly Gly Gly Gly Ser
1 5

<210> 108
<211> 7
<212> PRT
<213> Artificial

<220>
<223> An artificially synthesized peptide sequence

<400> 108

Ser Gly Gly Gly Gly Gly Gly
1 5